# EUROPEAN PATENT SPECIFICATION

(11) **EP 4 192 454 B1**
(45) Date of publication and mention of the grant of the patent: **22.04.2026**
(21) Application number: 21852740.6
(22) Date of filing: 04.08.2021
(51) Int. Cl.: C07D 451/04, C07D 498/08, A61K 31/53, A61K 31/439, A61P 25/00, A61P 35/00

(54) **AZABICYCLO-AMINO-TRIAZINE COMPOUNDS FOR MODULATING SPLICING FOR THE TREATMENT OF NEUROLOGICAL DISEASES**
AZABICYCLO-AMINO-TRIAZIN-VERBINDUNGEN ZUR MODULATION DES SPLEISSEN ZUR BEHANDLUNG NEUROLOGISCHER ERKRANKUNGEN
COMPOSÉS AZABICYCLO-AMINO-TRIAZINE POUR MODULER L'ÉPISSAGE POUR LE TRAITEMENT DES MALADIES NEUROLOGIQUES

(30) Priority: 05.08.2020 US 202063061742 P
(43) Date of publication of application: 14.06.2023
(73) Proprietor: Skyhawk Therapeutics, Inc., Waltham, MA 02451 (US)
(72) Inventor: LUZZIO, Michael, Waltham, Massachusetts 02451 (US); LUCAS, Brian, Waltham, Massachusetts 02451 (US); WANG, Tiansheng, Waltham, Massachusetts 02451 (US)
(74) Representative: Mewburn Ellis LLP
(86) International application number: PCT/US2021/044528
(87) International publication number: WO 2022/031838

(56) References cited:
- WO-A1-2019/028440
- WO-A1-2019/191229
- WO-A1-2019/199972
- WO-A1-2020/163405
- WO-A1-2020/163409
- WO-A2-2022/031998
- US-A1- 2013 029 963

## Description

### TECHNICAL FIELD

The present invention relates to certain compounds of Formula (I), and pharmaceutically acceptable salts thereof, pharmaceutical compositions comprising those compounds and salts thereof, and the use of those compounds, salts, and compositions in a method of treating a disease or condition.

### BACKGROUND

The majority of protein-coding genes in the human genome are composed of multiple exons (coding regions) that are separated by introns (non-coding regions). Gene expression results in a single precursor messenger RNA (pre-mRNA). The intron sequences are subsequently removed from the pre-mRNA by a process called splicing, which results in the mature messenger RNA (mRNA). By including different combinations of exons, alternative splicing gives rise to multiple mRNAs encoding distinct protein isoforms. The spliceosome, an intracellular complex of multiple proteins and ribonucleoproteins, catalyzes splicing.

Current therapeutic approaches to direct and control mRNA expression require methods such as gene therapy, genome editing, or a wide range of oligonucleotide technologies (antisense, RNAi, etc.). Gene therapy and genome editing act upstream of transcription of mRNA by influencing the DNA code and thereby changing mRNA expression. Oligonucleotides modulate the action of RNA via canonical base/base hybridization. The appeal of this approach is in the design of the basic pharmacophore of an oligonucleotide, which can be defined in a straightforward fashion by known base pairing to the target sequence subject. Each of these therapeutic modalities suffers from substantial technical, clinical, and regulatory challenges. Some limitations of oligonucleotides as therapeutics (*e.g*., antisense, RNAi) include unfavorable pharmacokinetics, lack of oral bioavailability, and lack of blood-brain-barrier penetration, with the latter precluding delivery to the brain or spinal cord after parenteral drug administration for the treatment of diseases (*e.g*., neurological diseases, brain cancers). In addition, oligonucleotides are not taken up effectively into solid tumors without a complex delivery system such as lipid nanoparticles. Further, most of the oligonucleotides taken up into cells and tissues remain in non-functional compartments (*e.g*., endosomes) and does not gain access to the cytosol and/or nucleus where the target is located.

Additionally, to anneal to a target, oligonucleotide therapies require access to complementary base pairs of the target. This approach assumes that pre-mRNA sequences exist as a linear strand of RNA in the cell. However, pre-mRNA is rarely linear; it has complex secondary and tertiary structure. Further, cis-acting elements (*e.g*., protein binding elements) and trans-acting factors (*e.g*., splicing complex components) can create additional two-dimensional and three-dimensional complexity (*e*.*g.*, by binding to the pre-mRNA). These features can be potency-and efficacy-limiting for oligonucleotide therapies.
US 2013/0029963 A1 (Heptares Therapeutics. - 31 January 2013) describes 1,2,4-triazine-amine derivatives which are allegedly useful in the treatment of a condition or disorder ameliorated by the inhibition of the A1-A2b or, particularly, the A2a receptor.
WO 2019/191229 A1 (PTC Therapeutics - 3 October 2019) describes phenylpyrazine compounds, forms, and pharmaceutical compositions thereof and methods of using such compounds, forms, or compositions thereof for treating or ameliorating Huntington's disease.
WO 2019/199972 A1 (Skyhawk Therapeutics - 17 October 2019) describes splice modifying compounds affecting splicing of mRNA, such as pre-mRNA, expressed from the FOXM1 gene, compositions comprising thereof, and methods using the same.
WO 2020/163405 A1 (Skyhawk Therapeutics - 13 August 2020) describes small molecule splicing modulator compounds that modulate splicing of mRNA, such as pre-mRNA, encoded by genes, and methods of use of the small molecule splicing modulator compounds for modulating splicing and treating diseases and conditions.
WO 2020/163409 A1 (Skyhawk Therapeutics - 13 August 2020) describes small molecule splicing modulator compounds that modulate splicing of mRNA, such as pre-mRNA, encoded by genes, and methods of use of the small molecule splicing modulator compounds for modulating splicing and treating diseases and conditions.
WO 2019/028440 A1 (Skyhawk Therapeutics - 07 February 2019) describes mall molecule splicing modulator compounds that modulate splicing of mRNA, such as pre-mRNA, encoded by genes, and methods of use of the small molecule splicing modulator compounds for modulating splicing and treating diseases and conditions.
WO 2022/031998 A2 (Skyhawk Therapeutics - 10 February 2022) describes processes for preparing compounds useful in the preparation of small molecule splicing modulator compounds that modulate splicing of mRNA, such as pre-mRNA, encoded by genes, and compounds used in such processes.

### SUMMARY OF THE INVENTION

The novel small molecule splicing modulators (SMSMs) described herein do not suffer from the limitations above, nor the structural and steric hindrances that greatly limit oligonucleotide therapies (*e.g*., by blocking hybridization to pre-mRNA targets). Small molecules have been essential in uncovering the mechanisms, regulations, and functions of many cellular processes, including DNA replication, transcription, and translation. While several recent reports have described screens for small molecule effectors of splicing, only a small number of constitutive or alternative splicing modulators have been identified and many of the small-molecule inhibitors lack specificity, lack selectivity, lack potency, exhibit toxicity, or are not orally available. Targeting the RNA transcriptome with small-molecule modulators represents an untapped therapeutic approach to treat a variety of RNA-mediated diseases. Accordingly, there remains a need to develop small-molecule RNA modulators useful as therapeutic agents. There is need in the art for novel modulators of splicing or splicing-dependent processes. Provided herein are small molecule splicing modulators and uses thereof that fulfill this need.

In one aspect, described herein is a compound of Formula (I): 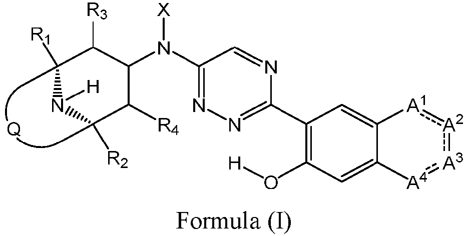
or a pharmaceutically acceptable salt thereof;
wherein:
   Q is C₂-C₄ alkylene optionally substituted with 1, 2, 3, or 4 substituents each independently selected from fluorine, OH, CH₃, and OCH₃; or
   Q is -CH₂OCH₂-, -OCH₂CH₂O-, -OCH₂CH₂OCH₂CH₂O-, or -OCH₂CH₂OCH₂CH₂OCH₂CH₂O-;
   X is hydrogen or CH₃; or
   X is unsubstituted C₃-C₆ cycloalkyl; or
   X is cyclopropyl, cyclobutyl, or cyclopentyl, each substituted with 1, 2, or 3 substituents each independently selected from fluorine, OH, CH₃, and OCH₃;
   each R₁ and R₂ is independently hydrogen, halogen, or CH₃;
   each R₃ and R₄ is independently hydrogen or halogen;
   each A¹, A², and A⁴ is independently N, -NR^{Y1}-, -O-, -S-, or CR^{A1};
   A³ is -NR^{Y1}-, -O-, -S-, or CR^{A1};
   each 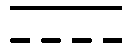 is independently a single or double bond;
   each R^{A1} is independently hydrogen, halogen, =O, or C₁-C₆ alkyl, wherein C₁-C₆ alkyl is optionally substituted with one or more halogen; and
   each R^{Y1} is independently hydrogen or C₁-C₆ alkyl, wherein C₁-C₆ alkyl is optionally substituted with one or more halogen.

Also provided herein are pharmaceutical compositions comprising a compound disclosed herein, or a pharmaceutically acceptable salt thereof, and a pharmaceutically acceptable excipient or carrier.

Also provided herein is a compound described herein, or a pharmaceutically acceptable salt thereof, or a pharmaceutical composition described herein, for use in a method of treating a disease or condition, wherein the method comprises administering the compound, salt or pharmaceutical composition to a subject in need thereof.

### DETAILED DESCRIPTION

References to methods of treatment by therapy or surgery or in vivo diagnosis methods in this description are to be interpreted as references to compounds, pharmaceutical compositions and medicaments of the present invention for use in those methods.

Certain specific details of this description are set forth in order to provide a thorough understanding of various embodiments. However, one skilled in the art will understand that the present disclosure may be practiced without these details. In other instances, well-known structures have not been shown or described in detail to avoid unnecessarily obscuring descriptions of the embodiments.

Unless otherwise defined, all technical and scientific terms used herein have the same meaning as commonly understood by one of ordinary skill in the art to which this disclosure belongs. Although methods and materials similar or equivalent to those described herein can be used in the practice or testing of the present disclosure, suitable methods and materials are described below.

### Definitions

The terms "compound(s) of this disclosure", "compound(s) of the present disclosure", "small molecule steric modulator(s)", "small molecule splicing modulator(s)" "steric modulator(s)", "splicing modulator(s)", "compound(s) that modify splicing" and "compound(s) modifying splicing", "SMSM" or "small molecule that binds a target RNA," are interchangeably used herein and refer to compounds as disclosed herein and stereoisomers, tautomers, solvates, and salts (*e.g*., pharmaceutically acceptable salts) thereof. The terms "compound(s) of this disclosure", "compound(s) of the present disclosure", "small molecule steric modulator(s)", "small molecule splicing modulator(s)" "steric modulator(s)", "splicing modulator(s)", "compound(s) that modify splicing" and "compound(s) modifying splicing", "SMSM" or "small molecule that binds a target RNA," denote a small molecule compound that binds to a cell component (*e.g.*, DNA, RNA, pre-mRNA, protein, RNP, snRNA, carbohydrates, lipids, co-factors, nutrients and/or metabolites) and modulates splicing of a target polynucleotide, e.g., a pre-mRNA. For example, an SMSM can bind directly or indirectly to a target polynucleotide, *e.g.,* RNA (*e.g.,* a pre-mRNA) with a mutated, non-mutated, bulged and/or aberrant splice site, resulting in modulation of splicing of the target polynucleotide. For example, an SMSM can bind directly or indirectly to a protein, *e.g.,* a spliceosome protein or a ribonuclear protein, resulting in steric modulation of the protein and modulation of splicing of a target RNA. For example, an SMSM can bind directly or indirectly to a spliceosome component, *e.g*., a spliceosome protein or snRNA resulting in steric modulation of the spliceosome protein or snRNA and modulation of splicing of target polynucleotide. These terms specifically exclude compounds consisting of oligonucleotides. These terms include small molecule compounds that may bind to one or more secondary or tertiary structure elements of a target RNA. These sites include RNA triplexes, 3WJs, 4WJs, parallel-Y junctions, hairpins, bulge loops, pseudoknots, internal loops, and other higher-order RNA structural motifs.

The term "RNA" (ribonucleic acid) as used herein, means naturally-occurring or synthetic oligoribonucleotides independent of source (e.g., the RNA may be produced by a human, animal, plant, virus, or bacterium, or may be synthetic in origin), biological context (e.g., the RNA may be in the nucleus, circulating in the blood, in vitro, cell lysate, or isolated or pure form), or physical form (e.g., the RNA may be in single-, double-, or triple-stranded form (including RNA-DNA hybrids), may include epigenetic modifications, native post-transcriptional modifications, artificial modifications (e.g., obtained by chemical or in vitro modification), or other modifications, may be bound to, e.g., metal ions, small molecules, proteins such as chaperones, or co-factors, or may be in a denatured, partially denatured, or folded state including any native or unnatural secondary or tertiary structure such as quadruplexes, hairpins, triplexes, three way junctions (3WJs), four way junctions (4WJs), parallel-Y junctions, hairpins, bulge loops, pseudoknots, and internal loops, etc., and any transient forms or structures adopted by the RNA). In some embodiments, the RNA is 20, 22, 50, 75, or 100 or more nucleotides in length. In some embodiments, the RNA is 250 or more nucleotides in length. In some embodiments, the RNA is 350, 450, 500, 600, 750, or 1,000, 2,000, 3,000, 4,000, 5,000, 7,500, 10,000, 15,000, 25,000, 50,000, or more nucleotides in length. In some embodiments, the RNA is between 250 and 1,000 nucleotides in length. In some embodiments, the RNA is a pre-RNA, pre-miRNA, or pretranscript. In some embodiments, the RNA is a non-coding RNA (ncRNA), messenger RNA (mRNA), micro-RNA (miRNA), a ribozyme, riboswitch, lncRNA, lincRNA, snoRNA, snRNA, scaRNA, piRNA, ceRNA, pseudo- gene, viral RNA, fungal RNA, parasitic RNA, or bacterial RNA.

"Steric alteration", "steric modification" or "steric modulation" herein refers to changes in the spatial orientation of chemical moieties with respect to each other. A person of ordinary skill in the art would recognize steric mechanisms include, but are not limited to, steric hindrance, steric shielding, steric attraction, chain crossing, steric repulsions, steric inhibition of resonance, and steric inhibition of protonation.

Any open valency appearing on a carbon, oxygen, sulfur or nitrogen atom in the structures herein indicates the presence of hydrogen, unless indicated otherwise.

The definitions described herein apply irrespective of whether the terms in question appear alone or in combination. It is contemplated that the definitions described herein can be appended to form chemically-relevant combinations, such as *e.g*. "heterocycloalkylaryl", "haloalkylheteroaryl", "arylalkylheterocycloalkyl", or "alkoxyalkyl". The last member of the combination is the radical which is binding to the rest of the molecule. The other members of the combination are attached to the binding radical in reversed order in respect of the literal sequence, *e.g*. the combination arylalkylheterocycloalkyl refers to a heterocycloalkyl-radical which is substituted by an alkyl which is substituted by an aryl.

When indicating the number of substituents, the term "one or more" refers to the range from one substituent to the highest possible number of substitution, i.e. replacement of one hydrogen up to replacement of all hydrogens by substituents.

The term "substituent" denotes an atom or a group of atoms replacing a hydrogen atom on the parent molecule.

The term "substituted" denotes that a specified group bears one or more substituents. Where any group can carry multiple substituents and a variety of possible substituents is provided, the substituents are independently selected and need not to be the same. The term "unsubstituted" means that the specified group bears no substituents. The term "optionally substituted" means that the specified group is unsubstituted or substituted by one or more substituents, independently chosen from the group of possible substituents.

The following abbreviations are used throughout the specification: acetic acid (AcOH); ethyl acetate (EtOAc); butyl alcohol (n-BuOH); 1,2-dichloroethane (DCE); dichloromethane (CH₂Cl₂, DCM); diisopropylethylamine (Diipea); dimethylformamide (DMF); hydrogen chloride (HCl); methanol (MeOH); methoxymethyl bromide (MOMBr); N-methyl-2-pyrrolidone (NMP); methyl Iodide (MeI); n-propanol (n-PrOH); p-methoxybenzyl (PMB); triethylamine (Et₃N); [1,1'-Bis(diphenylphosphino)ferrocene] dichloropalladium(II); (Pd(dppf)Cl₂); sodium ethane thiolate (EtSNa); sodium acetate (NaOAc); sodium hydride (NaH); sodium hydroxide (NaOH); tetrahydropyran (THP); tetrahydrofuran (THF).

As used herein, C₁-Cₓ includes C₁-C₂, C₁-C₃... C₁-Cₓ. By way of example only, a group designated as "C₁-C₄" indicates that there are one to four carbon atoms in the moiety, i.e. groups containing 1 carbon atom, 2 carbon atoms, 3 carbon atoms or 4 carbon atoms. Thus, by way of example only, "C₁-C₄ alkyl" indicates that there are one to four carbon atoms in the alkyl group, *i.e.,* the alkyl group is selected from among methyl, ethyl, propyl, *iso*-propyl, *n*-butyl, *iso*-butyl, *sec*-butyl, and *t*-butyl.

The term "oxo" refers to the =O substituent.

The term "thioxo" refers to the =S substituent.

The term "halo", "halogen", and "halide" are used interchangeably herein and denote fluoro, chloro, bromo, or iodo.

The term "alkyl" refers to a straight or branched hydrocarbon chain radical, having from one to twenty carbon atoms, and which is attached to the rest of the molecule by a single bond. An alkyl comprising up to 10 carbon atoms is referred to as a C₁-C₁₀ alkyl, likewise, for example, an alkyl comprising up to 6 carbon atoms is a C₁-C₆ alkyl. Alkyls (and other moieties defined herein) comprising other numbers of carbon atoms are represented similarly. Alkyl groups include, but are not limited to, C₁-C₁₀ alkyl, C₁-C₉ alkyl, C₁-C₈ alkyl, C₁-C₇ alkyl, C₁-C₆ alkyl, C₁-C₅ alkyl, C₁-C₄ alkyl, C₁-C₃ alkyl, C₁-C₂ alkyl, C₂-C₈ alkyl, C₃-C₈ alkyl and C₄-C₈ alkyl. Representative alkyl groups include, but are not limited to, methyl, ethyl, *n*-propyl, 1-methylethyl (*i*-propyl), *n*-butyl, *i*-butyl, *s*-butyl, *n*-pentyl, 1,1-dimethylethyl (*t*-butyl), 3-methylhexyl, 2-methylhexyl, 1-ethyl-propyl, and the like. In some embodiments, the alkyl is methyl or ethyl. In some embodiments, the alkyl is -CH(CH₃)₂ or -C(CH₃)₃. Unless stated otherwise specifically in the specification, an alkyl group may be optionally substituted. For the avoidance of doubt, the term "alkyl" does not encompass alkenyl or alkynyl groups. "Alkylene" or "alkylene chain" refers to a straight or branched divalent hydrocarbon chain linking the rest of the molecule to a radical group. In some embodiments, the alkylene is -CH₂-, -CH₂CH₂-, or -CH₂CH₂CH₂-. In some embodiments, the alkylene is - CH₂-. In some embodiments, the alkylene is -CH₂CH₂-. In some embodiments, the alkylene is -CH₂CH₂CH₂-. Unless stated otherwise specifically in the specification, an alkylene group may be optionally substituted. For the avoidance of doubt, the term "alkylene" does not encompass alkenylene or alkynylene groups.

The term "alkenyl" refers to a straight or branched hydrocarbon chain radical, having from one to twenty carbon atoms, in which at least one carbon-carbon double bond is present. In one embodiment, an alkenyl group has the formula -C(R)=CR^{a}₂, wherein R^{a} refers to the remaining portions of the alkenyl group, which may be the same or different. In some embodiments, R^{a} is H or an alkyl. In some embodiments, an alkenyl is selected from ethenyl (*i.e.,* vinyl), propenyl (*i.e.,* allyl), butenyl, pentenyl, pentadienyl, and the like. Non-limiting examples of an alkenyl group include -CH=CH₂, -C(CH₃)=CH₂, -CH=CHCH₃, - C(CH₃)=CHCH₃, and -CH₂CH=CH₂. The term "alkenylene" refers to a divalent alkenyl group.

The term "alkynyl" refers to a straight or branched hydrocarbon chain radical, having from one to twenty carbon atoms, in which at least one carbon-carbon triple bond is present. In one embodiment, an alkenyl group has the formula -C≡C-R^{a}, wherein R^{a} refers to the remaining portions of the alkynyl group. In some embodiments, R^{a} is H or an alkyl. In some embodiments, an alkynyl is selected from ethynyl, propynyl, butynyl, pentynyl, hexynyl, and the like. Non-limiting examples of an alkynyl group include -C≡CH, -C≡CCH₃ - C≡CCH₂CH₃, -CH₂C≡CH. The term "alkynylene" refers to a divalent alkynyl group.

The term "alkoxy" refers to a radical of the formula -OR^{a} where R^{a} is an alkyl radical as defined. Unless stated otherwise specifically in the specification, an alkoxy group may be optionally substituted. Representative alkoxy groups include, but are not limited to, methoxy, ethoxy, propoxy, butoxy, pentoxy. In some embodiments, the alkoxy is methoxy. In some embodiments, the alkoxy is ethoxy.

The term "aromatic" refers to a planar ring having a delocalized π-electron system containing 4n+2 π electrons, where n is an integer. Aromatics can be optionally substituted. The term "aromatic" includes both aryl groups (*e.g*., phenyl, naphthalenyl) and heteroaryl groups (*e.g*., pyridinyl, quinolinyl).

The term "aryl" refers to an aromatic ring, wherein each of the atoms forming the ring is a carbon atom. Aryl groups can be optionally substituted. Examples of aryl groups include, but are not limited to phenyl, and naphthyl. In some embodiments, the aryl is phenyl. Depending on the structure, an aryl group can be a monoradical or a diradical (i.e., an arylene group). Unless stated otherwise specifically in the specification, the term "aryl" or the prefix "ar-"(such as in "aralkyl") is meant to include aryl radicals that are optionally substituted. In some embodiments, an aryl group comprises a partially reduced cycloalkyl group defined herein (e.g., 1,2-dihydronaphthalene). In some embodiments, an aryl group comprises a fully reduced cycloalkyl group defined herein (e.g., 1,2,3,4-tetrahydronaphthalene). When aryl comprises a cycloalkyl group, the aryl is bonded to the rest of the molecule through an aromatic ring carbon atom.

The term "haloalkyl" denotes an alkyl group wherein at least one of the hydrogen atoms of the alkyl group has been replaced by same or different halogen atoms, particularly fluoro atoms. Examples of haloalkyl include monofluoro-, difluoro-or trifluoro-methyl, -ethyl or -propyl, for example 3,3,3-trifluoropropyl, 2-fluoroethyl, 2,2,2-trifluoroethyl, fluoromethyl, or trifluoromethyl. The term "perhaloalkyl" denotes an alkyl group where all hydrogen atoms of the alkyl group have been replaced by the same or different halogen atoms.

The term "bicyclic ring system" denotes two rings which are fused to each other via a common single or double bond (annelated bicyclic ring system), via a sequence of three or more common atoms (bridged bicyclic ring system) or via a common single atom (spiro bicyclic ring system). Bicyclic ring systems can be saturated, partially unsaturated, unsaturated or aromatic. Bicyclic ring systems can comprise heteroatoms selected from N, O and S.

The terms "carbocyclic" or "carbocycle" refer to a ring or ring system where the atoms forming the backbone of the ring are all carbon atoms. The term thus distinguishes carbocyclic from "heterocyclic" rings or "heterocycles" in which the ring backbone contains at least one atom which is different from carbon. In some embodiments, at least one of the two rings of a bicyclic carbocycle is aromatic. In some embodiments, both rings of a bicyclic carbocycle are aromatic. Carbocycle includes cycloalkyl and aryl.

The term "cycloalkyl" refers to a monocyclic or polycyclic non-aromatic radical, wherein each of the atoms forming the ring (i.e. skeletal atoms) is a carbon atom. In some embodiments, cycloalkyls are saturated or partially unsaturated. In some embodiments, cycloalkyls are spirocyclic or bridged compounds. In some embodiments, cycloalkyls are fused with an aromatic ring (in which case the cycloalkyl is bonded through a non-aromatic ring carbon atom). Cycloalkyl groups include groups having from 3 to 10 ring atoms. Representative cycloalkyls include, but are not limited to, cycloalkyls having from three to ten carbon atoms, from three to eight carbon atoms, from three to six carbon atoms, or from three to five carbon atoms. Monocyclic cycloalkyl radicals include, for example, cyclopropyl, cyclobutyl, cyclopentyl, cyclohexyl, cycloheptyl, and cyclooctyl. In some embodiments, the monocyclic cycloalkyl is cyclopropyl, cyclobutyl, cyclopentyl or cyclohexyl. In some embodiments, the monocyclic cycloalkyl is cyclopentenyl or cyclohexenyl. In some embodiments, the monocyclic cycloalkyl is cyclopentenyl. Polycyclic radicals include, for example, adamantyl, 1,2-dihydronaphthalenyl, 1,4-dihydronaphthalenyl, tetrainyl, decalinyl, 3,4-dihydronaphthalenyl-1(2H)-one, spiro[2.2]pentyl, norbornyl and bicycle[1.1.1]pentyl. Unless otherwise stated specifically in the specification, a cycloalkyl group may be optionally substituted.

The term "bridged" refers to any ring structure with two or more rings that contains a bridge connecting two bridgehead atoms. The bridgehead atoms are defined as atoms that are the part of the skeletal framework of the molecule and which are bonded to three or more other skeletal atoms. In some embodiments, the bridgehead atoms are C, N, or P. In some embodiments, the bridge is a single atom or a chain of atoms that connects two bridgehead atoms. In some embodiments, the bridge is a valence bond that connects two bridgehead atoms. In some embodiments, the bridged ring system is cycloalkyl. In some embodiments, the bridged ring system is heterocycloalkyl.

The term "fluoroalkyl" refers to an alkyl in which one or more hydrogen atoms are replaced by a fluorine atom. In one aspect, a fluoroalkyl is a C₁-C₆ fluoroalkyl. In some embodiments, a fluoroalkyl is selected from trifluoromethyl, difluoromethyl, fluoromethyl, 2,2,2-trifluoroethyl, 1-fluoromethyl-2-fluoroethyl, and the like.

The term "heterocycloalkyl" refers to a cycloalkyl group that includes at least one heteroatom selected from nitrogen, oxygen, and sulfur. Unless stated otherwise specifically in the specification, the heterocycloalkyl radical may be a monocyclic, or bicyclic ring system, which may include fused (when fused with an aryl or a heteroaryl ring, the heterocycloalkyl is bonded through a non-aromatic ring atom) or bridged ring systems. The nitrogen, carbon or sulfur atoms in the heterocyclyl radical may be optionally oxidized. The nitrogen atom may be optionally quaternized. The heterocycloalkyl radical is partially or fully saturated. Examples of heterocycloalkyl radicals include, but are not limited to, dioxolanyl, thienyl[1,3]dithianyl, tetrahydroquinolyl, tetrahydroisoquinolyl, decahydroquinolyl, decahydroisoquinolyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidonyl, pyrrolidinyl, pyrazolidinyl, quinuclidinyl, thiazolidinyl, tetrahydrofuryl, trithianyl, tetrahydropyranyl, thiomorpholinyl, thiamorpholinyl, 1-oxo-thiomorpholinyl, 1,1-dioxo-thiomorpholinyl. The term heterocycloalkyl also includes all ring forms of carbohydrates, including but not limited to monosaccharides, disaccharides and oligosaccharides. Unless otherwise noted, heterocycloalkyls have from 2 to 12 carbons in the ring. In some embodiments, heterocycloalkyls have from 2 to 10 carbons in the ring. In some embodiments, heterocycloalkyls have from 2 to 10 carbons in the ring and 1 or 2 N atoms. In some embodiments, heterocycloalkyls have from 2 to 10 carbons in the ring and 3 or 4 N atoms. In some embodiments, heterocycloalkyls have from 2 to 12 carbons, 0-2 N atoms, 0-2 O atoms, 0-2 P atoms, and 0-1 S atoms in the ring. In some embodiments, heterocycloalkyls have from 2 to 12 carbons, 1-3 N atoms, 0-1 O atoms, and 0-1 S atoms in the ring. It is understood that when referring to the number of carbon atoms in a heterocycloalkyl, the number of carbon atoms in the heterocycloalkyl is not the same as the total number of atoms (including the heteroatoms) that make up the heterocycloalkyl (i.e. skeletal atoms of the heterocycloalkyl ring). Unless stated otherwise specifically in the specification, a heterocycloalkyl group may be optionally substituted.

The term "heterocycle" or "heterocyclic" refers to heteroaromatic rings (also known as heteroaryls) and heterocycloalkyl rings (also known as heteroalicyclic groups) that includes at least one heteroatom selected from nitrogen, oxygen and sulfur, wherein each heterocyclic group has from 3 to 12 atoms in its ring system, and with the proviso that any ring does not contain two adjacent O or S atoms. Non-aromatic heterocyclic groups (also known as heterocycloalkyls) include rings having 3 to 12 atoms in its ring system and aromatic heterocyclic groups include rings having 5 to 12 atoms in its ring system. The heterocyclic groups include benzo-fused ring systems. Examples of non-aromatic heterocyclic groups are pyrrolidinyl, tetrahydrofuranyl, dihydrofuranyl, tetrahydrothienyl, oxazolidinonyl, tetrahydropyranyl, dihydropyranyl, tetrahydrothiopyranyl, piperidinyl, morpholinyl, thiomorpholinyl, thioxanyl, piperazinyl, aziridinyl, azetidinyl, oxetanyl, thietanyl, homopiperidinyl, oxepanyl, thiepanyl, oxazepinyl, diazepinyl, thiazepinyl, 1,2,3,6-tetrahydropyridinyl, pyrrolin-2-yl, pyrrolin-3-yl, indolinyl, 2H-pyranyl, 4H-pyranyl, dioxanyl, 1,3-dioxolanyl, pyrazolinyl, dithianyl, dithiolanyl, dihydropyranyl, dihydrothienyl, dihydrofuranyl, pyrazolidinyl, imidazolinyl, imidazolidinyl, 3-azabicyclo[3.1.0]hexanyl, 3-azabicyclo[4.1.0]heptanyl, ₃ h-indolyl, indolin-2-onyl, isoindolin-1-onyl, isoindoline-1,3-dionyl, 3,4-dihydroisoquinolin-1(2H)-onyl, 3,4-dihydroquinolin-2(1H)-onyl, isoindoline-1,3-dithionyl, benzo[d]oxazol-2(3H)-onyl, 1H-benzo[d]imidazol-2(3H)-onyl, benzo[d]thiazol-2(3H)-onyl, and quinolizinyl. Examples of aromatic heterocyclic groups are pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, quinolinyl, isoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, oxadiazolyl, thiadiazolyl, furazanyl, benzofurazanyl, benzothiophenyl, benzothiazolyl, benzoxazolyl, quinazolinyl, quinoxalinyl, naphthyridinyl, and furopyridinyl. The foregoing groups are either C-attached (or C-linked) or *N*-attached where such is possible. For instance, a group derived from pyrrole includes both pyrrol-1-yl (N-attached) or pyrrol-3-yl (C-attached). Further, a group derived from imidazole includes imidazol-1-yl or imidazol-3-yl (both *N*-attached) or imidazol-2-yl, imidazol-4-yl or imidazol-5-yl (all C-attached). The heterocyclic groups include benzo-fused ring systems. Non-aromatic heterocycles are optionally substituted with one or two oxo (=O) moieties, such as pyrrolidin-2-one. In some embodiments, at least one of the two rings of a bicyclic heterocycle is aromatic. In some embodiments, both rings of a bicyclic heterocycle are aromatic.

The term "heteroaryl" refers to an aryl group that includes one or more ring heteroatoms selected from nitrogen, oxygen and sulfur. In some embodiments, heteroaryl is monocyclic or bicyclic. Illustrative examples of monocyclic heteroaryls include pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, pyridazinyl, triazinyl, oxadiazolyl, thiadiazolyl, furazanyl, indolizine, indole, benzofuran, benzothiophene, indazole, benzimidazole, purine, quinolizine, quinoline, isoquinoline, cinnoline, phthalazine, quinazoline, quinoxaline, 1,8-naphthyridine, and pteridine. Illustrative examples of monocyclic heteroaryls include pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, pyridazinyl, triazinyl, oxadiazolyl, thiadiazolyl, and furazanyl. Illustrative examples of bicyclic heteroaryls include indolizine, indole, benzofuran, benzothiophene, indazole, benzimidazole, purine, quinolizine, quinoline, isoquinoline, cinnoline, phthalazine, quinazoline, quinoxaline, 1,8-naphthyridine, and pteridine. In some embodiments, heteroaryl is pyridinyl, pyrazinyl, pyrimidinyl, thiazolyl, thienyl, thiadiazolyl or furyl. In some embodiments, a heteroaryl contains 0-6 N atoms in the ring. In some embodiments, a heteroaryl contains 1-4 N atoms in the ring. In some embodiments, a heteroaryl contains 4-6 N atoms in the ring. In some embodiments, a heteroaryl contains 0-4 N atoms, 0-1 O atoms, 0-1 P atoms, and 0-1 S atoms in the ring. In some embodiments, a heteroaryl contains 1-4 N atoms, 0-1 O atoms, and 0-1 S atoms in the ring. In some embodiments, heteroaryl is a C₁-C₉ heteroaryl. In some embodiments, monocyclic heteroaryl is a C₁-C₅ heteroaryl. In some embodiments, monocyclic heteroaryl is a 5-membered or 6-membered heteroaryl. In some embodiments, a heteroaryl group comprises a partially reduced cycloalkyl or heterocycloalkyl group defined herein (e.g., 7,8-dihydroquinoline). In some embodiments, a heteroaryl group comprises a fully reduced cycloalkyl or heterocycloalkyl group defined herein (e.g., 5,6,7,8-tetrahydroquinoline). When heteroaryl comprises a cycloalkyl or heterocycloalkyl group, the heteroaryl is bonded to the rest of the molecule through a heteroaromatic ring carbon or hetero atom.

The term "moiety" refers to a specific segment or functional group of a molecule. Chemical moieties are often recognized chemical entities embedded in or appended to a molecule.

The term "tautomer" refers to a proton shift from one atom of a molecule to another atom of the same molecule. The compounds presented herein may exist as tautomers. Tautomers are compounds that are interconvertible by migration of a hydrogen atom, accompanied by a switch of a single bond and adjacent double bond. In bonding arrangements where tautomerization is possible, a chemical equilibrium of the tautomers will exist. All tautomeric forms of the compounds disclosed herein are contemplated. The exact ratio of the tautomers depends on several factors, including temperature, solvent, and pH. Some examples of tautomeric interconversions include:

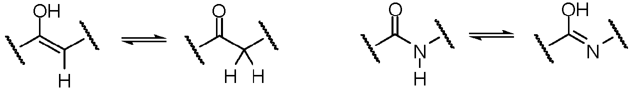

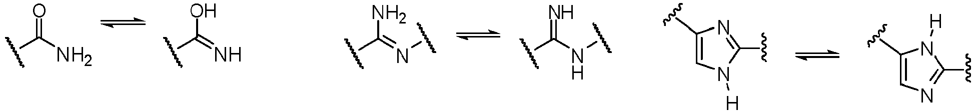

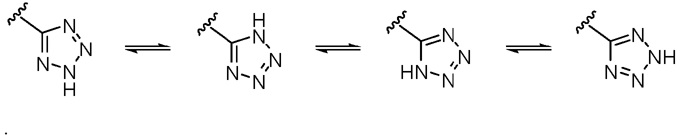

The terms "administer," "administering", "administration," and the like, as used herein, refer to the methods that may be used to enable delivery of compounds or compositions to the desired site of biological action. These methods include, but are not limited to oral routes (p.o.), intraduodenal routes (i.d.), parenteral injection (including intravenous (i.v.), subcutaneous (s.c.), intraperitoneal (i.p.), intramuscular (i.m.), intravascular or infusion (inf.)), topical (top.) and rectal (p.r.) administration. Those of skill in the art are familiar with administration techniques that can be employed with the compounds and methods described herein. In some embodiments, the compounds and compositions described herein are administered orally.

The terms "co-administration" or the like, as used herein, are meant to encompass administration of the selected therapeutic agents to a single patient, and are intended to include treatment regimens in which the agents are administered by the same or different route of administration or at the same or different time.

The terms "effective amount" or "therapeutically effective amount," as used herein, refer to a sufficient amount of an agent or a compound being administered which will relieve to some extent one or more of the symptoms of the disease or condition being treated; for example a reduction and/or alleviation of one or more signs, symptoms, or causes of a disease, or any other desired alteration of a biological system. For example, an "effective amount" for therapeutic uses can be an amount of an agent that provides a clinically significant decrease in one or more disease symptoms. An appropriate "effective" amount may be determined using techniques, such as a dose escalation study, in individual cases.

The terms "enhance" or "enhancing," as used herein, means to increase or prolong either in amount, potency or duration a desired effect. For example, in regard to enhancing splicing of a target, the term "enhancing" can refer to the ability to increase or prolong splicing, either in amount, potency or duration, of a target.

The term "subject" or "patient" encompasses mammals. Examples of mammals include, but are not limited to, any member of the mammalian class: humans, non-human primates such as chimpanzees, and other apes and monkey species; farm animals such as cattle, horses, sheep, goats, swine; domestic animals such as rabbits, dogs, and cats; laboratory animals including rodents, such as rats, mice and guinea pigs, and the like. In one aspect, the mammal is a human. The term "animal" as used herein comprises human beings and non-human animals. In one embodiment, a "non-human animal" is a mammal, for example a rodent such as rat or a mouse. In one embodiment, a non-human animal is a mouse.

The terms "pharmaceutical composition" and "pharmaceutical formulation" (or "formulation") are used interchangeably and denote a mixture or solution comprising a therapeutically effective amount of an active pharmaceutical ingredient together with one or more pharmaceutically acceptable excipients to be administered to a subject, *e.g.,* a human in need thereof.

The term "pharmaceutical combination" as used herein, means a product that results from mixing or combining more than one active ingredient and includes both fixed and non-fixed combinations of the active ingredients. The term "fixed combination" means that the active ingredients, e.g., a compound described herein and a co-agent, are both administered to a patient simultaneously in the form of a single entity or dosage. The term "non-fixed combination" means that the active ingredients, *e.g*. a compound described herein and a co-agent, are administered to a patient as separate entities either simultaneously, concurrently or sequentially with no specific intervening time limits, wherein such administration provides effective levels of the two compounds in the body of the patient. The latter also applies to cocktail therapy, *e.g*., administration of three or more active ingredients.

The term "pharmaceutically acceptable" denotes an attribute of a material which is useful in preparing a pharmaceutical composition that is generally safe, non-toxic, and neither biologically nor otherwise undesirable and is acceptable for veterinary as well as human pharmaceutical use. "Pharmaceutically acceptable" can refer to a material, such as a carrier or diluent, which does not abrogate the biological activity or properties of the compound, and is relatively nontoxic, i.e., the material may be administered to an individual without causing undesirable biological effects or interacting in a deleterious manner with any of the components of the composition in which it is contained.

The terms "pharmaceutically acceptable excipient", "pharmaceutically acceptable carrier" and "therapeutically inert excipient" can be used interchangeably and denote any pharmaceutically acceptable ingredient in a pharmaceutical composition having no therapeutic activity and being non-toxic to the subject administered, such as disintegrators, binders, fillers, solvents, buffers, tonicity agents, stabilizers, antioxidants, surfactants, carriers, diluents, excipients, preservatives or lubricants used in formulating pharmaceutical products.

The term "pharmaceutically acceptable salts" denotes salts which are not biologically or otherwise undesirable. Pharmaceutically acceptable salts include both acid and base addition salts. A "pharmaceutically acceptable salt" can refer to a formulation of a compound that does not cause significant irritation to an organism to which it is administered and/or does not abrogate the biological activity and properties of the compound. In some embodiments, pharmaceutically acceptable salts are obtained by reacting an SMSM compound of any one of Formulas (I)-(Io) with an acid. Pharmaceutically acceptable salts are also obtained by reacting a compound of any one of Formulas (I)-(Io) or with a base to form a salt.

The term "nucleic acid" as used herein generally refers to one or more nucleobases, nucleosides, or nucleotides, and the term includes polynucleobases, polynucleosides, and polynucleotides.

As used herein, a "small molecular weight compound" can be used interchangeably with "small molecule" or "small organic molecule". Small molecules refer to compounds other than peptides or oligonucleotides; and typically have molecular weights of less than about 2000 Daltons, *e.g*., less than about 900 Daltons.

A ribonucleoprotein (RNP) refers to a nucleoprotein that contains RNA. A RNP can be a complex of a ribonucleic acid and an RNA-binding protein. Such a combination can also be referred to as a protein-RNA complex. These complexes can function in a number of biological functions that include, but are not limited to, DNA replication, gene expression, metabolism of RNA, and pre-mRNA splicing. Examples of RNPs include the ribosome, the enzyme telomerase, vault ribonucleoproteins, RNase P, heterogeneous nuclear RNPs (hnRNPs) and small nuclear RNPs (snRNPs).

Nascent RNA transcripts from protein-coding genes and mRNA processing intermediates, collectively referred to as pre-mRNA, are generally bound by proteins in the nuclei of eukaryotic cells. From the time nascent transcripts first emerge from RNA polymerase (*e.g*., RNA polymerase II) until mature mRNAs are transported into the cytoplasm, the RNA molecules are associated with an abundant set of splicing complex components (*e.g*., nuclear proteins and snRNAs). These proteins can be components of hnRNPs, which can contain heterogeneous nuclear RNA (hnRNA) (*e.g*., pre-mRNA and nuclear RNA complexes) of various sizes.

Splicing complex components function in splicing and/or splicing regulation. Splicing complex components can include, but are not limited to, ribonuclear proteins (RNPs), splicing proteins, small nuclear RNAs (snRNAs), small nuclear ribonucleoproteins (snRNPs), and heterogeneous nuclear ribonucleoproteins (hnRNPs). Splicing complex components include, but are not limited to, those that may be required for splicing, such as constitutive splicing, alternative splicing, regulated splicing and splicing of specific messages or groups of messages. A group of related proteins, the serine arginine rich proteins (SR proteins), can function in constitutive pre-mRNA splicing and may also regulate alternative splice-site selection in a concentration-dependent manner. SR proteins typically have a modular structure that consists of one or two RNA-recognition motifs (RRMs) and a C-terminal rich in arginine and serine residues (RS domain). Their activity in alternative splicing may be antagonized by members of the hnRNP A/B family of proteins. Splicing complex components can also include proteins that are associated with one or more snRNAs. SR proteins in human include, but are not limited to, SC35, SRp55, SRp40, SRm300, SFRS10, TASR-1, TASR-2, SF2/ASF, 9G8, SRp75, SRp30c, SRp20 and P54/SFRS11. Other splicing complex components in human that can be involved in splice site selection include, but are not limited to, U2 snRNA auxiliary factors (*e.g*. U2AF65, U2AF35), Urp/U2AF1-RS2, SF1/BBP, CBP80, CBP 20, SF1 and PTB/hnRNP1. hnRNP proteins in humans include, but are not limited to, A1, A2/B1, L, M, K, U, F, H, G, R, I and C1/C2. Human genes encoding hnRNPs include *HNRNPA0, HNRNPA1, HNRNPA1L1, HNRNPA1L2, HNRNPA3, HNRNPA2B1, HNRNPAB, HNRNPB1, HNRNPC, HNRNPCL1, HNRNPD, HNRPDL, HNRNPF, HNRNPH1, HNRNPH2, HNRNPH3, HNRNPK, HNRNPL, HNRPLL, HNRNPM, HNRNPR, HNRNPU, HNRNPUL1, HNRNPUL2, HNRNPUL3,* and *FMR1.* Splicing complex components may be stably or transiently associated with a snRNP or with a transcript.

The term "intron" refers to both the DNA sequence within a gene and the corresponding sequence in the unprocessed RNA transcript. As part of the RNA processing pathway, introns can be removed by RNA splicing either shortly after or concurrent with transcription. Introns are found in the genes of most organisms and many viruses. They can be located in a wide range of genes, including those that generate proteins, ribosomal RNA (rRNA), and transfer RNA (tRNA).

An "exon" can be any part of a gene that encodes a part of the final mature RNA produced by that gene after introns have been removed by RNA splicing. The term "exon" refers to both the DNA sequence within a gene and to the corresponding sequence in RNA transcripts.

A "spliceosome" can be assembled from snRNAs and protein complexes. The spliceosome can remove introns from a transcribed pre-mRNA.

### Small Molecule Splicing Modulators (SMSMs)

The present disclosure provides the unexpected discovery that certain small chemical molecules can modify splicing events in pre-mRNA molecules, herein referred to as small molecule splicing modulators (SMSMs). These SMSMs can modulate specific splicing events in specific pre-mRNA molecules and are useful, therefore, in treating, preventing, or ameliorating a disease or condition associated with a specific RNA. These SMSMs can operate by a variety of mechanisms to modify splicing events. For example, the SMSMs of this disclosure can: 1) interfere with the formation and/or function and/or other properties of splicing complexes, spliceosomes, and/or their components such as hnRNPs, snRNPs, SR-proteins and other splicing factors or elements, resulting in the prevention or induction of a splicing event in a pre-mRNA molecule. As another example; 2) prevent and/or modify post-transcriptional regulation (*e.g*., splicing) of gene products, such as hnRNPs, snRNPs, SR-proteins and other splicing factors, which can subsequently be involved in the formation and/or function of a spliceosome or splicing complex component; 3) prevent and/or modify phosphorylation, glycosylation and/or other modifications of gene products including, but not limited to, hnRNPs, snRNPs, SR-proteins and other splicing factors, which can subsequently be involved in the formation and/or function of a spliceosome or splicing complex component; 4) bind to and/or otherwise affect specific pre-mRNA so that a specific splicing event is prevented or induced, *e.g*., via a mechanism that does not involve base pairing with RNA in a sequence-specific manner. The small molecules of this disclosure are different from and are not related to antisense or antigene oligonucleotides.

Described herein are compounds modifying splicing of gene products for use in the treatment, prevention and/or delay of progression of diseases or conditions.

In one aspect, described herein is a compound that has the structure of Formula (I): 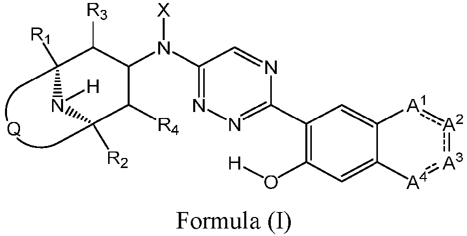
or a pharmaceutically acceptable salt thereof;
wherein:
   Q is C₂-C₄ alkylene optionally substituted with 1, 2, 3, or 4 substituents each independently selected from fluorine, OH, CH₃, and OCH₃; or
   Q is -CH₂OCH₂-, -OCH₂CH₂O-, -OCH₂CH₂OCH₂CH₂O-, or -OCH₂CH₂OCH₂CH₂OCH₂CH₂O-;
   X is hydrogen or CH₃; or
   X is unsubstituted C₃-C₆ cycloalkyl; or
   X is cyclopropyl, cyclobutyl, or cyclopentyl, each substituted with 1, 2, or 3 substituents each independently selected from fluorine, OH, CH₃, and OCH₃;
   each R₁ and R₂ is independently hydrogen, halogen, or CH₃;
   each R₃ and R₄ is independently hydrogen or halogen;
   each A¹, A², and A⁴ is independently N, -NR^{Y1}-, -O-, -S-, or CR^{A1};
   A³ is -NR^{Y1}-, -O-, -S-, or CR^{A1};
   each 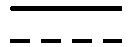 is independently a single or double bond;
   each R^{A1} is independently hydrogen, halogen, =O, or C₁-C₆ alkyl, wherein C₁-C₆ alkyl is optionally substituted with one or more halogen; and
   each R^{Y1} is independently hydrogen or C₁-C₆ alkyl, wherein C₁-C₆ alkyl is optionally substituted with one or more halogen.

In some embodiments, the compound of Formula (I) has the structure of Formula (Ia):

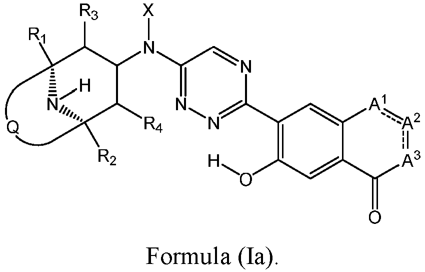

In some embodiments, the compound of Formula (I) has the structure of Formula (Iaa):

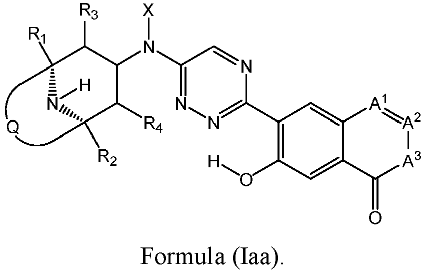

In some embodiments, the compound of Formula (I) has the structure of Formula (Iaa*):

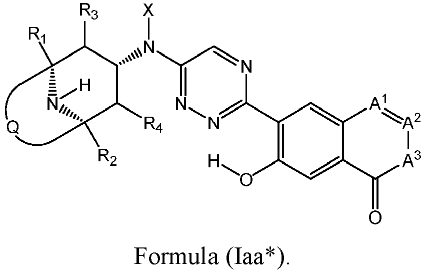

In some embodiments, the compound of Formula (I) has the structure of Formula (Iaaa):

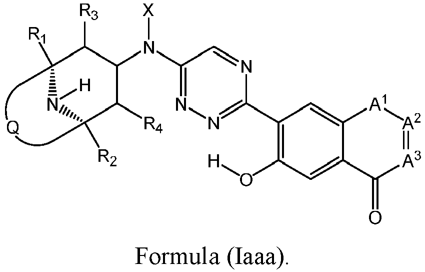

In some embodiments, the compound of Formula (I) has the structure of Formula (Iaaa*):

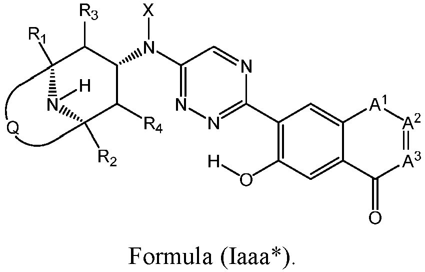

In some embodiments, the compound of Formula (I) has the structure of Formula (Ib):

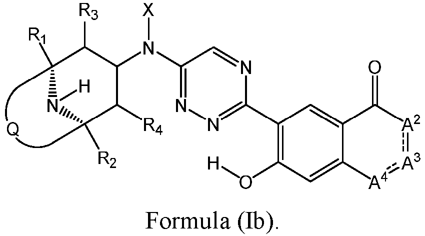

In some embodiments, the compound of Formula (I) has the structure of Formula (Ibb):

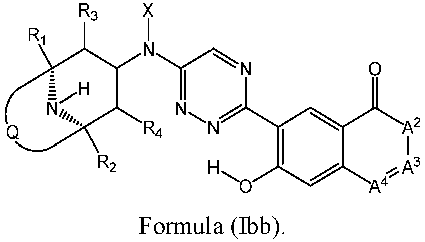

In some embodiments, the compound of Formula (I) has the structure of Formula (Ibb*):

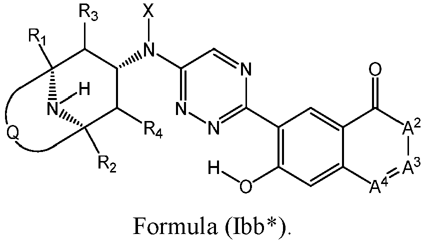

In some embodiments, the compound of Formula (I) has the structure of Formula (Ibbb):

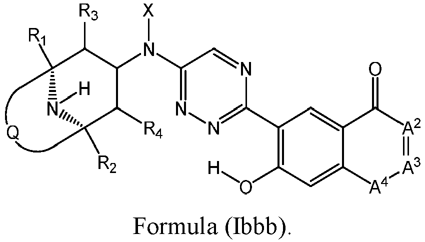

In some embodiments, the compound of Formula (I) has the structure of Formula (Ibbb*):

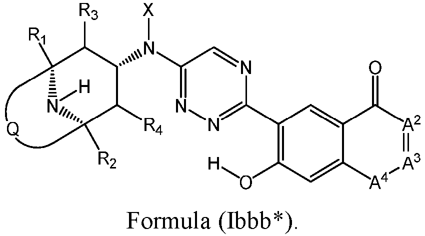

In some embodiments, the compound of Formula (I) has the structure of Formula (Ic):

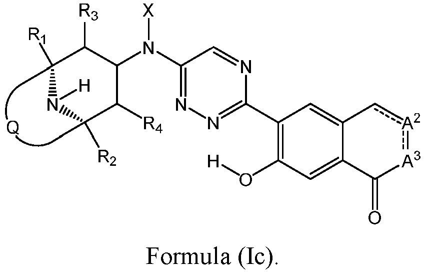

In some embodiments, the compound of Formula (I) has the structure of Formula (Icc):

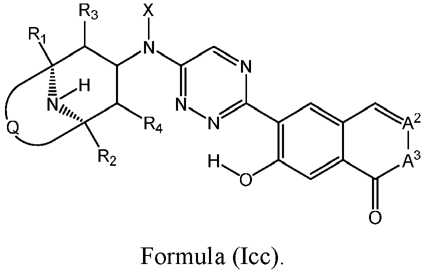

In some embodiments, the compound of Formula (I) has the structure of Formula (Id):

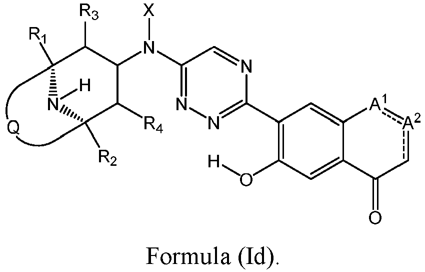

In some embodiments, the compound of Formula (I) has the structure of Formula (Idd):

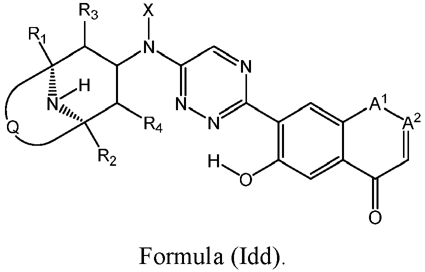

In some embodiments, the compound of Formula (I) has the structure of Formula (Ie):

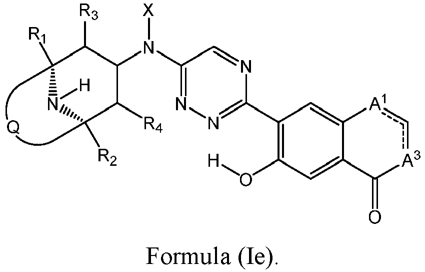

In some embodiments, the compound of Formula (I) has the structure of Formula (Iee):

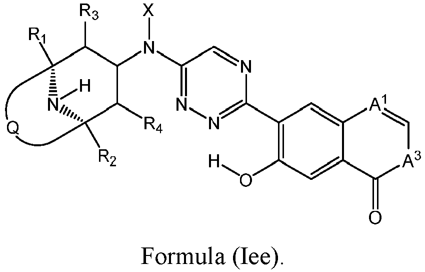

In some embodiments, the compound of Formula (I) has the structure of Formula (Ieee):

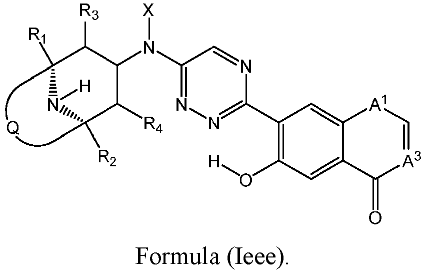

In some embodiments, the compound of Formula (I) has the structure of Formula (If):

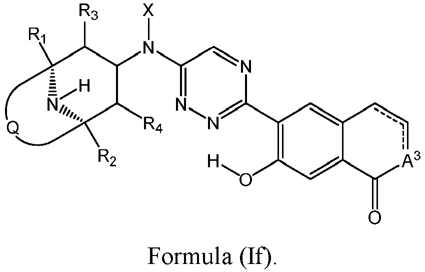

In some embodiments, the compound of Formula (I) has the structure of Formula (Iff):

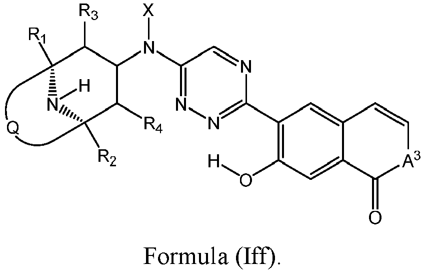

In some embodiments, the compound of Formula (I) has the structure of Formula (Ig):

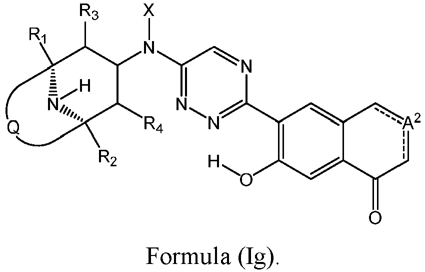

In some embodiments, the compound of Formula (I) has the structure of Formula (Ih):

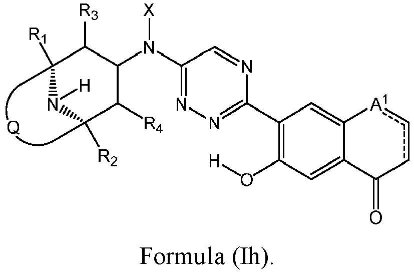

In some embodiments, the compound of Formula (I) has the structure of Formula (Ihh):

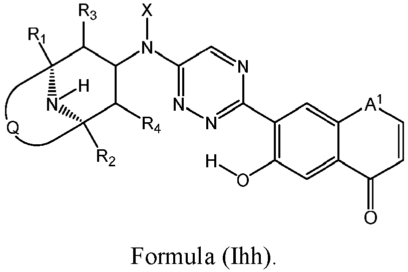

In some embodiments, the compound of Formula (I) has the structure of Formula (Ii):

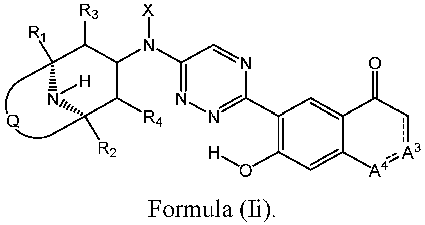

In some embodiments, the compound of Formula (I) has the structure of Formula (Ij):

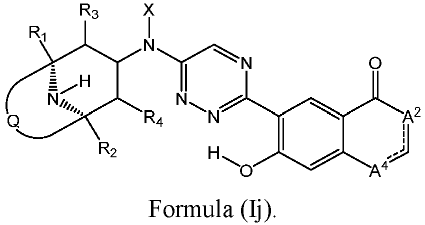

In some embodiments, the compound of Formula (I) has the structure of Formula (Ik):

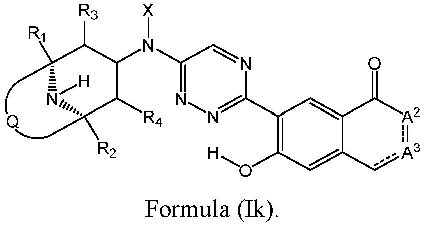

In some embodiments, the compound of Formula (I) has the structure of Formula (Il):

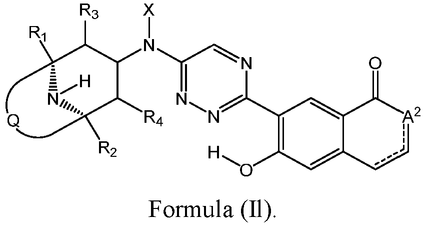

In some embodiments, the compound of Formula (I) has the structure of Formula (Im):

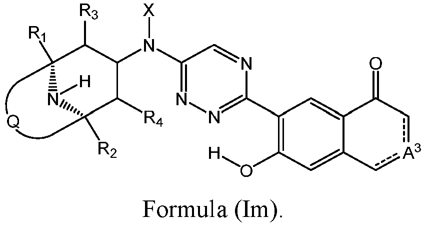

In some embodiments, the compound of Formula (I) has the structure of Formula (In):

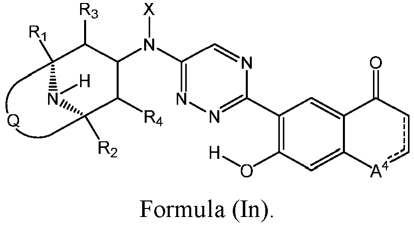

In some embodiments of a compound of Formula (I), Formula (Ia), Formula (Ib), Formula (Ic), Formula (Id), Formula (Ie), Formula (If), Formula (Ig), Formula (Ih), Formula (Ij), Formula (Ij), Formula (Ik), Formula (Il), Formula (Im), or Formula (In), the nitrogen atom bearing X group is in equatorial position with respect to the heterocyclic ring bearing R₁, R₂, R₃, and R₄ substituents.

In some embodiments of a compound of Formula (Iaa), Formula (Iaaa), Formula (Ibb), Formula (Ibbb), Formula (Icc), Formula (Idd), Formula (Iee), Formula (Ieee), Formula (Iff), or Formula (Ihh), the nitrogen atom bearing X group is in equatorial position with respect to the heterocyclic ring bearing R₁, R₂, R₃, and R₄ substituents.

In some embodiments of a compound of Formula (I), Formula (Ia), Formula (Ib), Formula (Ic), Formula (Id), Formula (Ie), Formula (If), Formula (Ig), Formula (Ih), Formula (Ij), Formula (Ij), Formula (Ik), Formula (Il), Formula (Im), Formula (In), Formula (Iaa), Formula (Iaaa), Formula (Ibb), Formula (Ibbb), Formula (Icc), Formula (Idd), Formula (Iee), Formula (Ieee), Formula (Iff), or Formula (Ihh), the nitrogen atom bearing X group and the N-H group are on the same side of a plane. In some embodiments of a compound of Formula (I), Formula (Ia), Formula (Ib), Formula (Ic), Formula (Id), Formula (Ie), Formula (If), Formula (Ig), Formula (Ih), Formula (Ij), Formula (Ij), Formula (Ik), Formula (Il), Formula (Im), Formula (In), Formula (Iaa), Formula (Iaaa), Formula (Ibb), Formula (Ibbb), Formula (Icc), Formula (Idd), Formula (Iee), Formula (Ieee), Formula (Iff), or Formula (Ihh), the nitrogen atom bearing X group and the Q group are on the opposite side of a plane.

In some embodiments, the compound of Formula (I) has the structure of Formula (Io):

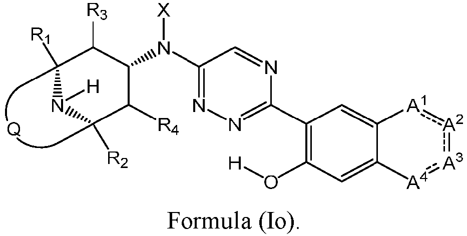

In some embodiments, at least one of R₃ or R₄ is fluorine. In some embodiments, R₃ is fluorine and R₄ is hydrogen. In some embodiments, R₃ is hydrogen and R₄ is fluorine. In some embodiments, R³ is hydrogen or F. In some embodiments, R³ is hydrogen. In some embodiments, R³ is F. In some embodiments, R⁴ is hydrogen or F. In some embodiments, R⁴ is hydrogen. In some embodiments, R⁴ is F.

In some embodiments, A¹ is CH, CF, C(CH₃), N, O, or C(=O). In some embodiments, A¹ is CH. In some embodiments, A¹ is CF. In some embodiments, A¹ is C(CH₃). In some embodiments, A¹ is N. In some embodiments, A¹ is O. In some embodiments, A¹ is C(=O). In some embodiments, A¹ is S. In some embodiments, A¹ is CR^{A1}. In some embodiments, A¹ is CR^{A1} and R^{A1} is H. In some embodiments, A¹ is CR^{A1} and R^{A1} is unsubstituted C₁-C₆ alkyl. In some embodiments, R^{A1} is C₁-C₆ alkyl that is optionally substituted with one or more halogen (such as F). In some embodiments, R^{A1} is C₁-C₃ alkyl that is optionally substituted with one or more F. In some embodiments, one or more hydrogens in R^{A1} are replaced by deuterium. In some embodiments, R^{A1} is H. In some embodiments, R^{A1} is methyl. In some embodiments, R^{A1} is methyl, ethyl, CF₃, CHF₂, or CH₂CF₃. In some embodiments, R^{A1} is CD₃ or CD₂CD₃. In some embodiments, R^{A1} is halogen. In some embodiments, R^{A1} is F. In some embodiments, A¹ is NR^{Y1}. In some embodiments, A¹ is NR^{Y1} and R^{Y1} is H. In some embodiments, A¹ is NR^{Y1} and R^{Y1} is unsubstituted C₁-C₆ alkyl. In some embodiments, R^{Y1} is C₁-C₆ alkyl that is optionally substituted with one or more halogen (such as F). In some embodiments, R^{Y1} is C₁-C₃ alkyl that is optionally substituted with one or more F. In some embodiments, one or more hydrogens in R^{Y1} are replaced by deuterium. In some embodiments, R^{Y1} is H. In some embodiments, R^{Y1} is methyl. In some embodiments, R^{Y1} is methyl, ethyl, CF₃, CHF₂, or CH₂CF₃. In some embodiments, R^{Y1} is CD₃ or CD₂CD₃.

In some embodiments, A² is CH, C(CH₃), N, or C(CH₃). In some embodiments, A² is CH. In some embodiments, A² is C(CH₃). In some embodiments, A² is N. In some embodiments, A² is C(CH₃). In some embodiments, A² is S. In some embodiments, A² is CR^{A1}. In some embodiments, A² is CR^{A1} and R^{A1} is H. In some embodiments, A² is CR^{A1} and R^{A1} is unsubstituted C₁-C₆ alkyl. In some embodiments, R^{A1} is C₁-C₆ alkyl that is optionally substituted with one or more halogen (such as F). In some embodiments, R^{A1} is C₁-C₃ alkyl that is optionally substituted with one or more F. In some embodiments, one or more hydrogens in R^{A1} are replaced by deuterium. In some embodiments, R^{A1} is H. In some embodiments, R^{A1} is methyl. In some embodiments, R^{A1} is methyl, ethyl, CF₃, CHF₂, or CH₂CF₃. In some embodiments, R^{A1} is CD₃ or CD₂CD₃. In some embodiments, R^{A1} is halogen. In some embodiments, R^{A1} is F. In some embodiments, A² is NR^{Y1}. In some embodiments, A² is NR^{Y1} and R^{Y1} is H. In some embodiments, A² is NR^{Y1} and R^{Y1} is unsubstituted C₁-C₆ alkyl. In some embodiments, R^{Y1} is C₁-C₆ alkyl that is optionally substituted with one or more halogen (such as F). In some embodiments, R^{Y1} is C₁-C₃ alkyl that is optionally substituted with one or more F. In some embodiments, one or more hydrogens in R^{Y1} are replaced by deuterium. In some embodiments, R^{Y1} is H. In some embodiments, R^{Y1} is methyl. In some embodiments, R^{Y1} is methyl, ethyl, CF₃, CHF₂, or CH₂CF₃. In some embodiments, R^{Y1} is CD₃ or CD₂CD₃.

In some embodiments, A³ is CH, C(CH₃), or C(CH₃). In some embodiments, A³ is CH. In some embodiments, A³ is C(CH₃). In some embodiments, A³ is C(CH₃). In some embodiments, A³ is S. In some embodiments, A³ is CR^{A1}. In some embodiments, A³ is CR^{A1} and R^{A1} is H. In some embodiments, A³ is CR^{A1} and R^{A1} is unsubstituted C₁-C₆ alkyl. In some embodiments, R^{A1} is C₁-C₆ alkyl that is optionally substituted with one or more halogen (such as F). In some embodiments, R^{A1} is C₁-C₃ alkyl that is optionally substituted with one or more F. In some embodiments, one or more hydrogens in R^{A1} are replaced by deuterium. In some embodiments, R^{A1} is H. In some embodiments, R^{A1} is methyl. In some embodiments, R^{A1} is methyl, ethyl, CF₃, CHF₂, or CH₂CF₃. In some embodiments, R^{A1} is CD₃ or CD₂CD₃. In some embodiments, R^{A1} is halogen. In some embodiments, R^{A1} is F. In some embodiments, A³ is NR^{Y1}. In some embodiments, A³ is NR^{Y1} and R^{Y1} is H. In some embodiments, A³ is NR^{Y1} and R^{Y1} is unsubstituted C₁-C₆ alkyl. In some embodiments, R^{Y1} is C₁-C₆ alkyl that is optionally substituted with one or more halogen (such as F). In some embodiments, R^{Y1} is C₁-C₃ alkyl that is optionally substituted with one or more F. In some embodiments, one or more hydrogens in R^{Y1} are replaced by deuterium. In some embodiments, R^{Y1} is H. In some embodiments, R^{Y1} is methyl. In some embodiments, R^{Y1} is methyl, ethyl, CF₃, CHF₂, or CH₂CF₃. In some embodiments, R^{Y1} is CD₃ or CD₂CD₃. In some embodiments, A³ is N(CH₃).

In some embodiments, A⁴ is CH, C(CH₃), N, O, or C(=O). In some embodiments, A⁴ is CH. In some embodiments, A⁴ is C(CH₃). In some embodiments, A⁴ is N. In some embodiments, A⁴ is O. In some embodiments, A⁴ is C(=O). In some embodiments, A⁴ is S. In some embodiments, A⁴ is CR^{A1}.

In some embodiments, A⁴ is CR^{A1}. and R^{A1} is H. In some embodiments, A⁴ is CR^{A1}. and R^{A1} is unsubstituted C₁-C₆ alkyl. In some embodiments, R^{A1} is C₁-C₆ alkyl that is optionally substituted with one or more halogen (such as F). In some embodiments, R^{A1} is C₁-C₃ alkyl that is optionally substituted with one or more F. In some embodiments, one or more hydrogens in R^{A1} are replaced by deuterium. In some embodiments, R^{A1} is H. In some embodiments, R^{A1} is methyl. In some embodiments, R^{A1} is methyl, ethyl, CF₃, CHF₂, or CH₂CF₃. In some embodiments, R^{A1} is CD₃ or CD₂CD₃. In some embodiments, R^{A1} is halogen. In some embodiments, R^{A1} is F. In some embodiments, A⁴ is NR^{Y1}. In some embodiments, A⁴ is NR^{Y1} and R^{Y1} is H. In some embodiments, A⁴ is NR^{Y1} and R^{Y1} is unsubstituted C₁-C₆ alkyl. In some embodiments, R^{Y1} is C₁-C₆ alkyl that is optionally substituted with one or more halogen (such as F). In some embodiments, R^{Y1} is C₁-C₃ alkyl that is optionally substituted with one or more F. In some embodiments, one or more hydrogens in R^{Y1} are replaced by deuterium. In some embodiments, R^{Y1} is H. In some embodiments, R^{Y1} is methyl. In some embodiments, R^{Y1} is methyl, ethyl, CF₃, CHF₂, or CH₂CF₃. In some embodiments, R^{Y1} is CD₃ or CD₂CD₃.

In some embodiments, one of A¹ , A², A³, and A⁴ is C(=O).

In some embodiments, the compound of Formula (I) comprises at least 20 carbon atoms, 5 nitrogen atoms and 1 fluorine atom. In some embodiments, the compound of Formula (I) comprises at least 18 carbon atoms (e.g., 18, 19 or 20 carbon atoms), 6 nitrogen atoms and 1 fluorine atom. In some embodiments, the compound of Formula (I) comprises at least 18 carbon atoms (e.g., 18, 19 or 20 carbon atoms), 7 nitrogen atoms and 1 fluorine atom.

In some embodiments, X is hydrogen. In some embodiments, X is -CH₃. In some embodiments, X is -CD₃. In some embodiments, X is unsubstituted C₃-C₆ cycloalkyl. In some embodiments, X is unsubstituted cyclopropyl, unsubstituted cyclobutyl, or unsubstituted cyclopentyl. In some embodiments, X is cyclopropyl, cyclobutyl, or cyclopentyl, each substituted with 1, 2, or 3 substituents each independently selected from fluorine, OH, CH₃, and OCH₃. In some embodiments, X is cyclopropyl.

In some embodiments, R₁ is hydrogen or CH₃. In some embodiments, R₁ is hydrogen. In some embodiments, R₁ is CH₃.

In some embodiments, R₂ is hydrogen or CH₃. In some embodiments, R₂ is hydrogen. In some embodiments, R₂ is CH₃.

In some embodiments, Q is unsubstituted C₂-C₄ alkylene. In some embodiments, Q is C₂-C₄ alkylene substituted with 1, 2, 3, or 4 substituents each independently selected from fluorine, OH, CH₃, and OCH₃. In some embodiments, Q is -CH₂CH₂-. In some embodiments, Q is -CH₂CH₂CH₂-. In some embodiments, Q is -CH₂OCH₂-. In some embodiments, Q is C₂-C₄ alkylene optionally substituted by halogen. In some embodiments, Q is C₂-C₄ alkylene optionally substituted by one or more F.

In some embodiments, 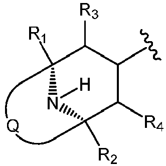 is selected from the group consisting of

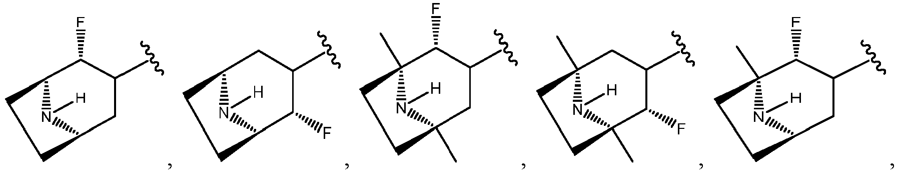

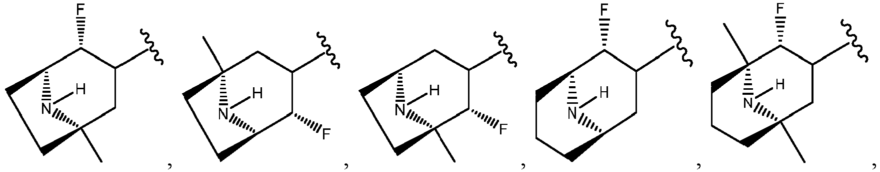

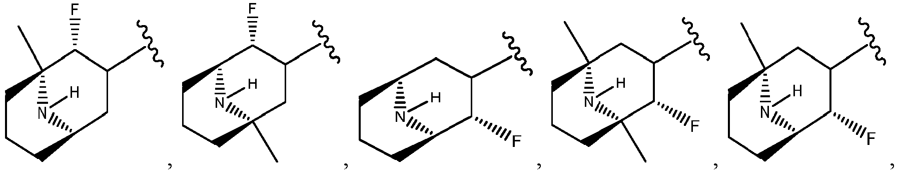

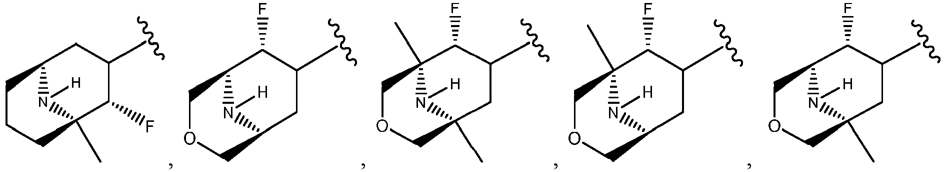

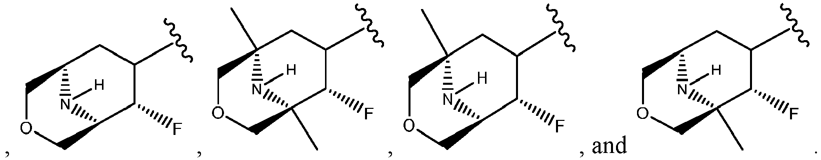

In some embodiments, 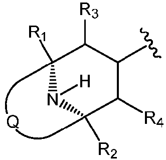 is selected from the group consisting of 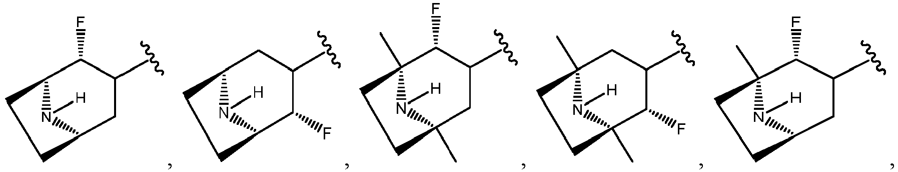 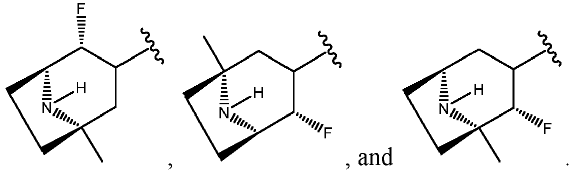 In some embodiments, 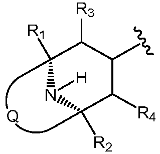 is selected from the group consisting of 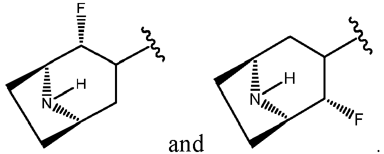 In some embodiments, 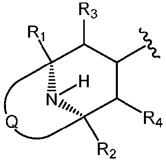 is selected from the group consisting of 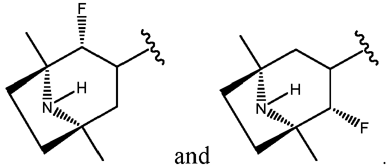 In some embodiments, 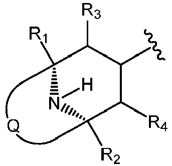 is selected from the group consisting of

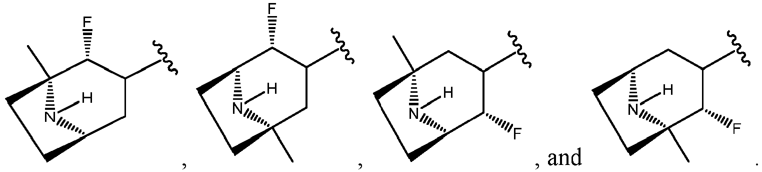

In some embodiments, 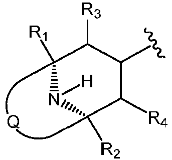 is selected from the group consisting of 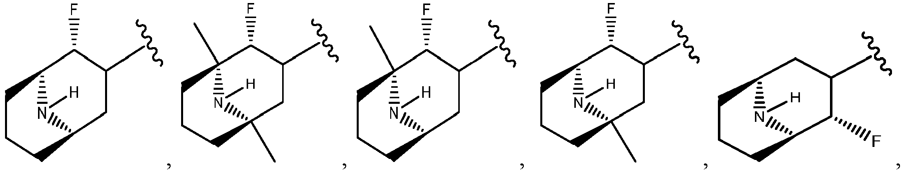 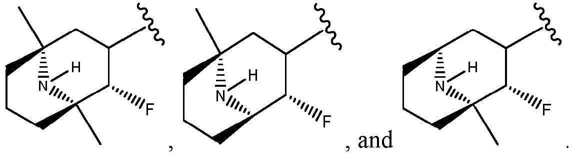 In some embodiments, 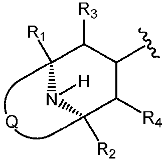 is selected from the group consisting of 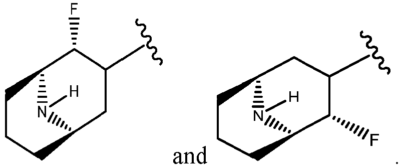 In some embodiments, 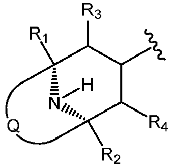 is selected from the group consisting of 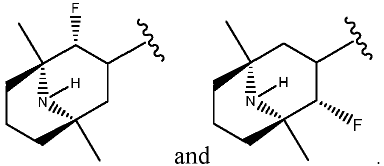 In some embodiments, 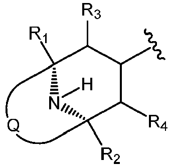 is selected from the group consisting of

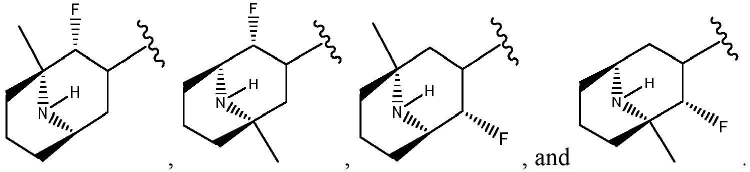

In some embodiments, the compound of Formula (I) is selected from the group consisting of

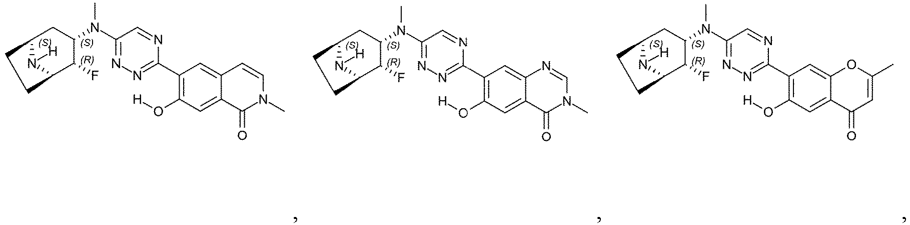

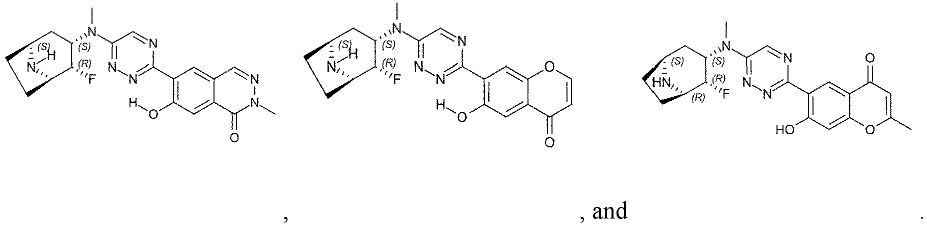

In some embodiments, the compound of Formula I is selected from the compounds in Table 1. In some embodiments, the compound of Formula I is selected from the compounds in Table 2. In some embodiments, the compound of Formula (I), is: 6-(6-{[(2R,3S,5S)-2-fluoro-8-azabicyclo[3.2.1]octan-3-yl](methyl)amino}-1,2,4-triazin-3-yl)-7-hydroxy-2-methyl-1,2-dihydroisoquinolin-1-one; 7-(6-f [(2R,3S,SS)-2-fluoro-8-azabicyclo[3.2.1]octan-3-yl](methyl)amino}-1,2,4-triazin-3-yl)-6-hydroxy-3-methyl-3,4-dihydroquinazolin-4-one; 7-(6-{[(2R,3S,5S)-2-fluoro-8-azabicyclo[3.2.1]octan-3-yl](methyl)amino}-1,2,4-triazin-3-yl)-6-hydroxy-2-methyl-4H-chromen-4-one; 6-(6-{[(2R,3S,5S)-2-fluoro-8-azabicyclo[3.2.1]octan-3-yl](methyl)amino}-1,2,4-triazin-3-yl)-7-hydroxy-2-methyl-1,2-dihydrophthalazin-1-one; 7-(6-{[(2R,3S,SS)-2-fluoro-8-azabicyclo[3.2.1]octan-3-yl](methyl)amino}-1,2,4-triazin-3-yl)-6-hydroxy-4H-chromen-4-one; or 6-(6-{[(1R,2R,3S,SS)-2-fluoro-8-azabicyclo[3.2.1]octan-3-yl](methyl)amino}-1,2,4-triazin-3-yl)-7-hydroxy-2-methyl-4H-chromen-4-one. In some embodiments, the compound of Formula (I) is 6-(6-[[(1S,2S,3R,5R)-2-fluoro-8-azabicyclo[3.2.1]octan-3-yl](methyl)amino]-1,2,4-triazin-3-yl)-7-hydroxy-2-methylphthalazin-1-one. In some embodiments, the compound of Formula (I) is 6-(6-[[(1R,2R,3S,SS)-2-fluoro-8-azabicyclo[3.2.1]octan-3-yl](methyl)amino]-1,2,4-triazin-3-yl)-7-hydroxy-2-methylphthalazin-1-one. In some embodiments, the compound of Formula (I) is 6-(6-(((1S,2S,3R,SR)-2-fluoro-8-azabicyclo[3.2.1]octan-3-yl)(methyl)amino)-1,2,4-triazin-3-yl)-7-hydroxy-2-methyl-4H-chromen-4-one. In some embodiments, the compound of Formula (I) is 6-(6-(((1R,2R,3S,5S)-2-fluoro-8-azabicyclo[3.2.1]octan-3-yl)(methyl)amino)-1,2,4-triazin-3-yl)-7-hydroxy-2-methyl-4H-chromen-4-one. In some embodiments, the compound of Formula (I) is 7-(6-(((1S,2S,3R,5R)-2-fluoro-8-azabicyclo[3.2.1]octan-3-yl)(methyl)amino)-1,2,4-triazin-3-yl)-6-hydroxy-3-methylquinazolin-4(3H)-one. In some embodiments, the compound of Formula (I) is 7-(6-(((1R,2R,3S,5S)-2-fluoro-8-azabicyclo[3.2.1]octan-3-yl)(methyl)amino)-1,2,4-triazin-3-yl)-6-hydroxy-3-methylquinazolin-4(3H)-one. In some embodiments, the compound of Formula (I) is 6-(6-(((1S,2S,3R,5R)-2-fluoro-8-azabicyclo[3.2.1]octan-3-yl)(methyl)amino)-1,2,4-triazin-3-yl)-7-hydroxy-2-methylisoquinolin-1(2H)-one. In some embodiments, the compound of Formula (I) is 6-(6-(((1R,2R,3S,5S)-2-fluoro-8-azabicyclo[3.2.1]octan-3-yl)(methyl)amino)-1,2,4-triazin-3-yl)-7-hydroxy-2-methylisoquinolin-1(2H)-one. In some embodiments, the compound of Formula (I) is 7-(6-(((1S,2S,3R,5R)-2-fluoro-8-azabicyclo[3.2.1]octan-3-yl)(methyl)amino)-1,2,4-triazin-3-yl)-6-hydroxy-2-methyl-4H-chromen-4-one. In some embodiments, the compound of Formula (I) is 7-(6-(((1R,2R,3S,5S)-2-fluoro-8-azabicyclo[3.2.1]octan-3-yl)(methyl)amino)-1,2,4-triazin-3-yl)-6-hydroxy-2-methyl-4H-chromen-4-one. In some embodiments, the compound of Formula (I) is 7-(6-(((1S,2S,3R,5R)-2-fluoro-8-azabicyclo[3.2.1]octan-3-yl)(methyl)amino)-1,2,4-triazin-3-yl)isoquinolin-6-ol. In some embodiments, the compound of Formula (I) is 7-(6-(((1R,2R,3S,5S)-2-fluoro-8-azabicyclo[3.2.1]octan-3-yl)(methyl)amino)-1,2,4-triazin-3-yl)isoquinolin-6-ol. In some embodiments, the compound of Formula (I) is 4-Fluoro-6-(6-{[(2R,3S,SS)-2-fluoro-8-azabicyclo[3.2.1]octan-3-yl](methyl)amino}-1,2,4-triazin-3-yl)-7-hydroxy-2-methylisoquinolin-1-one. In some embodiments, the compound of Formula (I) is 2-Ethyl-4-fluoro-6-(6-{[(2R,3S,5S)-2-fluoro-8-azabicyclo[3.2.1]octan-3-yl(methyl)amino}-1,2,4-triazin-3-yl)-7-hydroxyisoquinolin-1-one. In some embodiments, described herein is a salt or solvate of any of the described compound. In some embodiments, described herein is a pharmaceutically acceptable salt or solvate of any of the described compound.

Described herein is a method of modulating splicing comprising contacting a compound of any one of the preceding claims to cells, wherein the compound modulates splicing at a splice site sequence of a pre-mRNA that encodes a mRNA, wherein the mRNA encodes a target protein or a functional RNA.

In one aspect, disclosed herein is a compound, or a pharmaceutically acceptable salt thereof, or a pharmaceutical composition, for use in a method of treating a disease or condition comprising administering a compound of the present invention.

In some embodiments, an SMSM described herein, possesses one or more stereocenters and each stereocenter exists independently in either the R or S configuration. The compounds presented herein include all diastereomeric, enantiomeric, and epimeric forms as well as the appropriate mixtures thereof. The compounds and methods provided herein include all cis, trans, syn, anti, entgegen (E), and zusammen (Z) isomers as well as the appropriate mixtures thereof. In certain embodiments, compounds described herein are prepared as their individual stereoisomers by reacting a racemic mixture of the compound with an optically active resolving agent to form a pair of diastereoisomeric compounds/salts, separating the diastereomers and recovering the optically pure enantiomers. In some embodiments, resolution of enantiomers is carried out using covalent diastereomeric derivatives of the compounds described herein. In another embodiment, diastereomers are separated by separation/resolution techniques based upon differences in solubility. In other embodiments, separation of stereoisomers is performed by chromatography or by the forming diastereomeric salts and separation by recrystallization, or chromatography, or any combination thereof. Jean Jacques, Andre Collet, Samuel H. Wilen, "Enantiomers, Racemates and Resolutions", John Wiley And Sons, Inc., 1981. In one aspect, stereoisomers are obtained by stereoselective synthesis.

In some embodiments, compounds described herein are prepared as prodrugs. A "prodrug" refers to an agent that is converted into the parent drug *in vivo.* Prodrugs are often useful because, in some situations, they may be easier to administer than the parent drug. They may, for instance, be bioavailable by oral administration whereas the parent is not. The prodrug may also have improved solubility in pharmaceutical compositions over the parent drug. In some embodiments, the design of a prodrug increases the effective water solubility. An example, without limitation, of a prodrug is a compound described herein, which is administered as an ester (the "prodrug") to facilitate transmittal across a cell membrane where water solubility is detrimental to mobility but which then is metabolically hydrolyzed to the carboxylic acid, the active entity, once inside the cell where water-solubility is beneficial. A further example of a prodrug might be a short peptide (polyaminoacid) bonded to an acid group where the peptide is metabolized to reveal the active moiety. In certain embodiments, upon *in vivo* administration, a prodrug is chemically converted to the biologically, pharmaceutically or therapeutically active form of the compound. In certain embodiments, a prodrug is enzymatically metabolized by one or more steps or processes to the biologically, pharmaceutically or therapeutically active form of the compound.

In one aspect, prodrugs are designed to alter the metabolic stability or the transport characteristics of a drug, to mask side effects or toxicity, to improve the flavor of a drug or to alter other characteristics or properties of a drug. By virtue of knowledge of pharmacokinetic, pharmacodynamic processes and drug metabolism *in vivo,* once a pharmaceutically active compound is known, the design of prodrugs of the compound is possible. (see, for example, Nogrady (1985) Medicinal Chemistry A Biochemical Approach, Oxford University Press, New York, pages 388-392; Silverman (1992), The Organic Chemistry of Drug Design and Drug Action, Academic Press, Inc., San Diego, pages 352-401, Rooseboom et al., Pharmacological Reviews, 56:53-102, 2004; Aesop Cho, "Recent Advances in Oral Prodrug Discovery", Annual Reports in Medicinal Chemistry, Vol. 41, 395-407, 2006; T. Higuchi and V. Stella, Pro-drugs as Novel Delivery Systems, Vol. 14 of the A.C.S. Symposium Series).

In some cases, some of the herein-described compounds may be a prodrug for another derivative or active compound.

In some embodiments, sites on the aromatic ring portion of compounds described herein are susceptible to various metabolic reactions, therefore incorporation of appropriate substituents on the aromatic ring structures will reduce, minimize or eliminate this metabolic pathway. In specific embodiments, the appropriate substituent to decrease or eliminate the susceptibility of the aromatic ring to metabolic reactions is, by way of example only, a halogen, or an alkyl group.

In another embodiment, the compounds described herein are labeled isotopically (e.g. with a radioisotope) or by another other means, including, but not limited to, the use of chromophores or fluorescent moieties, bioluminescent labels, or chemiluminescent labels.

Compounds described herein include isotopically-labeled compounds, which are identical to those recited in the various formulae and structures presented herein, but for the fact that one or more atoms are replaced by an atom having an atomic mass or mass number different from the atomic mass or mass number usually found in nature. Examples of isotopes that can be incorporated into the present compounds include isotopes of hydrogen, carbon, nitrogen, oxygen, sulfur, fluorine and chlorine, such as, for example, ²H, ³H, ¹³C, ¹⁴C, ¹⁵N, ¹⁸O, ¹⁷O, ³⁵S, ¹⁸F, and ³⁶Cl. In one aspect, isotopically-labeled compounds described herein, for example those into which radioactive isotopes such as ³H and ¹⁴C are incorporated, are useful in drug and/or substrate tissue distribution assays. In one aspect, substitution with isotopes such as deuterium affords certain therapeutic advantages resulting from greater metabolic stability, such as, for example, increased *in vivo* half-life or reduced dosage requirements.

In additional or further embodiments, the compounds described herein are metabolized upon administration to an organism in need to produce a metabolite that is then used to produce a desired effect, including a desired therapeutic effect.

Compounds described herein may be formed as, and/or used as, pharmaceutically acceptable salts. The type of pharmaceutical acceptable salts, include, but are not limited to: (1) acid addition salts, formed by reacting the free base form of the compound with a pharmaceutically acceptable: inorganic acid, such as, for example, hydrochloric acid, hydrobromic acid, sulfuric acid, phosphoric acid, metaphosphoric acid, and the like; or with an organic acid, such as, for example, acetic acid, propionic acid, hexanoic acid, cyclopentanepropionic acid, glycolic acid, pyruvic acid, lactic acid, malonic acid, succinic acid, malic acid, maleic acid, fumaric acid, trifluoroacetic acid, tartaric acid, citric acid, benzoic acid, 3-(4-hydroxybenzoyl)benzoic acid, cinnamic acid, mandelic acid, methanesulfonic acid, ethanesulfonic acid, 1,2-ethanedisulfonic acid, 2-hydroxyethanesulfonic acid, benzenesulfonic acid, toluenesulfonic acid, 2-naphthalenesulfonic acid, 4-methylbicyclo-[2.2.2]oct-2-ene-1-carboxylic acid, glucoheptonic acid, 4,4'-methylenebis-(3-hydroxy-2-ene-1-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, butyric acid, phenylacetic acid, phenylbutyric acid, valproic acid, and the like; (2) salts formed when an acidic proton present in the parent compound is replaced by a metal ion, *e.g.,* an alkali metal ion (*e.g.* lithium, sodium, potassium), an alkaline earth ion (*e.g.* magnesium, or calcium), or an aluminum ion. In some cases, compounds described herein may coordinate with an organic base, such as, but not limited to, ethanolamine, diethanolamine, triethanolamine, tromethamine, N-methylglucamine, dicyclohexylamine, tris(hydroxymethyl)methylamine. In other cases, compounds described herein may form salts with amino acids such as, but not limited to, arginine, lysine, and the like. Acceptable inorganic bases used to form salts with compounds that include an acidic proton, include, but are not limited to, aluminum hydroxide, calcium hydroxide, potassium hydroxide, sodium carbonate, sodium hydroxide, and the like.

It should be understood that a reference to a pharmaceutically acceptable salt includes the solvent addition forms, particularly solvates. Solvates contain either stoichiometric or non-stoichiometric amounts of a solvent, and may be formed during the process of crystallization with pharmaceutically acceptable solvents such as water, ethanol, and the like. Hydrates are formed when the solvent is water, or alcoholates are formed when the solvent is alcohol. In some embodiments, solvates of compounds described herein are conveniently prepared or formed during the processes described herein. In addition, the compounds provided herein can exist in unsolvated as well as solvated forms. In general, the solvated forms are considered equivalent to the unsolvated forms for the purposes of the compounds and methods provided herein.

In some embodiments, an SMSM has a molecular weight of at most about 2000 Daltons, 1500 Daltons, 1000 Daltons or 900 Daltons. In some embodiments, an SMSM has a molecular weight of at least 100 Daltons, 200 Daltons, 300 Daltons, 400 Daltons or 500 Daltons. In some embodiments, an SMSM does not comprise a phosphodiester linkage.

### Methods of Making Compounds

Compounds described herein can be synthesized using standard synthetic techniques or using methods known in the art in combination with methods described herein. Unless otherwise indicated, conventional methods of mass spectroscopy, NMR, HPLC, protein chemistry, biochemistry, recombinant DNA techniques and pharmacology can be employed. Compounds can be prepared using standard organic chemistry techniques such as those described in, for example, March's Advanced Organic Chemistry, 6th Edition, John Wiley and Sons, Inc. Alternative reaction conditions for the synthetic transformations described herein may be employed such as variation of solvent, reaction temperature, reaction time, as well as different chemical reagents and other reaction conditions. The starting materials can be available from commercial sources or can be readily prepared. By way of example only, provided are schemes for preparing the exemplary SMSMs.

A scheme for preparing an SMSM described herein is Scheme 1. 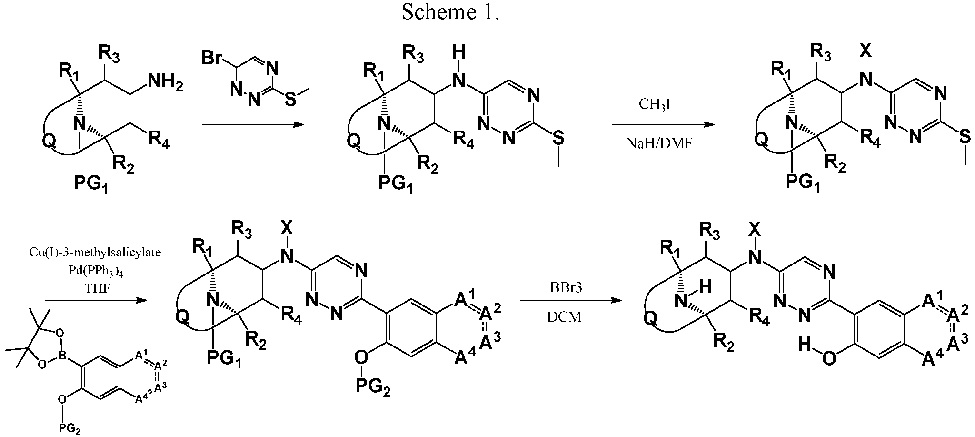 wherein, PG₁ is a suitable protecting group 1 and PG₂ is a suitable protecting group 2.
In some embodiments, PG₁ and PG₂ are as described in the examples.

In some embodiments, an SMSM compound described herein is selected from **Table 1 or Table 2** (except Compounds 21, 22, 39, 40, 43, 44, 47, 48, 133, and 134, which are included as reference Examples). Compounds in **Table 1 and Table 2** can be made using the procedures outlined in General Scheme 1 above and Examples 1-8 below.

**Table 1 - Exemplary Compounds**

| Compound | Structure |
|---|---|
| 1A & 1B | 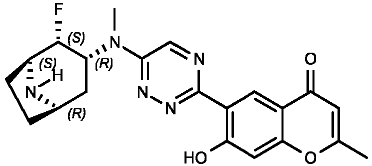 |
| | 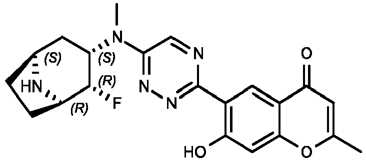 |
| 2A & 2B | 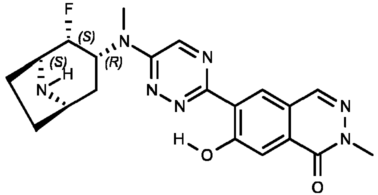 |
| | 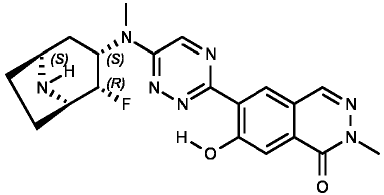 |
| 3A & 3B | 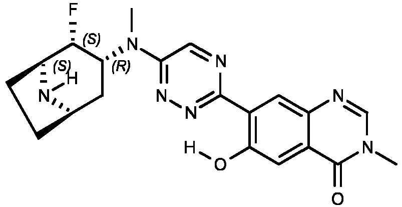 |
| | 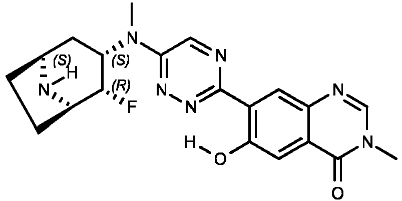 |
| 4A & 4B | 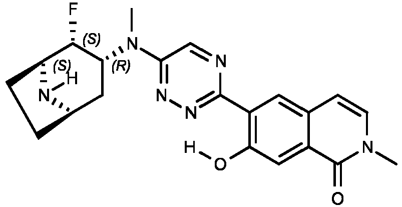 |
| | 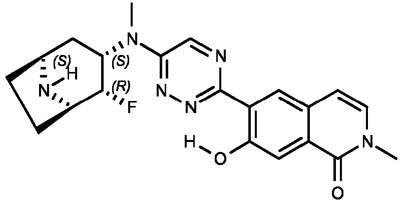 |
| 5A & 5B | 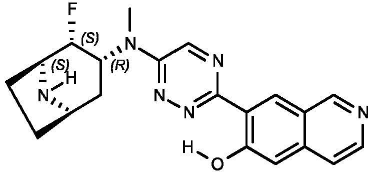 |
| | 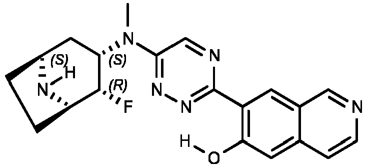 |
| 6A & 6B | 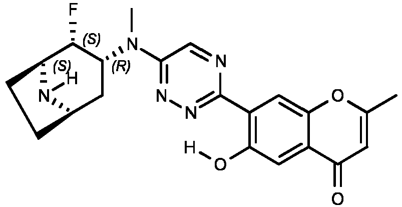 |
| | 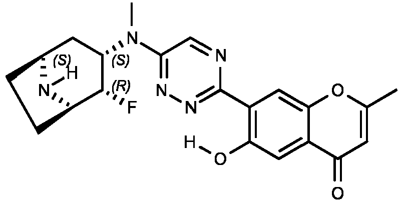 |
| 7A & 7B | 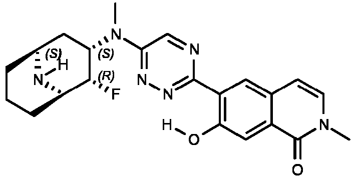 |
| | 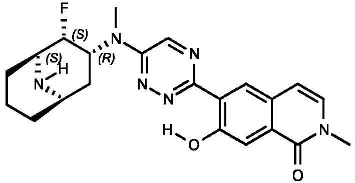 |
| 8A & 8B | 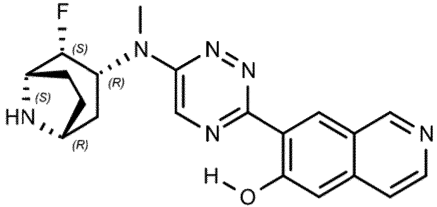 |
| | 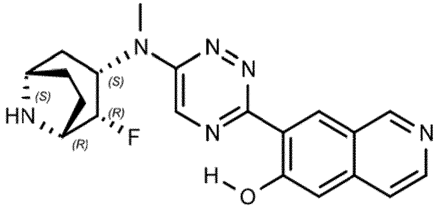 |

**Table 2 - Exemplary Compounds**

| (Compounds 21, 22, 39, 40, 43, 44, 47, 48, 133, and 134 are included as reference Examples) | | | |
|---|---|---|---|
| **Compound** | **Structure** | **Compound** | **Structure** |
| 9 | 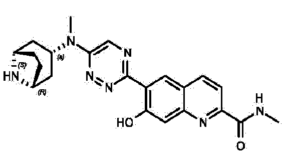 | 122 | 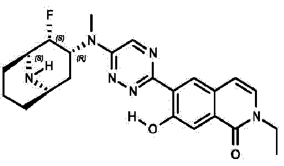 |
| 10 | 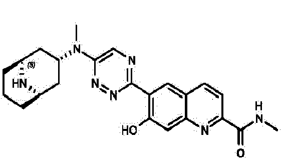 | 123 | 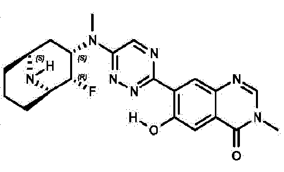 |
| 12 | 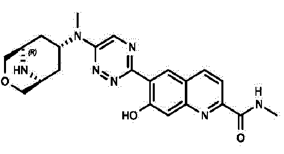 | 124 | 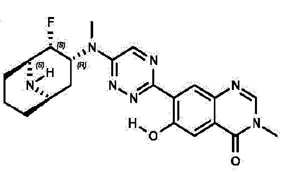 |
| 13 | 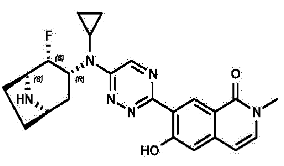 | 125 | 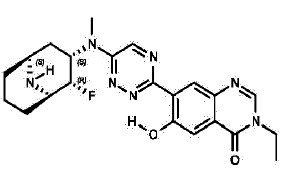 |
| 14 | 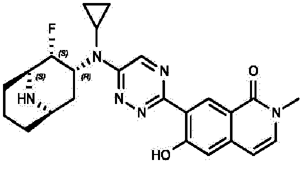 | 126 | 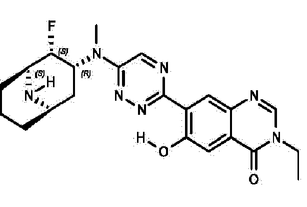 |
| 15 | 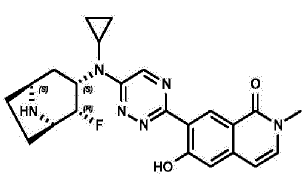 | 127 | 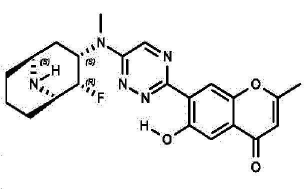 |
| 16 | 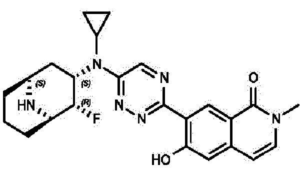 | 128 | 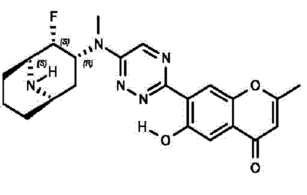 |
| 17 | 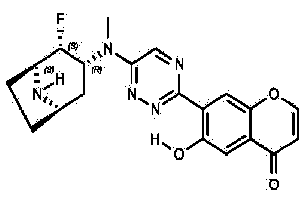 | 129 | 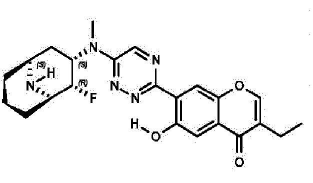 |
| 18 | 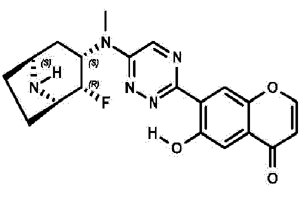 | 130 | 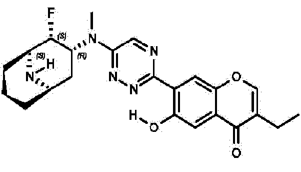 |
| 19 | 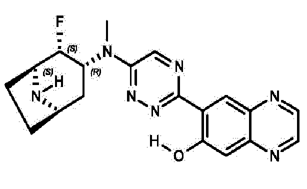 | 131 | 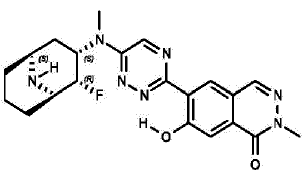 |
| 20 | 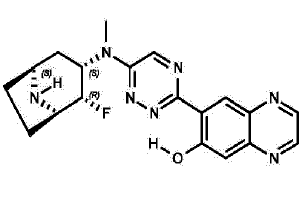 | 132 | 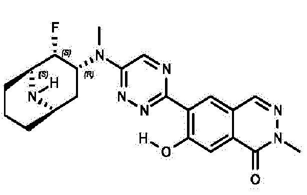 |
| 21 | 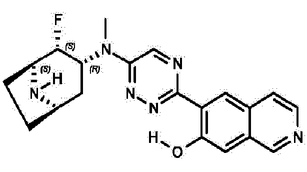 | 133 | 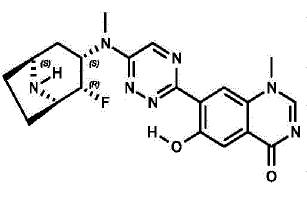 |
| 22 | 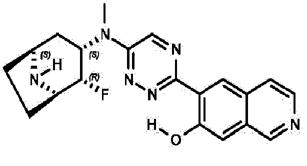 | 134 | 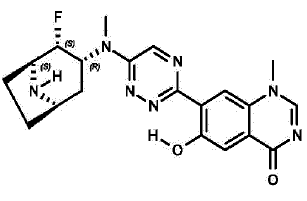 |
| 23 | 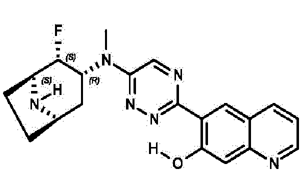 | 135 | 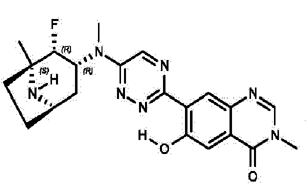 |
| 24 | 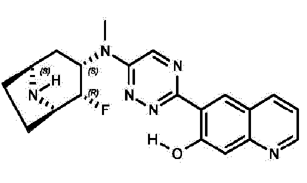 | 136 | 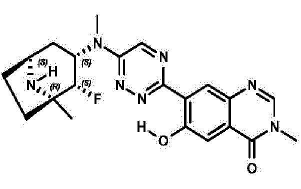 |
| 25 | 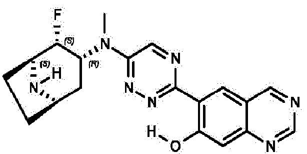 | 137 | 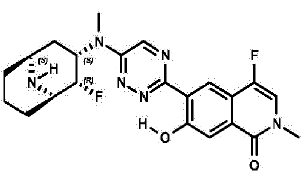 |
| 26 | 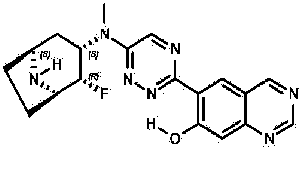 | 138 | 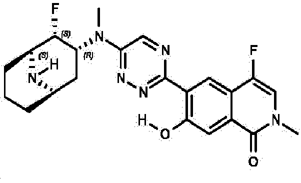 |
| 27 | 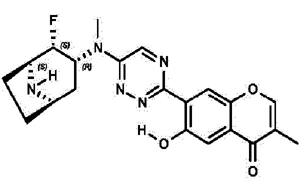 | 139 | 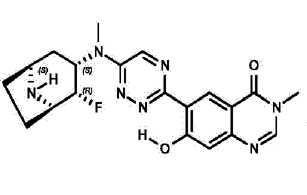 |
| 28 | 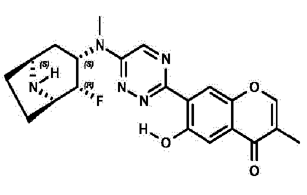 | 140 | 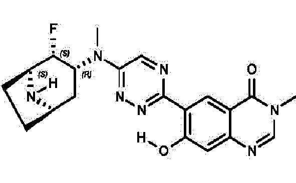 |
| 29 | 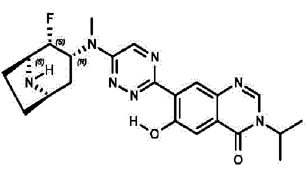 | 141 | 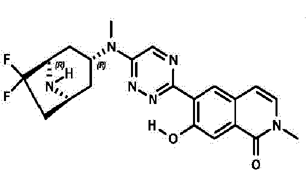 |
| 30 | 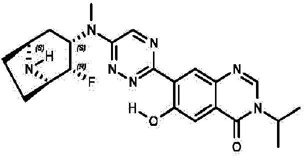 | 142 | 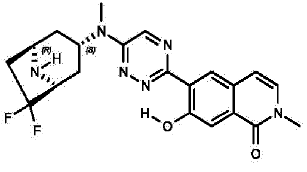 |
| 31 | 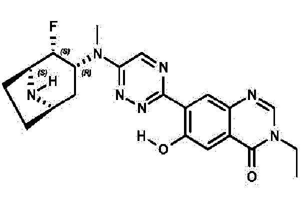 | 143 | 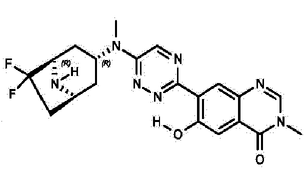 |
| 32 | 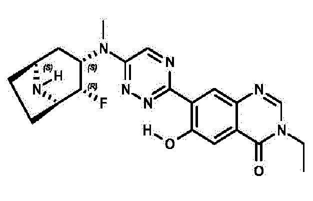 | 144 | 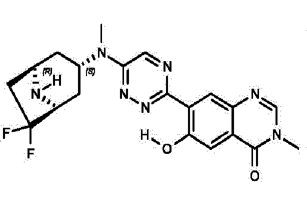 |
| 33 | 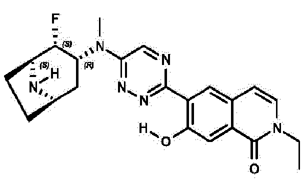 | 145 | 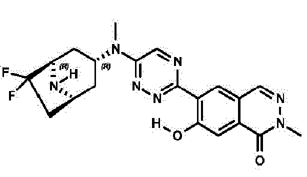 |
| 34 | 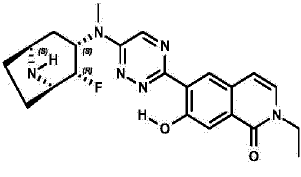 | 146 | 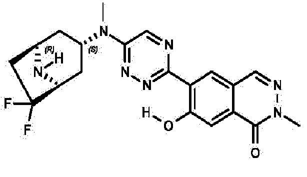 |
| 35 | 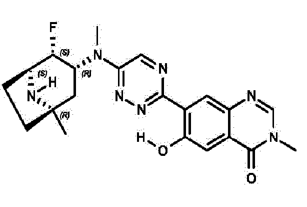 | 147 | 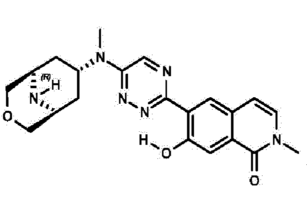 |
| 36 | 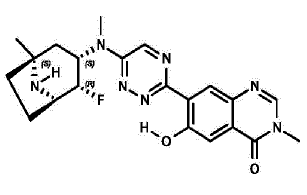 | 148 | 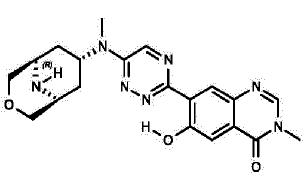 |
| 37 | 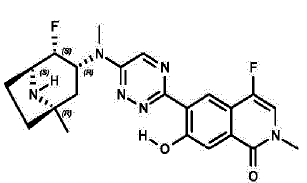 | 149 | 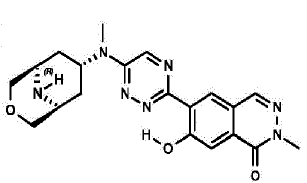 |
| 38 | 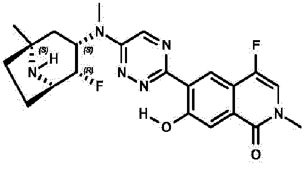 | 150 | 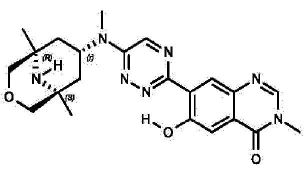 |
| 39 | 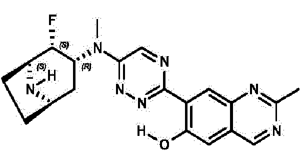 | 151 | 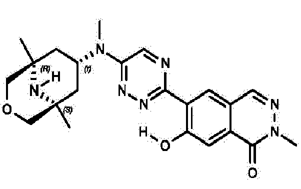 |
| 40 | 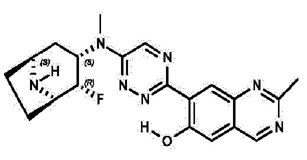 | 152 | 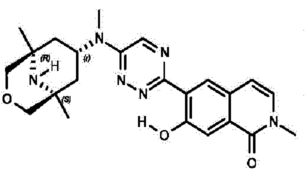 |
| 41 | 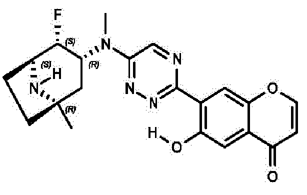 | 153 | 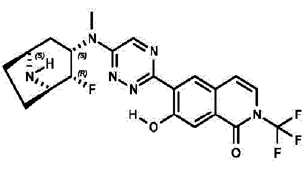 |
| 42 | 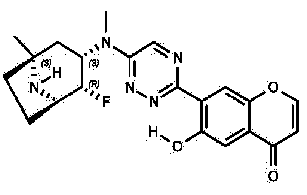 | 154 | 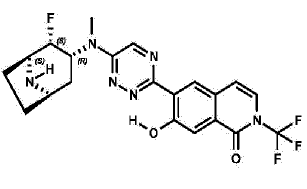 |
| 43 | 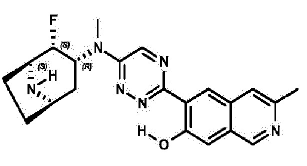 | 155 | 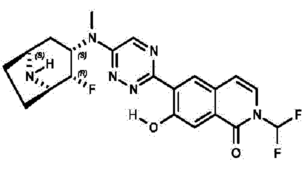 |
| 44 | 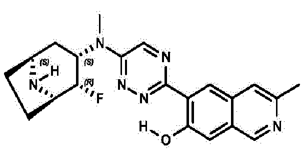 | 156 | 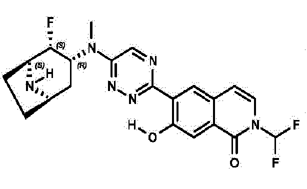 |
| 45 | 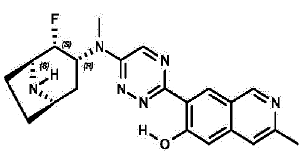 | 157 | 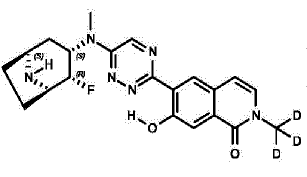 |
| 46 | 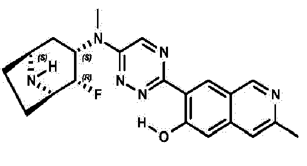 | 158 | 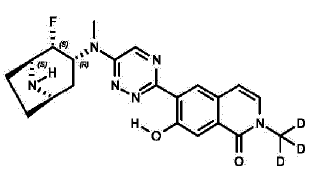 |
| 47 | 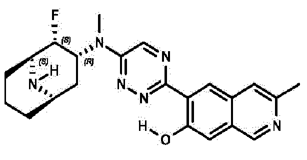 | 159 | 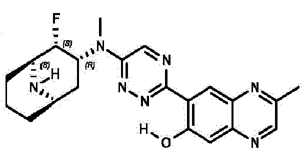 |
| 48 | 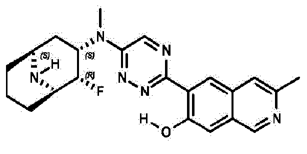 | 160 | 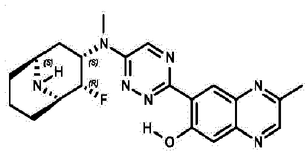 |
| 49 | 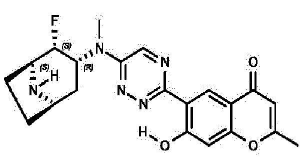 | 161 | 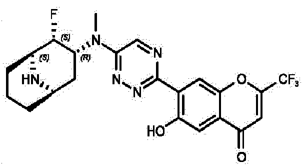 |
| 50 | 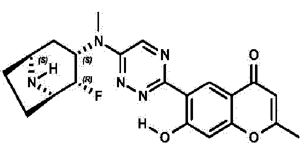 | 162 | 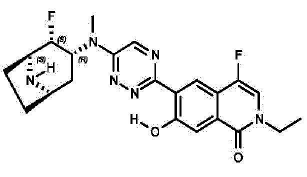 |
| 51 | 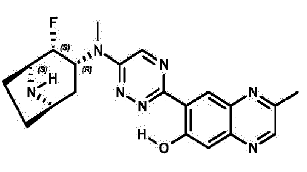 | 163 | 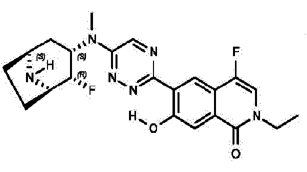 |
| 52 | 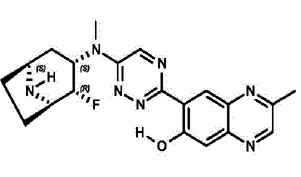 | 164 | 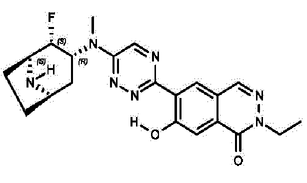 |
| 53 | 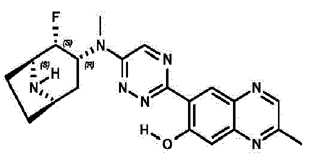 | 165 | 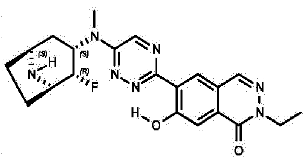 |
| 54 | 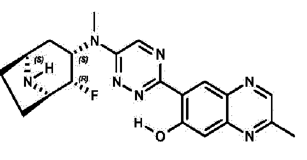 | 166 | 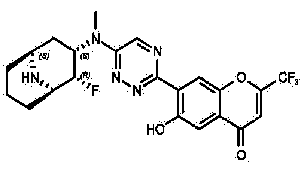 |
| 55 | 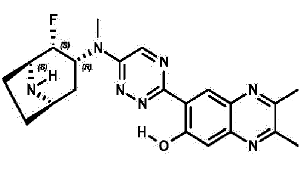 | 167 | 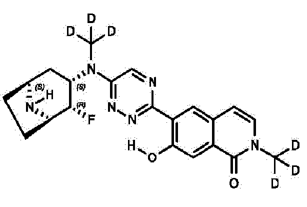 |
| 56 | 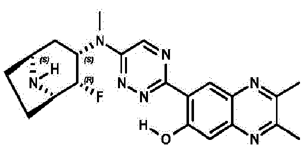 | 168 | 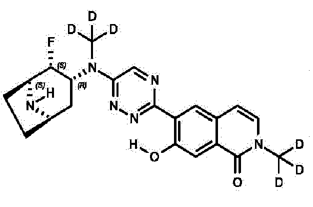 |
| 57 | 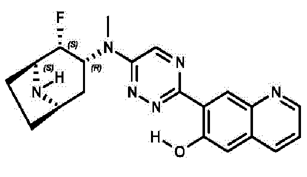 | 169 | 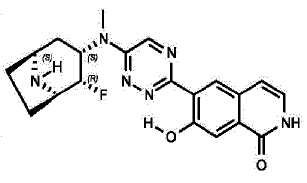 |
| 58 | 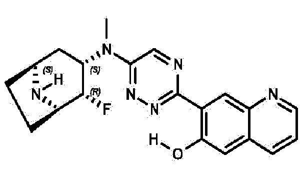 | 170 | 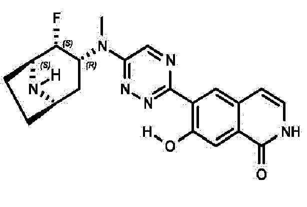 |
| 59 | 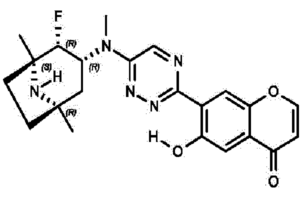 | 171 | 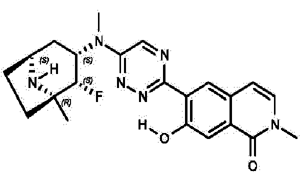 |
| 60 | 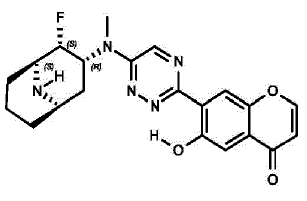 | 172 | 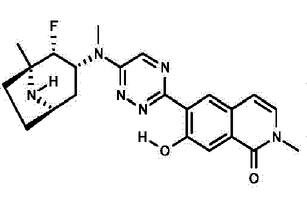 |
| 61 | 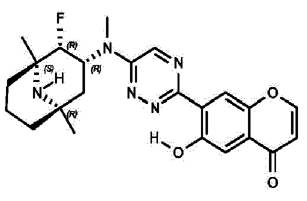 | 173 | 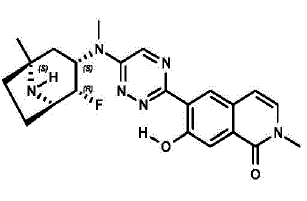 |
| 62 | 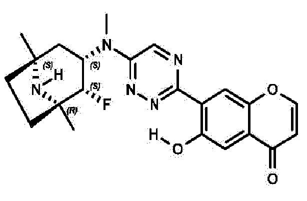 | 174 | 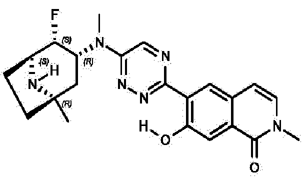 |
| 63 | 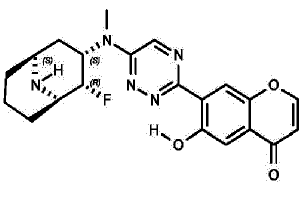 | 175 | 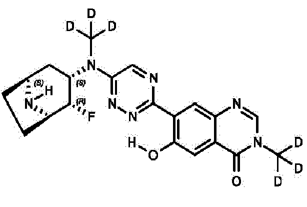 |
| 64 | 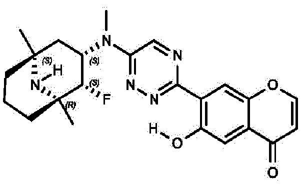 | 176 | 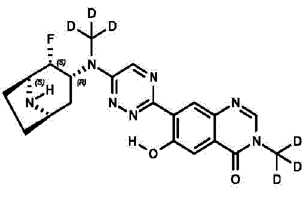 |
| 65 | 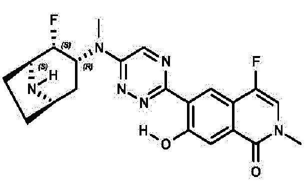 | 177 | 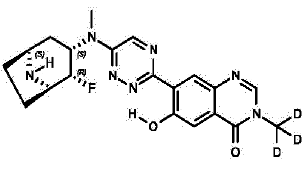 |
| 66 | 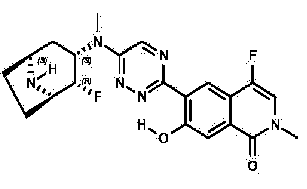 | 178 | 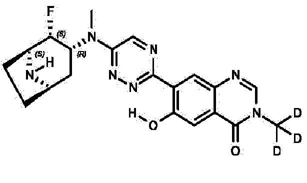 |
| 67 | 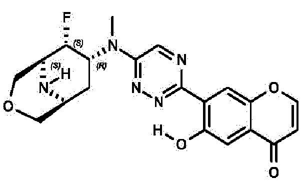 | 179 | 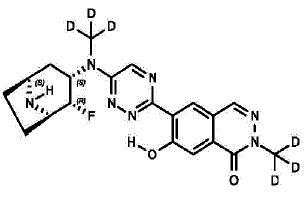 |
| 68 | 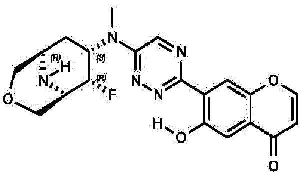 | 180 | 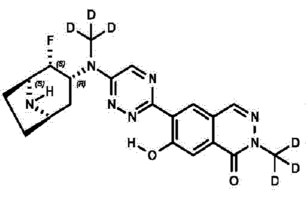 |
| 69 | 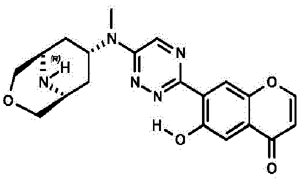 | 181 | 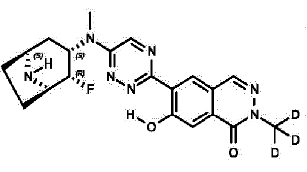 |
| 70 | 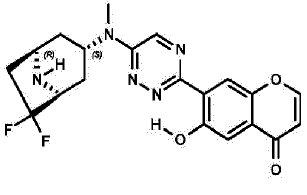 | 182 | 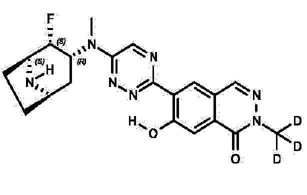 |
| 71 | 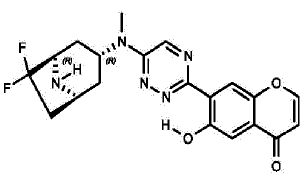 | 183 | 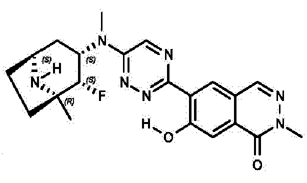 |
| 72 | 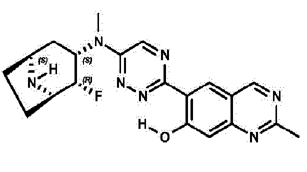 | 184 | 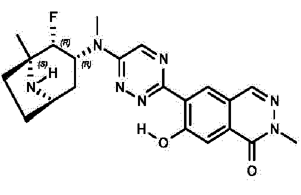 |
| 73 | 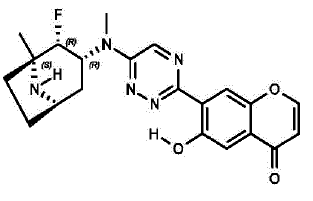 | 185 | 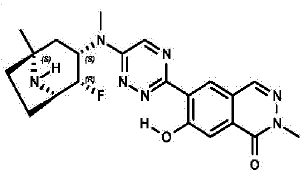 |
| 74 | 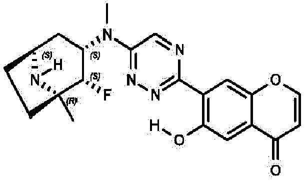 | 186 | 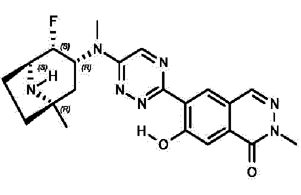 |
| 75 | 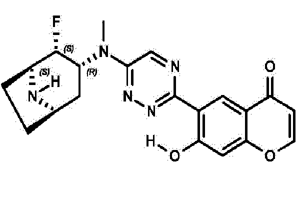 | 187 | 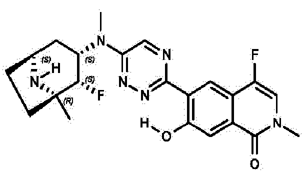 |
| 76 | 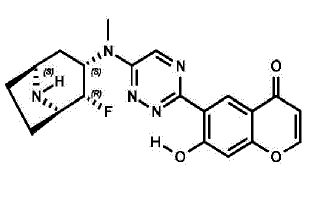 | 188 | 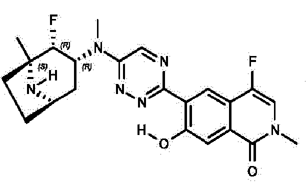 |
| 77 | 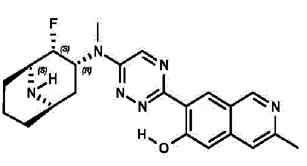 | 189 | 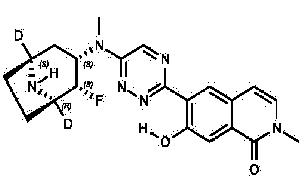 |
| 78 | 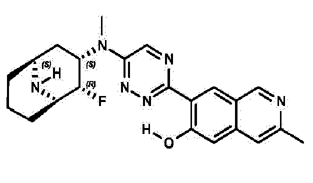 | 190 | 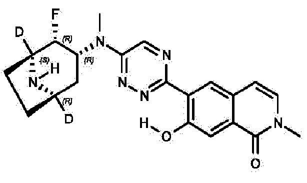 |
| 79 | 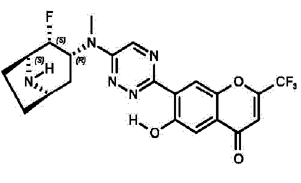 | 191 | 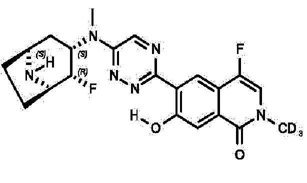 |
| 80 | 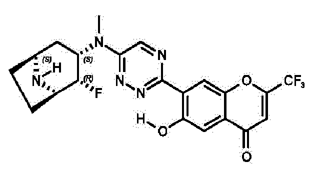 | 192 | 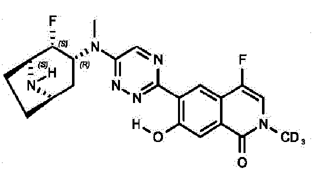 |
| 81 | 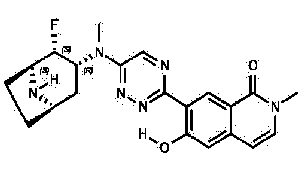 | 193 | 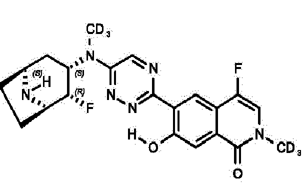 |
| 82 | 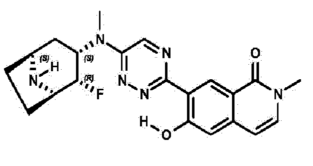 | 194 | 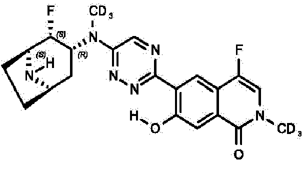 |
| 83 | 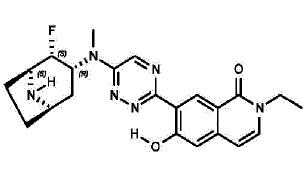 | 195 | 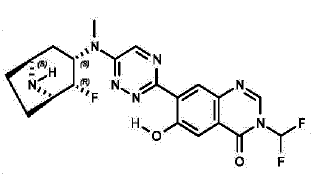 |
| 84 | 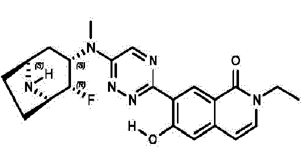 | 196 | 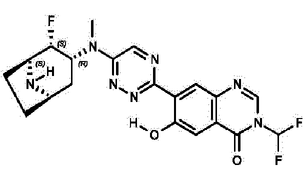 |
| 85 | 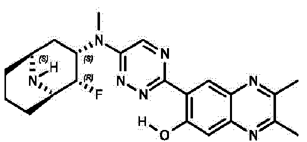 | 197 | 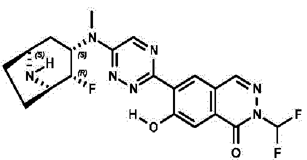 |
| 86 | 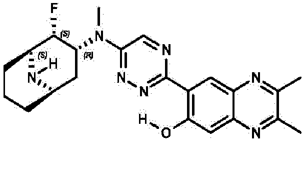 | 198 | 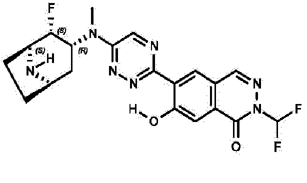 |
| 87 | 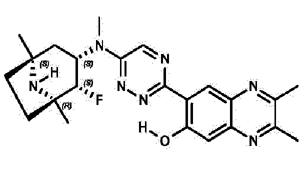 | 199 | 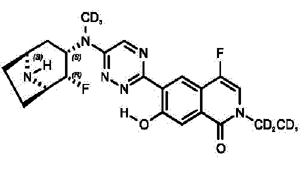 |
| 88 | 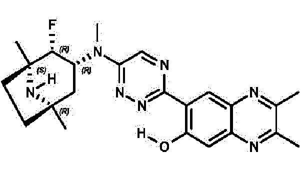 | 200 | 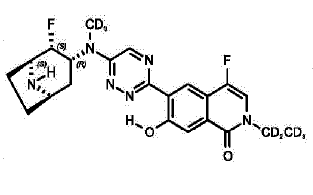 |
| 89 | 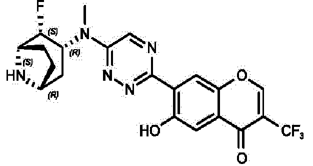 | 201 | 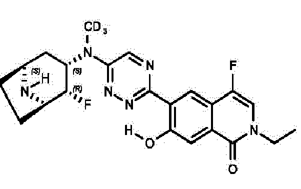 |
| 90 | 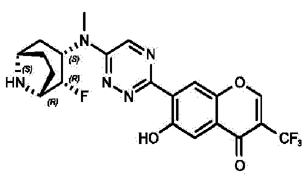 | 202 | 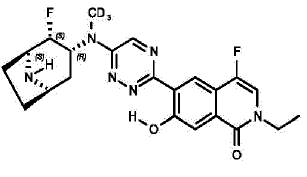 |
| 91 | 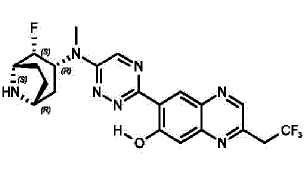 | 203 | 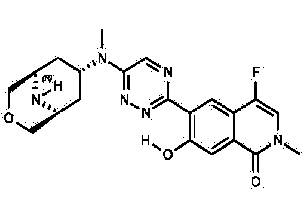 |
| 92 | 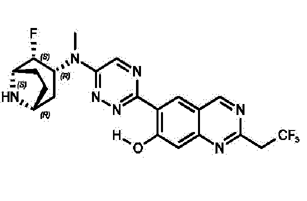 | 204 | 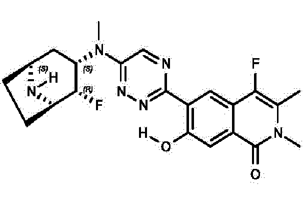 |
| 93 | 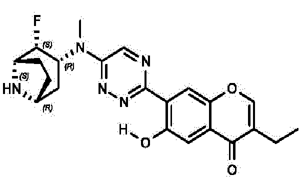 | 205 | 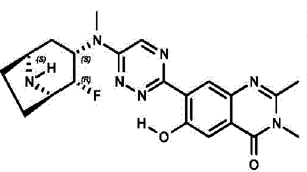 |
| 94 | 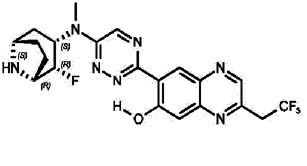 | 206 | 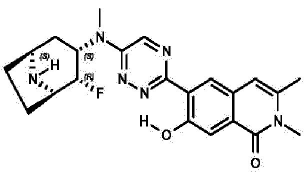 |
| 95 | 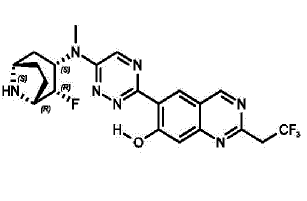 | 207 | 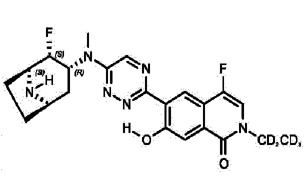 |
| 96 | 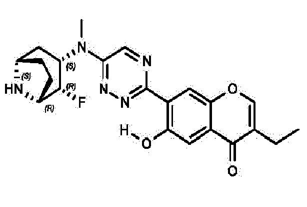 | 208 | 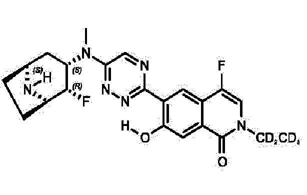 |
| 97 | 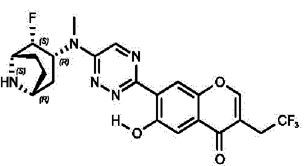 | 209 | 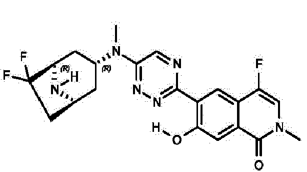 |
| 98 | 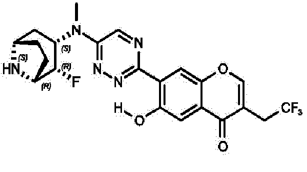 | 210 | 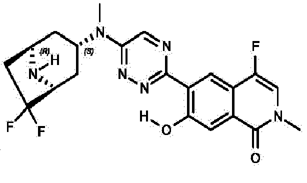 |
| 99 | 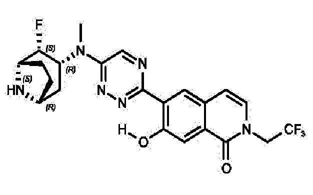 | 211 | 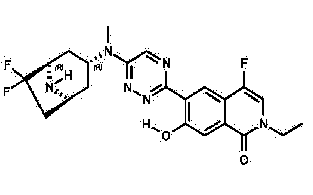 |
| 100 | 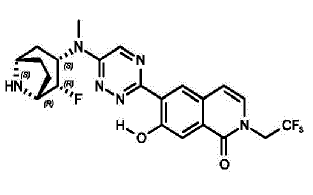 | 212 | 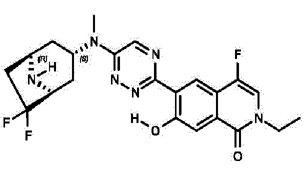 |
| 101 | 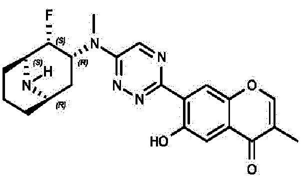 | 213 | 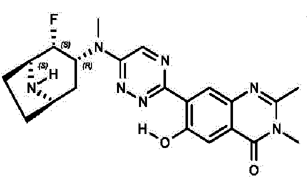 |
| 102 | 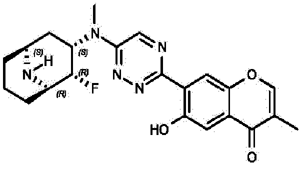 | 214 | 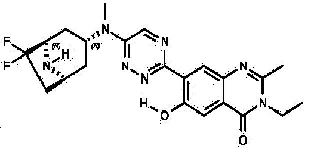 |
| 103 | 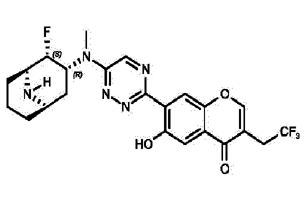 | 215 | 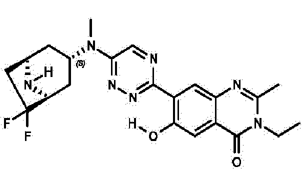 |
| 104 | 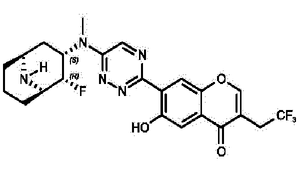 | 216 | 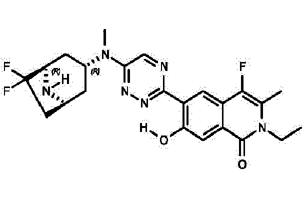 |
| 105 | 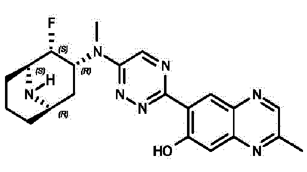 | 217 | 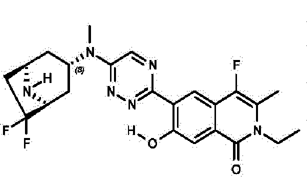 |
| 106 | 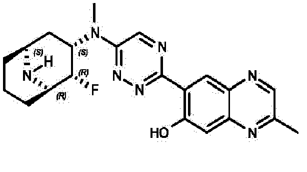 | 218 | 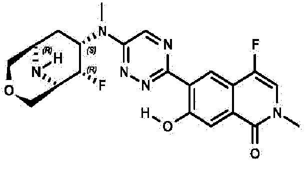 |
| 107 | 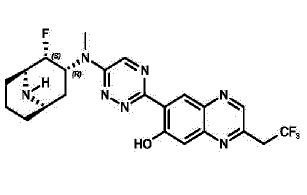 | 219 | 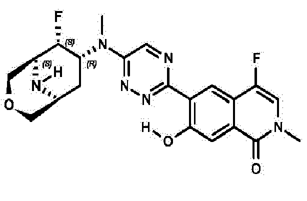 |
| 108 | 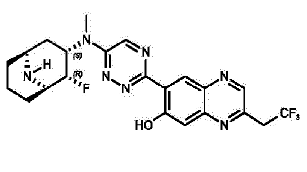 | 220 | 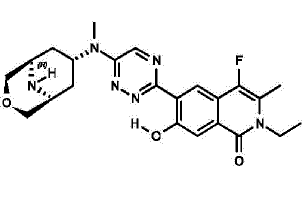 |
| 109 | 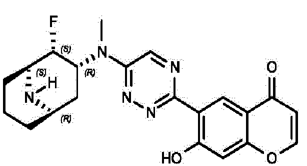 | 221 | 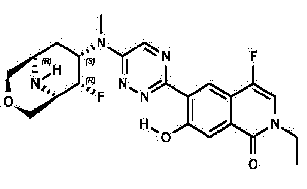 |
| 110 | 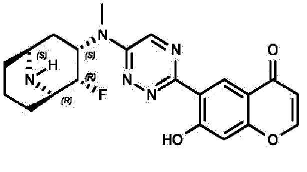 | 222 | 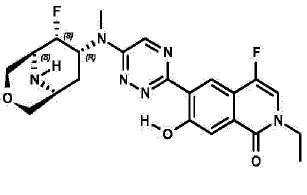 |
| 111 | 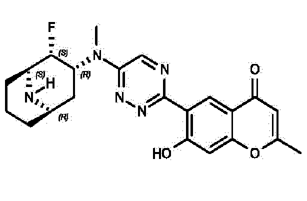 | 223 | 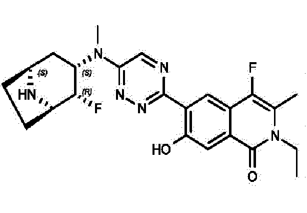 |
| 112 | 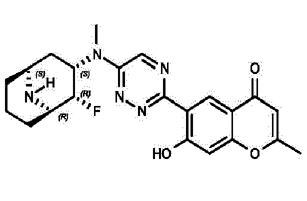 | 224 | 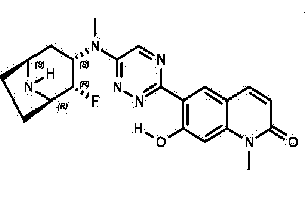 |
| 113 | 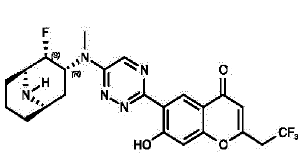 | 225 | 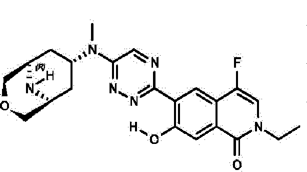 |
| 114 | 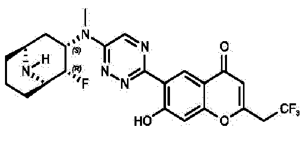 | 118 | 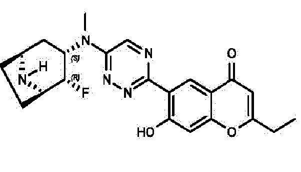 |
| 115 | 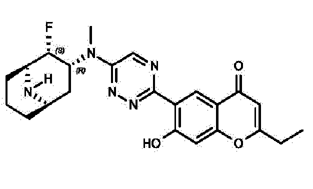 | 119 | 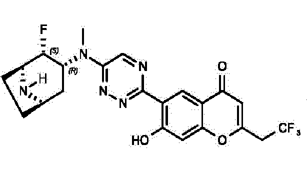 |
| 116 | 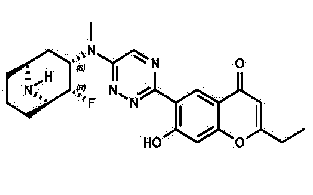 | 120 | 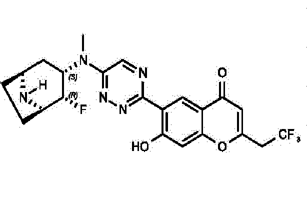 |
| 117 | 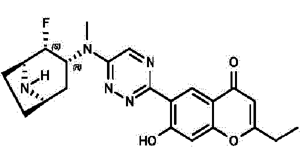 | 121 | 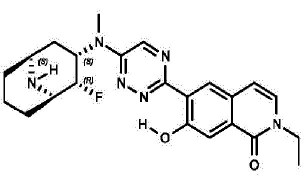 |

In some embodiments, disclosed herein is a pharmaceutically acceptable salt or pharmaceutically acceptable solvate of an SMSM compound from **Table 1 or Table 2** (excluding reference Examples).

### Pharmaceutical Compositions

In some embodiments, the compounds described herein are formulated into pharmaceutical compositions. Pharmaceutical compositions are formulated in a conventional manner using one or more pharmaceutically acceptable inactive ingredients that facilitate processing of the active compounds into preparations that can be used pharmaceutically. Proper formulation is dependent upon the route of administration chosen. A summary of pharmaceutical compositions described herein can be found, for example, in Remington: The Science and Practice of Pharmacy, Nineteenth Ed (Easton, Pa.: Mack Publishing Company, 1995); Hoover, John E., Remington's Pharmaceutical Sciences, Mack Publishing Co., Easton, Pennsylvania 1975; Liberman, H.A. and Lachman, L., Eds., Pharmaceutical Dosage Forms, Marcel Decker, New York, N.Y., 1980; and Pharmaceutical Dosage Forms and Drug Delivery Systems, Seventh Ed. (Lippincott Williams & Wilkins1999).

A pharmaceutical composition can be a mixture of an SMSM described herein with one or more other chemical components (i.e. pharmaceutically acceptable ingredients), such as carriers, excipients, binders, filling agents, suspending agents, flavoring agents, sweetening agents, disintegrating agents, dispersing agents, surfactants, lubricants, colorants, diluents, solubilizers, moistening agents, plasticizers, stabilizers, penetration enhancers, wetting agents, anti-foaming agents, antioxidants, preservatives, or one or more combination thereof. The pharmaceutical composition facilitates administration of the compound to an organism.

The compositions described herein can be administered to the subject in a variety of ways, including parenterally, intravenously, intradermally, intramuscularly, colonically, rectally or intraperitoneally. In some embodiments, the small molecule splicing modulator or a pharmaceutically acceptable salt thereof is administered by intraperitoneal injection, intramuscular injection, subcutaneous injection, or intravenous injection of the subject. In some embodiments, the pharmaceutical compositions can be administered parenterally, intravenously, intramuscularly or orally. The oral agents comprising a small molecule splicing modulator can be in any suitable form for oral administration, such as liquid, tablets, capsules, or the like. The oral formulations can be further coated or treated to prevent or reduce dissolution in stomach. The compositions of the present disclosure can be administered to a subject using any suitable methods known in the art. Suitable formulations for use in the present disclosure and methods of delivery are generally well known in the art. For example, the small molecule splicing modulators described herein can be formulated as pharmaceutical compositions with a pharmaceutically acceptable diluent, carrier or excipient. The compositions may contain pharmaceutically acceptable auxiliary substances as required to approximate physiological conditions including pH adjusting and buffering agents, tonicity adjusting agents, wetting agents and the like, such as, for example, sodium acetate, sodium lactate, sodium chloride, potassium chloride, calcium chloride, sorbitan monolaurate, triethanolamine oleate, etc.

Pharmaceutical formulations described herein can be administrable to a subject in a variety of ways by multiple administration routes, including but not limited to, oral, parenteral (*e.g.,* intravenous, subcutaneous, intramuscular, intramedullary injections, intrathecal, direct intraventricular, intraperitoneal, intralymphatic, intranasal injections), intranasal, buccal, topical or transdermal administration routes. The pharmaceutical formulations described herein include, but are not limited to, aqueous liquid dispersions, self-emulsifying dispersions, solid solutions, liposomal dispersions, aerosols, solid dosage forms, powders, immediate release formulations, controlled release formulations, fast melt formulations, tablets, capsules, pills, delayed release formulations, extended release formulations, pulsatile release formulations, multiparticulate formulations, and mixed immediate and controlled release formulations.

In some embodiments, the pharmaceutical formulation is in the form of a tablet. In other embodiments, pharmaceutical formulations containing an SMSM described herein are in the form of a capsule. In one aspect, liquid formulation dosage forms for oral administration are in the form of aqueous suspensions or solutions selected from the group including, but not limited to, aqueous oral dispersions, emulsions, solutions, elixirs, gels, and syrups.

For administration by inhalation, an SMSM described herein can be formulated for use as an aerosol, a mist or a powder. For buccal or sublingual administration, the compositions may take the form of tablets, lozenges, or gels formulated in a conventional manner. In some embodiments, an SMSM described herein can be prepared as transdermal dosage forms. In some embodiments, an SMSM described herein can be formulated into a pharmaceutical composition suitable for intramuscular, subcutaneous, or intravenous injection. In some embodiments, an SMSM described herein can be administered topically and can be formulated into a variety of topically administrable compositions, such as solutions, suspensions, lotions, gels, pastes, medicated sticks, balms, creams or ointments. In some embodiments, an SMSM described herein can be formulated in rectal compositions such as enemas, rectal gels, rectal foams, rectal aerosols, suppositories, jelly suppositories, or retention enemas.

### Splicing Modulation

The present disclosure contemplates use of small molecules with favorable drug properties that modulate the activity of splicing of a target RNA. Provided herein are small molecule splicing modulators (SMSMs) that modulate splicing of a target polynucleotide. In some embodiments, the SMSMs bind and modulate target RNA. In some embodiments, provided herein is a library of SMSMs that bind and modulate one or more target RNAs. In some embodiments, the target RNA is mRNA. In some embodiments, the target RNA is mRNA a noncoding RNA. In some embodiments, the target RNA is a pre-mRNA. In some embodiments, the target RNA is hnRNA. In some embodiments, the small molecules modulate splicing of the target RNA. In some embodiments, a small molecule provided herein modulates splicing at a sequence of the target RNA. In some embodiments, a small molecule provided herein modulates splicing at a cryptic splice site sequence of the target RNA. In some embodiments, a small molecule provided herein binds to a target RNA. In some embodiments, a small molecule provided herein binds to a splicing complex component. In some embodiments, a small molecule provided herein binds to a target RNA and a splicing complex component.

Thus, described herein are methods of preventing or inducing a splicing event in a pre-mRNA molecule, comprising contacting the pre-mRNA molecule and/or other elements of the splicing machinery (*e.g.,* within a cell) with a compound provided herein to prevent or induce the splicing event in the pre-mRNA molecule. The splicing event that is prevented or induced can be, *e.g.,* an aberrant splicing event, a constitutive splicing event or an alternate splicing event.

Further described herein is a method of identifying a compound capable of preventing or inducing a splicing event in a pre-mRNA molecule, comprising contacting the compound with splicing elements and/or factors involved in alternative, aberrant and/or constitutive splicing as described herein (*e.g.,* within cells) under conditions whereby a positive (prevention or induction of splicing) or negative (no prevention or induction of splicing) effect is produced and detected and identifying a compound that produces a positive effect as a compound capable of preventing or inducing a splicing event.

In some embodiments, a small molecule compound described herein in a pharmaceutically acceptable carrier prevents or induces an alternative or aberrant splicing event in a pre-mRNA molecule. As noted above, the small molecule compounds provided herein are not antisense or antigene oligonucleotides.

In some embodiments, a method of treating a subject with a disease or condition comprises administering a small molecule splicing modulator compound (SMSM) to a subject with a disease or condition. In some embodiments, the subject is a mammal. In some embodiments, the mammal is a human. In some embodiments, the polynucleotide is a pre-mRNA. In some embodiments, the method further comprises administering an additional therapeutic molecule to the subject. In some embodiments, the SMSM is a compound described herein.

The compounds and formulations described herein are also useful as therapeutic agents in the treatment of disease involving aberrant and/or alternate splicing. Thus, in some embodiments, a method of treating a subject having a condition or disorder associated with an alternative or aberrant splicing event in a pre-mRNA molecule, comprises administering to the subject a therapeutically effective amount of a compound described herein to modulate an alternative splicing event or prevent an aberrant splicing event, thereby treating the subject. The method can, *e.g.,* restore a correct splicing event in a pre-mRNA molecule. The method can, *e.g.,* utilize a small molecule compound described herein in a pharmaceutically acceptable carrier.

Formulations containing the small molecules described herein can comprise a physiologically or pharmaceutically acceptable carrier, such as an aqueous carrier. Thus, formulations for use in the methods described herein include, but are not limited to, those suitable for oral administration, parenteral administration, including subcutaneous, intradermal, intramuscular, intravenous and intra-arterial administration, as well as topical administration (e.g., administration of an aerosolized formulation of respirable particles to the lungs of a patient afflicted with cystic fibrosis or lung cancer or a cream or lotion formulation for transdermal administration of patients with psoriasis). The formulations may conveniently be presented in unit dosage form and may be prepared by any of the methods well known in the art. The most suitable route of administration in any given case may depend upon the subject, the nature and severity of the condition being treated, and the particular active compound, which is being used, as would be readily determined by one of skill in the art.

Also described herein are methods for the use of a compound described herein having the characteristics set forth above for the preparation of a medicament for upregulating or downregulating RNA expression in a patient having a disorder associated with aberrant or alternate splicing of a pre-mRNA molecule, as discussed above. In some embodiments, the medicament upregulates gene expression. In other embodiments, the medicament downregulates gene expression. In the manufacture of a medicament according to the disclosure, the compound can be admixed with, inter alia, a pharmaceutically acceptable carrier. The carrier may be a solid or a liquid. One or more compounds may be incorporated in any combination in the formulations described herein, which may be prepared by any of the well-known techniques of pharmacy, such as admixing the components, and/or including one or more accessory therapeutic ingredients.

The present disclosure identifes low molecular weight compounds (sometimes referred to herein as small molecules, which block mRNA splicing and/or enhance (facilitate, augment) mRNA splicing. The splicing that can be regulated by the methods described herein include alternative splicing, *e.g.,* exon skipping, intron retention, pseudoexons skipping, exon exclusion, partial intron exclusion and others. Depending on factors such as the splicing sequence and the RNA (or gene encoding the RNA) or exon involved, modulation of splicing can be accomplished in the presence of, or in the absence of, antisense oligonucleotides (AOs) that are specific for splicing sequences of interest. In some embodiments, a small molecule and an AO act synergistically.

In some embodiments, the present disclosure provides a compound, or a pharmaceutically acceptable salt thereof, or a pharmaceutical compostion for use in a method of treating a subject afflicted with a disease or condition associated with aberrant splicing of a pre-mRNA. The method can comprise administering an SMSM, or a composition comprising an SMSM, to a subject, wherein the SMSM binds to a pre-mRNA or a splicing complex component and modulates splicing of the pre-mRNA to inhibit expression of one or more isoforms of a transcript. The method can comprise administering an SMSM, or a composition comprising an SMSM, to a subject, wherein the SMSM binds to a pre-mRNA or a splicing complex component and modulates the splicing of the pre-mRNA to increase expression of one or more isoforms of a transcript.

A number of diseases are associated with expression of an aberrant gene product (*e.g*., an RNA transcript or protein) of a gene. For example, aberrant amounts of a RNA transcript may lead to disease due to corresponding changes in protein expression. Changes in the amount of a particular RNA transcript may be the result of several factors. First, changes in the amount of RNA transcripts may be due to an aberrant level of transcription of a particular gene, such as by the perturbation of a transcription factor or a portion of the transcription process, resulting in a change in the expression level of a particular RNA transcript. Second, changes in the splicing of particular RNA transcripts, such as by perturbation of a particular splicing process or mutations in the gene that lead to modified splicing can change the levels of a particular RNA transcript. Changes to the stability of a particular RNA transcript or to components that maintain RNA transcript stability, such as the process of poly-A tail incorporation or an effect on certain factors or proteins that bind to and stabilize RNA transcripts, may lead to changes in the levels of a particular RNA transcript. The level of translation of particular RNA transcripts can also affect the amount of those transcripts, affecting or upregulating RNA transcript decay processes. Finally, aberrant RNA transport or RNA sequestration may also lead to changes in functional levels of RNA transcripts, and may have an effect on the stability, further processing, or translation of the RNA transcripts.

Described herein are methods for modulating the amount of one, two, three or more RNA transcripts encoded by a pre-mRNA, comprising contacting a cell with an SMSM compound or a pharmaceutically acceptable salt thereof. The cell may be contacted with an SMSM compound or a pharmaceutically acceptable salt thereof in a cell culture. The cell may be contacted with an SMSM compound or a pharmaceutically acceptable salt thereof in a subject (*e.g.,* a non-human animal subject or a human subject).

In some embodiments, provided herein are compounds, or a pharmaceutically acceptable salt thereof, or a pharmaceutical composition, for use in methods for treatment, prevention and/or delay of progression of a disease or condition comprising administering an effective amount of a small molecule splicing modulator as described herein to a subject, in particular to a mammal.

In some embodiments, binding of an SMSM compound or a pharmaceutically acceptable salt thereof to pre-mRNA prevents splicing out of one or more exons and/or introns and/or proteins thereof, from the population of pre-mRNAs to produce mRNA encoding the target protein or functional RNA. In some embodiments, the cell comprises a population of pre-mRNAs transcribed from the gene encoding the target protein or functional RNA, wherein the population of pre-mRNAs comprises a mutation that causes the splicing out of one or more exons, and wherein an SMSM compound or a pharmaceutically acceptable salt thereof binds to the mutation that causes the splicing out of the one or more exons in the population of pre-mRNAs. In some embodiments, the binding of an SMSM compound or a pharmaceutically acceptable salt thereof to the mutation that causes the splicing out of the one or more exons prevents splicing out of the one or more exons from the population of pre-mRNAs to produce mRNA encoding the target protein or functional RNA. In some embodiments, the condition is a disease or disorder. In some embodiments, the method further comprises assessing protein expression. In some embodiments, an SMSM compound or a pharmaceutically acceptable salt thereof binds to a targeted portion of a pre-mRNA.

In some embodiments, the binding of an SMSM compound or a pharmaceutically acceptable salt thereof catalyzes the inclusion of a missing exon or removal of an undesired retained intron or portions thereof, resulting in healthy mRNA and proteins. In some embodiments, the binding of an SMSM compound or a pharmaceutically acceptable salt thereof has minimal to no effect on non-diseased cells.

### SMSM Targets

Aberrant splicing of mRNA, such as pre-mRNA, can result in a defective protein and can cause a disease or a disorder in a subject. The compositions and methods described herein can reduce this aberrant splicing of mRNA, such as pre-mRNA, and treat a disease or a disorder caused by this aberrant splicing.

Diseases associated with changes to RNA transcript amount are often treated with a focus on the aberrant protein expression. However, if the processes responsible for the aberrant changes in RNA levels, such as components of the splicing process or associated transcription factors or associated stability factors, could be targeted by treatment with a small molecule, it would be possible to restore protein expression levels such that the unwanted effects of the expression of aberrant levels of RNA transcripts or associated proteins. Therefore, there is a need for methods of modulating the amount of RNA transcripts encoded by certain genes as a way to prevent or treat diseases associated with aberrant expression of the RNA transcripts or associated proteins.

### Methods of Treatment

References to methods of treatment by therapy or surgery or in vivo diagnosis methods in this description are to be interpreted as references to compounds, pharmaceutical compositions and medicaments of the present invention for use in those methods.

The compositions described herein can be used for treating a human disease or disorder associated with aberrant splicing, such as aberrant pre-mRNA splicing. The compositions described herein can be used for treating a human disease or disorder by modulating mRNA, such as pre-mRNA. In some embodiments, the compositions described herein can be used for treating a human disease or disorder by modulating splicing of a nucleic acid even when that nucleic acid is not aberrantly spliced in the pathogenesis of the disease or disorder being treated.

In some embodiments, an effective amount in the context of the administration of an SMSM compound or a pharmaceutically acceptable salt thereof, or composition or medicament thereof refers to an amount of an SMSM compound or a pharmaceutically acceptable salt thereof to a patient which has a therapeutic effect and/or beneficial effect. In certain specific embodiments, an effective amount in the context of the administration of an SMSM compound or a pharmaceutically acceptable salt thereof, or composition or medicament thereof to a patient results in one, two or more of the following effects: (i) reduces or ameliorates the severity of a disease; (ii) delays onset of a disease; (iii) inhibits the progression of a disease; (iv) reduces hospitalization of a subject; (v) reduces hospitalization length for a subject; (vi) increases the survival of a subject; (vii) improves the quality of life of a subject; (viii) reduces the number of symptoms associated with a disease; (ix) reduces or ameliorates the severity of a symptom associated with a disease; (x) reduces the duration of a symptom associated with a disease associated; (xi) prevents the recurrence of a symptom associated with a disease; (xii) inhibits the development or onset of a symptom of a disease; and/or (xiii) inhibits of the progression of a symptom associated with a disease. In some embodiments, an effective amount of an SMSM compound or a pharmaceutically acceptable salt thereof is an amount effective to restore the amount of a RNA transcript of a gene to the amount of the RNA transcript detectable in healthy patients or cells from healthy patients. In other embodiments, an effective amount of an SMSM compound or a pharmaceutically acceptable salt thereof is an amount effective to restore the amount an RNA isoform and/or protein isoform of gene to the amount of the RNA isoform and/or protein isoform detectable in healthy patients or cells from healthy patients.

In some embodiments, an effective amount of an SMSM compound or a pharmaceutically acceptable salt thereof is an amount effective to decrease the aberrant amount of an RNA transcript of a gene which is associated with a disease. In some embodiments, an effective amount of an SMSM compound or a pharmaceutically acceptable salt thereof is an amount effective to decrease the amount of the aberrant expression of an isoform of a gene. In some embodiments, an effective amount of an SMSM compound or a pharmaceutically acceptable salt thereof is an amount effective to result in a substantial change in the amount of an RNA transcript (e.g., mRNA transcript), alternative splice variant or isoform.

In some embodiments, an effective amount of an SMSM compound or a pharmaceutically acceptable salt thereof is an amount effective to increase or decrease the amount of an RNA transcript (*e.g.,* an mRNA transcript) of gene which is beneficial for the prevention and/or treatment of a disease. In some embodiments, an effective amount of an SMSM compound or a pharmaceutically acceptable salt thereof is an amount effective to increase or decrease the amount of an alternative splice variant of an RNA transcript of a gene which is beneficial for the prevention and/or treatment of a disease. In some embodiments, an effective amount of an SMSM compound or a pharmaceutically acceptable salt thereof is an amount effective to increase or decrease the amount of an isoform of gene which is beneficial for the prevention and/or treatment of a disease. In some embodiments, the gene is SMN2. In some embodiments, modulating splicing of the polynucleotide comprises inhibiting skipping of exon 7. In some embodiments, the SMSM compounds and methods of their use described herein can modulate splicing of a pre-mRNA of SMN2. For example, the SMSM compounds and methods of their use described herein can modulate splicing of exon 7 of a pre-mRNA of SMN2. In some embodiments, the SMSM compounds and methods modulate splicing of the polynucleotide through the splicing sequence disclosed and to treat the diseases disclosed in Table 3.

**Table 3**

| Gene | Diseases | Splice Site Sequence | Description | Exon |
|---|---|---|---|---|
| SMN2 | Spinal muscular atrophy | GGAguaagu | IVS7+6C>U Mutation inducing loss of U1snRNA affinity | 7 |

An SMSM described herein can be used in the preparation of medicaments for the treatment of diseases or conditions described herein. In addition, a method for treating any of the diseases or conditions described herein in a subject in need of such treatment, can involve administration of pharmaceutical compositions that includes at least one SMSM described herein or a pharmaceutically acceptable salt, thereof, in a therapeutically effective amount to a subject.

In certain embodiments, an SMSM described herein can be administered for prophylactic and/or therapeutic treatments. In certain therapeutic applications, the compositions are administered to a patient already suffering from a disease or condition, in an amount sufficient to cure or at least partially arrest at least one of the symptoms of the disease or condition. Amounts effective for this use depend on the severity and course of the disease or condition, previous therapy, the patient's health status, weight, and response to the drugs, and the judgment of the treating physician. Therapeutically effective amounts are optionally determined by methods including, but not limited to, a dose escalation clinical trial. In prophylactic applications, compositions containing an SMSM described herein can be administered to a patient susceptible to or otherwise at risk of a particular disease, disorder or condition. In certain embodiments, the dose of drug being administered may be temporarily reduced or temporarily suspended for a certain length of time (*i.e*., a "drug holiday"). Doses employed for adult human treatment typically range of 0.01mg-5000 mg per day or from about 1 mg to about 1000 mg per day. In some embodiments, a desired dose is conveniently presented in a single dose or in divided doses.

For combination therapies described herein, dosages of the co-administered compounds can vary depending on the type of co-drug(s) employed, on the specific drug(s) employed, on the disease or condition being treated and so forth. In additional embodiments, when co-administered with one or more other therapeutic agents, the compound provided herein is administered either simultaneously with the one or more other therapeutic agents, or sequentially. If administration is simultaneous, the multiple therapeutic agents can be, by way of example only, provided in a single, unified form, or in multiple forms.

### Methods of Administering

The compositions described herein can be administered to the subject in a variety of ways, including parenterally, intravenously, intradermally, intramuscularly, colonically, rectally or intraperitoneally. In some embodiments, the small molecule splicing modulator or a pharmaceutically acceptable salt thereof is administered by intraperitoneal injection, intramuscular injection, subcutaneous injection, or intravenous injection of the subject. In some embodiments, the pharmaceutical compositions can be administered parenterally, intravenously, intramuscularly or orally. The oral agents comprising a small molecule splicing modulator can be in any suitable form for oral administration, such as liquid, tablets, capsules, or the like. The oral formulations can be further coated or treated to prevent or reduce dissolution in stomach. The compositions of the present disclosure can be administered to a subject using any suitable methods known in the art. Suitable formulations for use in the present disclosure and methods of delivery are generally well known in the art. For example, the small molecule splicing modulators described herein can be formulated as pharmaceutical compositions with a pharmaceutically acceptable diluent, carrier or excipient. The compositions may contain pharmaceutically acceptable auxiliary substances as required to approximate physiological conditions including pH adjusting and buffering agents, tonicity adjusting agents, wetting agents and the like, such as, for example, sodium acetate, sodium lactate, sodium chloride, potassium chloride, calcium chloride, sorbitan monolaurate, triethanolamine oleate, etc.

Pharmaceutical formulations described herein can be administrable to a subject in a variety of ways by multiple administration routes, including but not limited to, oral, parenteral (*e.g.,* intravenous, subcutaneous, intramuscular, intramedullary injections, intrathecal, direct intraventricular, intraperitoneal, intralymphatic, intranasal injections), intranasal, buccal, topical or transdermal administration routes. The pharmaceutical formulations described herein include, but are not limited to, aqueous liquid dispersions, self-emulsifying dispersions, solid solutions, liposomal dispersions, aerosols, solid dosage forms, powders, immediate release formulations, controlled release formulations, fast melt formulations, tablets, capsules, pills, delayed release formulations, extended release formulations, pulsatile release formulations, multiparticulate formulations, and mixed immediate and controlled release formulations.

In some embodiments, the pharmaceutical compositions described herein are administered orally. In some embodiments, the pharmaceutical compositions described herein are administered topically. In such embodiments, the pharmaceutical compositions described herein are formulated into a variety of topically administrable compositions, such as solutions, suspensions, lotions, gels, pastes, shampoos, scrubs, rubs, smears, medicated sticks, medicated bandages, balms, creams or ointments. In some embodiments, the pharmaceutical compositions described herein are administered topically to the skin. In some embodiments, the pharmaceutical compositions described herein are administered by inhalation. In some embodiments, the pharmaceutical compositions described herein are formulated for intranasal administration. Such formulations include nasal sprays, nasal mists, and the like. In some embodiments, the pharmaceutical compositions described herein are formulated as eye drops. In some embodiments, the pharmaceutical compositions described herein are: (a) systemically administered to the mammal; and/or (b) administered orally to the mammal; and/or (c) intravenously administered to the mammal; and/or (d) administered by inhalation to the mammal; and/or (e) administered by nasal administration to the mammal; or and/or (f) administered by injection to the mammal; and/or (g) administered topically to the mammal; and/or (h) administered by ophthalmic administration; and/or (i) administered rectally to the mammal; and/or (j) administered non-systemically or locally to the mammal. In some embodiments, the pharmaceutical compositions described herein are administered orally to the mammal. In certain embodiments, an SMSM described herein is administered in a local rather than systemic manner. In some embodiments, an SMSM described herein is administered topically. In some embodiments, an SMSM described herein is administered systemically.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser that contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

SMSMs suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL^{™} (BASF, Parsippany, NJ) or phosphate buffered saline (PBS). In all cases, the composition must be sterile and should be fluid to the extent that easy syringability exists. It must be stable under the conditions of manufacture and storage and must be preserved against contamination from microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition.

### Dosing and Schedules

The SMSMs utilized in the methods of the disclosure can be, e.g., administered at dosages that may be varied depending upon the requirements of the subject the severity of the condition being treated and/or imaged, and/or the SMSM being employed. For example, dosages can be empirically determined considering the type and stage of disease diagnosed in a particular subject and/or the type of imaging modality being used in conjunction with the SMSMs. The dose administered to a subject, in the context of the present disclosure should be sufficient to affect a beneficial diagnostic or therapeutic response in the subject. The size of the dose also can be determined by the existence, nature, and extent of any adverse side-effects that accompany the administration of a SMSM in a particular subject.

It is advantageous to formulate compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the disclosure are dictated by and directly dependent on the unique characteristics of the active compound and the particular therapeutic effect to be achieved, and the limitations inherent in the art of compounding such an active compound for the treatment of individuals. Toxicity and therapeutic efficacy of such compounds can be determined by procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compounds that exhibit large therapeutic indices are preferred. While compounds that exhibit toxic side effects may be used, care should be taken to design a delivery system that targets such compounds to the site of affected tissue to minimize potential damage to uninfected cells and, thereby, reduce side effects.

Therapeutic index data obtained from cell culture assays and/or animal studies can be used in predicting the therapeutic index *in vivo* and formulating a range of dosages for use in subjects, such as human subjects. The data obtained from the cell culture assays and animal studies can be used in formulating a range of dosage for use in humans. The dosage of such compounds lies preferably within a range of circulating concentrations that include the ED₅₀ with little or no toxicity. The dosage may vary within this range depending upon the dosage form employed and the route of administration utilized. For any compound used in the method of the disclosure, the therapeutically effective dose can be estimated initially from cell culture assays. A dose may be formulated in animal models to achieve a circulating plasma concentration range that includes the concentration of the test compound which achieves a half-maximal inhibition of symptoms as determined in cell culture. Such information can be used to more accurately determine useful doses in humans. Levels in plasma may be measured, for example, by high performance liquid chromatography. Various animal models and clinical assays for evaluating effectiveness of a particular SMSM in preventing or reducing a disease or condition are known in the art may be used in the present disclosure. The dosage may vary within this range depending upon the dosage form employed and the route of administration utilized. The exact formulation, route of administration and dosage can be chosen by the individual physician in view of the patient's condition. (See e.g. Fingl et al, 1975, In: The Pharmacological Basis of Therapeutics. Ch. 1 pi).

The compositions of the present disclosure can be administered as frequently as necessary, including hourly, daily, weekly or monthly.

In any of the aforementioned aspects are further embodiments comprising single administrations of an effective amount of an SMSM described herein, including further embodiments in which (i) the compound is administered once; (ii) the compound is administered to the mammal multiple times over the span of one day; (iii) continually; or (iv) continuously.

In any of the aforementioned aspects are further embodiments comprising multiple administrations of the effective amount of an SMSM described herein, including further embodiments in which (i) the compound is administered continuously or intermittently: as in a single dose; (ii) the time between multiple administrations is every 6 hours; (iii) the compound is administered to the mammal every 8 hours; (iv) the compound is administered to the mammal every 12 hours; (v) the compound is administered to the mammal every 24 hours. In further or alternative embodiments, the method comprises a drug holiday, wherein the administration of an SMSM described herein is temporarily suspended or the dose of the compound being administered is temporarily reduced; at the end of the drug holiday, dosing of the compound is resumed. In one embodiment, the length of the drug holiday varies from 2 days to 1 year.

### Combination Therapies

In certain instances, it is appropriate to administer at least one SMSM described herein in combination with another therapeutic agent. For example, a compound SMSM described herein can be co-administered with a second therapeutic agent, wherein SMSM and the second therapeutic agent modulate different aspects of the disease, disorder or condition being treated, thereby providing a greater overall benefit than administration of either therapeutic agent alone.

In some embodiments, an SMSM may be administered in combination with one or more other SMSMs.

A SMSM may be administered to a subject in need thereof prior to, concurrent with, or following the administration of other therapeutic agents. For instance, SMSMs may be administered to a subject at least 8 hours, 7 hours, 6 hours, 5 hours, 4 hours, 3 hours, 2 hours, 1.5 hours, 1 hour, or 30 minutes before the starting time of the administration of the other therapeutic agent(s). In certain embodiments, they may be administered concurrent with the administration of the other therapeutic agent(s). In other words, in these embodiments, SMSMs are administrated at the same time when the administration of the other therapeutic agent(s) starts. In other embodiments, SMSMs may be administered following the starting time of administration of the other therapeutic agent(s) (e.g., at least 30 minutes, 1 hour, 2 hours, 3 hours, 4 hours, 5 hours, 6 hours, 7 hours or 8 hours after the starting time of administration of the other therapeutic agents). Alternatively, SMSMs may be administered at least 30 minutes, 1 hour, 2 hours, 3 hours, 4 hours, 5 hours, 6 hours, 7 hours or 8 hours after the completion of administration of the toher therapeutic agents. Generally, these SMSMs are administered for a sufficient period of time so that the disease or condition is prevented or reduced. Such sufficient period of time may be identical to, or different from, the period during which other therapeutic agent(s) are administered. In certain embodiments, multiple doses of SMSMs are administered for each administration of another therapeutic agent or a combination of multiple other therapeutic agents.

In certain embodiments, an appropriate dosage of a SMSM is combined with a specific timing and/or a particular route to achieve the optimum effect in preventing or reducing the disease or condition. For instance, an SMSM may be administered to a human orally at least 1 hour, 2 hours, 3 hours, 4 hours, 5 hours, 6 hours, 7 hours 8 hours, 9 hours, 10 hours, 11 hours or 12 hours; or at least 1 day, 2 days, 3 days, 4 days, 5 days, 6 days, 7 days, 8 days, 9 days, 10 days, 11 days, 12 days; or at least 1 week, 2 weeks, 3 weeks or 4 weeks; or at least 1 month, 2 months, 3 months, 4 months, 5 months, 6 months, 7 months, 8 months, 9 months, 10 months, 11 months or 12 months; prior to or after the beginning or the completion, of the administration of another therapeutic agent or a combination of other therapeutic agents.

### Subjects

The subjects that can be treated with the SMSMs and methods described herein can be any subject that produces mRNA that is subject to alternative splicing, *e.g.,* the subject may be a eukaryotic subject, such as a plant or an animal. In some embodiments, the subject is a mammal, *e.g.,* human. In some embodiments, the subject is a human. In some embodiments, the subject is a non-human animal. In some embodiments, the subject is a fetus, an embryo, or a child. In some embodiments, the subject is a non-human primate such as chimpanzee, and other apes and monkey species; farm animals such as cattle, horses, sheep, goats, swine; domestic animals such as rabbits, dogs, and cats; laboratory animals including rodents, such as rats, mice and guinea pigs, and the like. In some embodiments, the subject is prenatal (*e.g.,* a fetus), a child (*e.g.,* a neonate, an infant, a toddler, a preadolescent), an adolescent, a pubescent, or an adult (*e.g.,* an early adult, a middle aged adult, a senior citizen).

### EXAMPLES

Compounds described herein can be synthesized using standard synthetic techniques or using methods known in the art in combination with methods described herein. Unless otherwise indicated, conventional methods of mass spectroscopy, NMR, HPLC, protein chemistry, biochemistry, recombinant DNA techniques and pharmacology can be employed. Compounds can be prepared using standard organic chemistry techniques such as those described in, for example, March's Advanced Organic Chemistry, 6th Edition, John Wiley and Sons, Inc. Alternative reaction conditions for the synthetic transformations described herein may be employed such as variation of solvent, reaction temperature, reaction time, as well as different chemical reagents and other reaction conditions. The starting materials and reagents used for the synthesis of the compounds described herein may be synthesized or can be obtained from commercial sources, such as, but not limited to, Sigma-Aldrich, Acros Organics, Fluka, and Fischer Scientific. The starting materials can be available from commercial sources or can be readily prepared. By way of example only, provided are schemes for preparing the Examples described herein.

The following abbreviations are used: DCM - dichloromethane; DIPEA - N,N-diisopropylethylamine; DMSO - dimethyl sulfoxide; DMF - N,N-dimethylformamide; EDCI - N-(3-Dimethylaminopropyl)-N'-ethylcarbodiimide; Et2O - diethyl ether; EtOAc - ethyl acetate; EtOH - ethyl alcohol; HOBt - 1-hydroxybenzotriazole; LCMS - liquid chromatography mass spectrometer; MeCN - acetonitrile; MeOH - methyl alcohol; Ms - mesylate; MTBE - methyl tert-butyl ether; Selectfluor - 1-Chloromethyl-4-fluoro-1,4-diazoniabicyclo[2.2.2]octane bis(tetrafluoroborate); SFC - supercritical fluid chromatography; THFtetrahydrofuran; TMSCl - trimethylsilyl chloride; h - hour; min - minute; rt - room temperature (22-25 °C); g - grams; mL - milliliters; mg - milligrams; mmol - millimoles.

Suitable reference books and treatise that detail the synthesis of reactants useful in the preparation of compounds described herein, or provide references to articles that describe the preparation, include for example, "Synthetic Organic Chemistry", John Wiley & Sons, Inc., New York; S. R. Sandler et al., "Organic Functional Group Preparations," 2nd Ed., Academic Press, New York, 1983; H. O. House, "Modern Synthetic Reactions", 2nd Ed., W. A. Benjamin, Inc. Menlo Park, Calif. 1972; T. L. Gilchrist, "Heterocyclic Chemistry", 2nd Ed., John Wiley & Sons, New York, 1992; J. March, "Advanced Organic Chemistry: Reactions, Mechanisms and Structure", 4th Ed., Wiley Interscience, New York, 1992. Additional suitable reference books and treatise that detail the synthesis of reactants useful in the preparation of compounds described herein, or provide references to articles that describe the preparation, include for example, Fuhrhop, J. and Penzlin G. "Organic Synthesis: Concepts, Methods, Starting Materials", Second, Revised and Enlarged Edition (1994) John Wiley & Sons ISBN: 3 527-29074-5; Hoffman, R.V. "Organic Chemistry, An Intermediate Text" (1996) Oxford University Press, ISBN 0-19-509618-5; Larock, R. C. "Comprehensive Organic Transformations: A Guide to Functional Group Preparations" 2nd Edition (1999) Wiley-VCH, ISBN: 0-471-19031-4; March, J. "Advanced Organic Chemistry: Reactions, Mechanisms, and Structure" 4th Edition (1992) John Wiley & Sons, ISBN: 0-471-60180-2; Otera, J. (editor) "Modern Carbonyl Chemistry" (2000) Wiley-VCH, ISBN: 3-527-29871-1; Patai, S. "Patai's 1992 Guide to the Chemistry of Functional Groups" (1992) Interscience ISBN: 0-471-93022-9; Solomons, T. W. G. "Organic Chemistry" 7th Edition (2000) John Wiley & Sons, ISBN: 0-471-19095-0; Stowell, J.C., "Intermediate Organic Chemistry" 2nd Edition (1993) Wiley-Interscience, ISBN: 0-471-57456-2; "Industrial Organic Chemicals: Starting Materials and Intermediates: An Ullmann's Encyclopedia" (1999) John Wiley & Sons, ISBN: 3-527-29645-X, in 8 volumes; "Organic Reactions" (1942-2000) John Wiley & Sons, in over 55 volumes; and "Chemistry of Functional Groups" John Wiley & Sons, in 73 volumes.

In the reactions described, it may be necessary to protect reactive functional groups, for example hydroxy, amino, imino, thio or carboxy groups, where these are desired in the final product, in order to avoid their unwanted participation in reactions. A detailed description of techniques applicable to the creation of protecting groups and their removal are described in Greene and Wuts, Protective Groups in Organic Synthesis, 3rd Ed., John Wiley & Sons, New York, NY, 1999, and Kocienski, Protective Groups, Thieme Verlag, New York, NY, 1994).

Examples can be made using known techniques and further chemically modified, in some embodiments, to facilitate intranuclear transfer to, *e.g.,* a splicing complex component, a spliceosome or a pre-mRNA molecule. One of ordinary skill in the art will appreciate the standard medicinal chemistry approaches for chemical modifications for intranuclear transfer (*e.g.,* reducing charge, optimizing size, and/or modifying lipophilicity).

### Stereochemistry:

(±) or racemic indicates that the product is a racemic mixture of enantiomers. For example (±) (1*S,*2*S,*3*R,*5*R*) or racemic (1*S,*2*S,*3*R,*5*R*) indicates that the relative product stereochemistry shown is based on known stereochemistry of similar compounds and or reactions and the product is a racemic mixture of enantiomers of both (1*S,*2*S,*3*R,*5*R*) and (1*R*,2*R*,3*S*,5*S*) stereoisomers. A compound in which the absolute stereochemistry of separated enantiomers is undetermined is represented as being either of the single enantiomers, for example (1*S,*2*S,*3*R,*5*R*) or (1*R,*2*R,*3*S,*5*S*) or drawn as being either possible single enantiomer. In such cases, the product is pure and a single enantiomer, but absolute stereochemistry is not identified, but relative stereochemistry is known and indicated.

### Example 1: Preparation of 6-(6-(((1S,2S,3R,55R)-2-fluoro-8-azabicyclo[3.2.1]octan-3-yl)(methyl)amino)-1,2,4-triazin-3-yl)-7-hydroxy-2-methyl-4H-chromen-4-one and 6-(6-(((1R,2R,3S,SS)-2-fluoro-8-azabicyclo[3.2.1]octan-3-yl)(methyl)amino)-1,2,4-triazin-3-yl)-7-hydroxy-2-methyl-4H-chromen-4-one (Compound 1A & 1B).

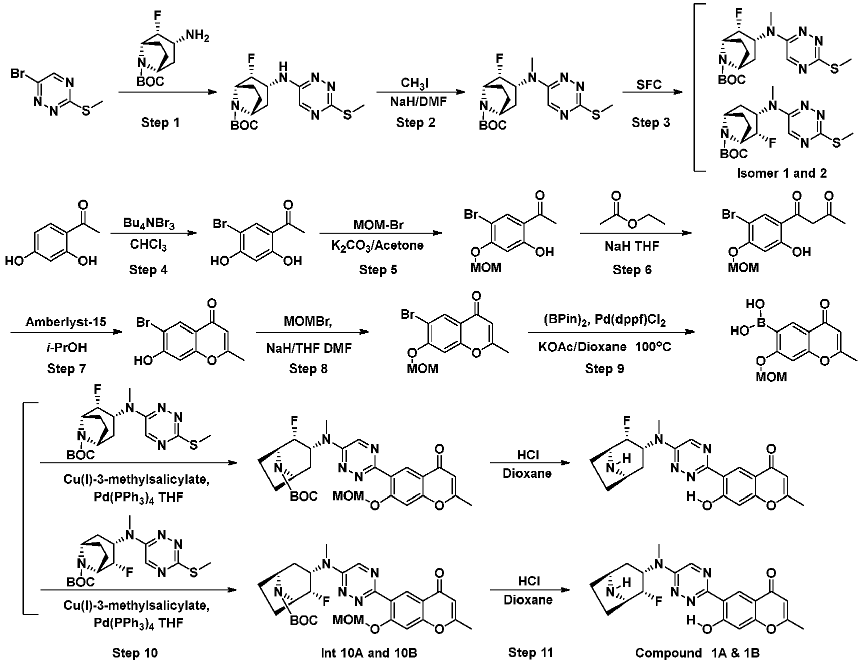

### Step 1: Preparation of racemic tert-butyl (1S,2R,3R,SR)-2-fluoro-3-((3-(methylthio)-1,2,4-triazin-6-yl)amino)-8-azabicyclo[3.2.1]octane-8-carboxylate, and tert-butyl (1R,2S,3S,5S)-2-fluoro-3-((3-(methylthio)-1,2,4-triazin-6-yl)amino)-8-azabicyclo[3.2.1]octane-8-carboxylate.

To a 1000 mL 3-necked round-bottom flask was added 6-bromo-3-(methylsulfanyl)-1,2,4-triazine (24.00 g, 116.5 mmol), a racemic mixture of tert-butyl (1S,2R,3R,5R)-3-amino-2-fluoro-8-azabicyclo[3.2.1]octane-8-carboxylate and tert-butyl (1R,2S,3S,5S)-3-amino-2-fluoro-8-azabicyclo[3.2.1]octane-8-carboxylate (43.0 g, 176 mmol), DIEA(60 mL, 344.5 mmol) and n-BuOH (360 mL) under nitrogen. The resulting solution was placed in a preheated oil bath stirred at 120°C for 2 hours under nitrogen. The reaction mixture was removed from heat and cooled to room temperature and diluted with water (700 mL) and then extracted with ethyl acetate (3 x 250 mL). The organic layers were combined, washed with brine (250 mL), and dried over anhydrous Na₂SO₄. The mixture was filtered and then concentrated under reduced pressure. The residue was purified by silica gel column chromatography (CH₂Cl₂/ EtOAc (4:1) which gave a racemic mixture of tert-butyl (1S,2R,3R,5R)-2-fluoro-3-((3-(methylthio)-1,2,4-triazin-6-yl)amino)-8-azabicyclo[3.2.1]octane-8-carboxylate, and tert-butyl (1R,2S,3S,5S)-2-fluoro-3-((3-(methylthio)-1,2,4-triazin-6-yl)amino)-8-azabicyclo[3.2.1]octane-8-carboxylate as a yellow solid (24g, 55.8%) [M+H]+=370.

### Step 2: Preparation of racemic mixture of tert-butyl (1S,2R,3R,5R)-2-fluoro-3-(methyl(3-(methylthio)-1,2,4-triazin-6-yl)amino)-8-azabicyclo[3.2.1]octane-8-carboxylate, tert-butyl (1R,2S,3S,SS)-2-fluoro-3-(methyl(3-(methylthio)-1,2,4-triazin-6-yl)amino)-8-azabicyclo[3.2.1]octane-8-carboxylate.

To a 1000 mL 3-necked round-bottom flask was added tert-butyl (1S,2R,3R,5R)-2-fluoro-3-((3-(methylthio)-1,2,4-triazin-6-yl)amino)-8-azabicyclo[3.2.1]octane-8-carboxylate, and tert-butyl (1R,2S,3S,5S)-2-fluoro-3-((3-(methylthio)-1,2,4-triazin-6-yl)amino)-8-azabicyclo[3.2.1]octane-8-carboxylate (21 g, 56.84 mmol) and dimethylformamide (420 mL) under nitrogen. The resultant mixture was cooled to 0°C followed by portion wise addition of sodium hydride (4.54 g, 113.51 mmol, 60%) with the internal temperature maintained at 0°C under nitrogen. The cooling bath was removed, and the mixture was stirred for additional 0.5 h and allowed to come to room temperature under nitrogen. Methyl iodide (7.20 mL, 115.66 mmol) was added dropwise at room temperature with stirring. The resulting mixture was stirred for additional 1 h at room temperature. The reaction mixture was then cooled to 0°C and quenched with water (1 L). The resulting mixture was extracted with ethyl acetate (3 x 250 mL). The combined organic layers were washed with water (2x250 mL) and saturated brine (250 mL), and then dried over anhydrous Na₂SO₄. After filtration, the filtrate was concentrated under reduced pressure and purified by silica gel column chromatography (CH₂Cl₂/EtOAc (19:1)) which gave 17 g (78%) of tert-butyl (1S,2R,3R,5R)-2-fluoro-3-(methyl(3-(methylthio)-1,2,4-triazin-6-yl)amino)-8-azabicyclo[3.2.1]octane-8-carboxylate and tert-butyl (1R,2S,3S,5S)-2-fluoro-3-(methyl(3-(methylthio)-1,2,4-triazin-6-yl)amino)-8-azabicyclo[3.2.1]octane-8-carboxylate as a racemic yellow solid, [M+H]⁺=384.

### Step 3: Chiral Separation of tert-butyl (1S,2R,3R,5R)-2-fluoro-3-(methyl(3-(methylthio)-1,2,4-triazin-6-yl)amino)-8-azabicyclo[3.2.1]octane-8-carboxylate and tert-butyl (1R,2S,3S,5S)-2-fluoro-3-(methyl(3-(methylthio)-1,2,4-triazin-6-yl)amino)-8-azabicyclo[3.2.1]octane-8-carboxylate.

The racemic mixture of tert-butyl (1S,2R,3R,5R)-2-fluoro-3-(methyl(3-(methylthio)-1,2,4-triazin-6-yl)amino)-8-azabicyclo[3.2.1]octane-8-carboxylate and tert-butyl (1R,2S,3S,5S)-2-fluoro-3-(methyl(3-(methylthio)-1,2,4-triazin-6-yl)amino)-8-azabicyclo[3.2.1]octane-8-carboxylate (11.00 g, 28.684 mmol) was purified into individual isomers by Prep-SFC. The following conditions were used (Column: CHIRALPAK IG, 2*25cm,5um; Mobile Phase A: CO₂, Mobile Phase B: EtOH--HPLC; Flow rate: 40 mL/min; Gradient: 50% B; 250 nm; RT1: 3.99 min; RT2: 6.19 min; Injection volume: 4 ml; Number Of Runs: 80;) which gave isomer 1 (5.0 g) as a yellow solid and isomer 2 (5.2g) as a yellow solid.

### Step 4: Preparation of 1-(5-bromo-2,4-dihydroxyphenyl)ethenone.

To a 2000-mL 3-necked round-bottom flask, was added 2,4-dihydroxyacetophenone (35.00 g, 230.04 mmol), tetrabutylammonium tribromide (122.01 g, 253.04 mmol), and CHCl₃ (600 mL). The resulting solution was stirred for 2 hours at room temperature. The reaction was then quenched with water (400 mL). The organic phase was separated, and the resulting aqueous phase was extracted with 2x300 mL of dichloromethane. The organic phases were combined, dried over anhydrous sodium sulfate and concentrated. The residue was chromatographed (silica gel: ethyl acetate/petroleum ether (20/80)) which after isolation gave 37 g of 1-(5-bromo-2,4-dihydroxyphenyl)ethanone as a white solid.

### Step 5: Preparation of 1-[5-bromo-2-hydroxy-4-(methoxymethoxy)phenyl]ethenone.

To a 2000-mL 3-necked round-bottom flask, was added 1-(5-bromo-2,4-dihydroxyphenyl)ethanone (37 g, 160.14 mmol), acetone (500 mL), potassium carbonate (35.41 g, 256.23 mmol), and bromomethyl methyl ether (25.81 g, 208.19 mmol). The resulting mixture was stirred overnight at room temperature. The solids were filtered, and the filtrate was then concentrated. The resulting residue was combined with 400 mL of H₂O and extracted with 2x500 mL of dichloromethane. The organic phases were combined, dried with sodium sulfate-anhydrous, filtered, concentrated and chromatographed (silica gel column: petroleum ether/ethyl acetate (20/1) which gave after isolation approximately 30 g of 1-[5-bromo-2-hydroxy-4-(methoxymethoxy)phenyl]ethanone as a white solid.

### Step 6: Preparation of 1-[5-bromo-2-hydroxy-4-(methoxymethoxy)phenyl]butane-1,3-dione.

To a 1000-mL 3-necked round-bottom flask, was added 1-[5-bromo-2-hydroxy-4-(methoxymethoxy)phenyl] ethanone (28 g, 101.78 mmol), tetrahydrofuran (300 mL). To this mixture under dry nitrogen atmosphere was added portion-wise with stirring, sodium hydride (9.77 g, 407.13 mmol) at 0°C. The resulting mixture was stirred at 0°C for 1hour followed by the addition of dry ethyl acetate (17.94 g, 203.57 mmol). After ethyl acetate addition, the cooling bath was removed and resulting mixture was then stirred for 2 hours. The reaction was then carefully quenched by the addition of 100 mL of water. The resulting mixture was extracted with 2x200 mL of ethyl acetate, the organic phases were combined, dried over Na₂SO₄, filtered and concentrated which directly gave approximately 25 g of 1-[5-bromo-2-hydroxy-4-(methoxymethoxy)phenyl]butane-1,3-dione as a light yellow solid.

### Step 7: Preparation of 6-bromo-7-hydroxy-2-methylchromen-4-one.

To a 1000-mL round-bottom flask, was combined 1-[5-bromo-2-hydroxy-4-(methoxymethoxy)phenyl] butane-1,3-dione (25 g, 78.83 mmol), Amberlyst-15 (20.00 g), *i-*PrOH (300 mL). The resulting mixture was placed in a preheated oil bath and was stirred for 2 hours at reflux. The reaction was removed from heat, allowed to cool. The solids were filtered and washed with 3x200 ml MeOH. The filtrates were combined and concentrated which gave a residue which upon drying solidified. The residue was purified by crystallization from methanol (~200 mL) which gave after isolation 15 g of 6-bromo-7-hydroxy-2-methylchromen-4-one as a grey solid.

### Step 8: Preparation of 6-bromo-7-(methoxymethoxy)-2-methylchromen-4-one.

To a 500-mL 3-necked round-bottom flask purged with nitrogen, was added 6-bromo-7-hydroxy-2-methylchromen-4-one (15 g, 58.81 mmol, 1.00), tetrahydrofuran (150 mL), and dimethylformamide (15 mL). The mixture was cooled to 0°C followed by the careful portion-wise addition of sodium hydride (2.12 g, 88.21 mmol) under nitrogen atmosphere. The resulting mixture was stirred at 0°C for 1hour followed by the addition of methoxymethyl bromide (11.02 g, 88.212 mmol). The cooling bath was removed, and the resulting mixture was then stirred for an additional 1 hour at ambient temperature. The reaction was quenched by the addition of water (100 mL), and the resulting solution was extracted with 2x200 mL of ethyl acetate. The combined organic phase was dried over Na₂SO₄, filtered and concentrated. The crude product solidified and was purified by re-crystallization from ethyl acetate/petroleum ether (4:1) which gave after isolation 15 g (85.27%) of 6-bromo-7-(methoxymethoxy)-2-methylchromen-4-one as a light pink solid.

### Step 9: Preparation of 7-(methoxymethoxy)-2-methyl-6-(4,4,5,5-tetramethyl-1,3,2-dioxaborolan-2-yl)chromen-4-one.

To a 100-mL round-bottom flask purged and maintained with an inert atmosphere of nitrogen, was added 6-bromo-7-(methoxymethoxy)-2-methylchromen-4-one (3.00 g, 10.03 mmol), Dioxane (30 mL), 4,4,4',4',5,5,5',5'-octamethyl-2,2'-bi(1,3,2-dioxaborolane) (3.06 g, 12.035 mmol), 1,1 -Bis(diphenylphosphino)ferrocene-palladium(II)dichloride dichloromethane complex (0.37 g, 0.501 mmol), and potassium acetate (1.97 g, 20.06 mmol). The resulting mixture was placed in a preheated oil bath and stirred at 100°C for 12 hours. The reaction was removed from heat, cooled and then quenched by the addition of 50 mL of water. The resulting mixture was extracted with 3x50 mL of ethyl acetate and the organic layers combined. The combined organic layers was washed with 1 x50 ml of brine, dried over anhydrous sodium sulfate, filtered and concentrated under vacuum. The residue was purified by silica gel chromatography (ethyl acetate/petroleum ether (0-80% gradient) which gave after isolation 0.8 g (23.04%) of 7-(methoxymethoxy)-2-methyl-6-(4,4,5,5-tetramethyl-1,3,2-dioxaborolan-2-yl)chromen-4-one as a yellow solid.

### Step 10: Preparation of tert-butyl (1S,2R,3R,5R)-2-fluoro-3-((3-(7-(methoxymethoxy)-2-methyl-4-oxo-4H-chromen-6-yl)-1,2,4-triazin-6-yl)(methyl)amino)-8-azabicyclo[3.2.1]octane-8-carboxylate and tert-butyl (1R,2S,3S,5S)-2-fluoro-3-((3-(7-(methoxymethoxy)-2-methyl-4-oxo-4H-chromen-6-yl)-1,2,4-triazin-6-yl)(methyl)amino)-8-azabicyclo[3.2.1]octane-8-carboxylate.

To an 8-mL vial purged and maintained with an inert atmosphere of nitrogen, was added Isomer 1 (150 mg, 0.391 mmol), tetrahydrofuran (3.00 mL), 7-(methoxymethoxy)-2-methyl-6- (4,4,5,5-tetramethyl-1,3,2-dioxaborolan-2-yl)chromen-4-one (406.23 mg, 1.173 mmol), copper(I)-3-methylsalicylate (251.92 mg, 1.173 mmol), and tetrakis(triphenylphosphine)palladium(0) (22.60 mg, 0.020 mmol). The resulting mixture was placed in a preheated oil bath and stirred at 70°C for 12 hours under nitrogen. The reaction mixture was then removed from heat and allowed to cool. The reaction was then quenched by the addition of 20 mL of water. The resulting solution was extracted with 3x20 mL of ethyl acetate and the organic layers were combined. The resulting organic mixture was washed with brine (20 ml), dried over anhydrous sodium sulfate, filtered, and concentrated under vacuum. The residue was purified by silica gel chromatography (ethyl acetate/petroleum ether (0-80%) gradient), which gave after isolation 80 mg (36.8%) of INT 10A as a yellow solid.

Following the procedure above, but starting with Isomer 2 (150 mg, 0.391 mmol) which gave after isolation gave 81 mg of INT 10B as a yellow solid.

### Step 11: Preparation of 6-(6-(((1S,2S,3R,5R)-2-fluoro-8-azabicyclo[3.2.1]octan-3-yl)(methyl)amino)-1,2,4-triazin-3-yl)-7-hydroxy-2-methyl-4H-chromen-4-one and 6-(6-(((1R,2R,3S,5S)-2-fluoro-8-azabicyclo[3.2.1]octan-3-yl)(methyl)amino)-1,2,4-triazin-3-yl)-7-hydroxy-2-methyl-4H-chromen-4-one.

To an 8-mL round-bottom flask purged and maintained with an inert atmosphere of nitrogen, was added INT 10A (80.00 mg, 0.144 mmol) and HCl in dioxane (2.00 mL). The resulting mixture was stirred for 3 hours at room temperature. The reaction was then quenched by the addition of 20 mL of saturated aqueous sodium bicarbonate. The resulting mixture was extracted with dichloromethane (3x20 mL) and the organic layers were combined. The resulting mixture was then washed with saturated brine (20 ml). The organic mixture was dried over anhydrous sodium sulfate, filtered, and concentrated under vacuum. The resultant residue was purified by Flash-Prep-HPLC (Column, C18; mobile phase, 0.01% NH₄HCO₃/H₂O:ACN=20% increasing to 0.01% NH₄HCO₃/H₂O:ACN=60% within 30 min; Detector, 254 nm which gave after isolation 25 mg of Compound 1A 6 as a yellow solid, (ES, m/z) [M+H]⁺=412.20. ¹H-NMR (300 MHz, DMSO-d6) δ 8.91 - 8.80 (m, 1H), 8.53 (s, 1H), 7.10 (s, 1H), 6.40 - 5.91 (m, 1H), 5.30 (d, J = 15.5 Hz, 1H), 4.75 (dd, J = 49.2, 7.7 Hz, 1H), 3.66 (s, 2H), 3.18 - 2.92 (m, 3H), 2.33 (d, J = 31.4 Hz, 3H), 2.11 (d, J = 10.2 Hz, 1H), 1.85 - 1.72 (m, 4H), 1.34 (s, 1H).

Following the procedure above, but starting with INT 10B gave 25 mg of Compound 1B as a yellow solid (ES, m/z) [M+H]⁺=412.20. ¹H-NMR (300 MHz, DMSO-d6) δ 8.85 (d, J = 8.9 Hz, 1H), 8.53 (s, 1H), 7.11 (s, 1H), 6.39 - 5.93 (m, 1H), 5.34 - 4.90 (m, 1H), 4.86 - 4.55 (m, 1H), 3.61 (d, J = 28.7 Hz, 2H), 3.19 - 2.92 (m, 3H), 2.33 (d, J = 31.8 Hz, 3H), 2.23 (s, 1H), 1.88 - 1.50 (m, 4H), 1.29 (d, J = 29.6 Hz, 1H).

### Example 2: Preparation of 6-(6-[[(1S,2S,3R,SR)-2-fluoro-8-azabicyclo[3.2.1]octan-3-yl](methyl)amino] -1,2,4-triazin-3-yl) -7-hydroxy-2-methylphthalazin-1-one and 6-(6-[[(1R,2R,3S,SS)-2-fluoro-8-azabicyclo[3.2.1]octan-3-yl](methyl)amino]-1,2,4-triazin-3-yl)-7-hydroxy-2-methylphthalazin-1-one (Compound 2A & 2B).

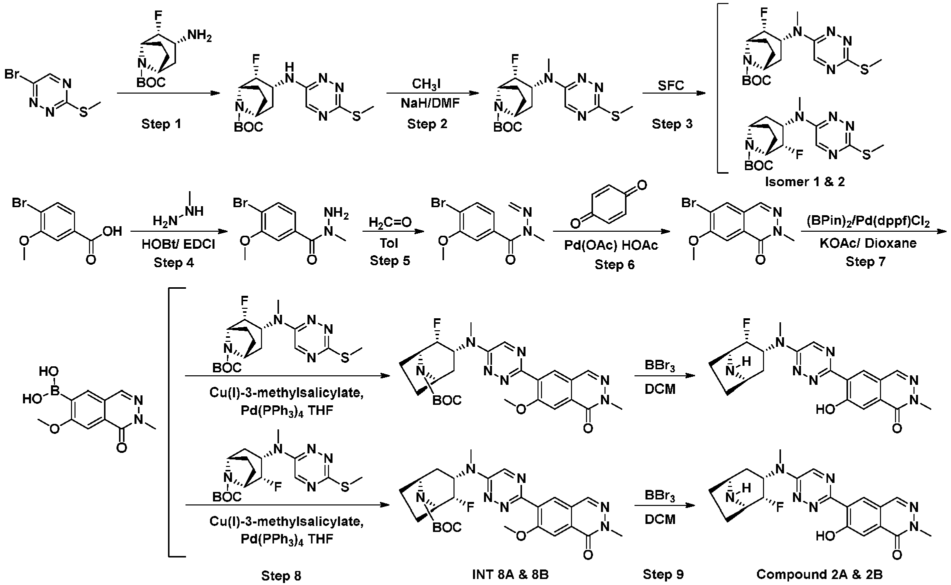

### Step 1: Preparation of racemic tert-butyl (1S,2R,3R,SR)-2-fluoro-3-((3-(methylthio)-1,2,4-triazin-6-yl)amino)-8-azabicyclo[3.2.1]octane-8-carboxylate, and tert-butyl (1R,2S,3S,5S)-2-fluoro-3-((3-(methylthio)-1,2,4-triazin-6-yl)amino)-8-azabicyclo[3.2.1]octane-8-carboxylate.

6-bromo-3-(methylsulfanyl)-1,2,4-triazine (24.00 g, 116.5 mmol), a racemic mixture of tert-butyl (1S,2R,3R,5R)-3-amino-2-fluoro-8-azabicyclo[3.2.1]octane-8-carboxylate and tert-butyl (1R,2S,3S,5S)-3-amino-2-fluoro-8-azabicyclo[3.2.1]octane-8-carboxylate (43.0 g, 176 mmol), DIEA(60 mL, 344.5 mmol) and n-BuOH (360 mL) was added to a 1000ml 3-necked round bottom flask under nitrogen. The resulting solution was placed in a preheated oil bath stirred at 120°C for 2 hours under nitrogen. The reaction mixture was removed from heat and cooled to room temperature and diluted with water (700 mL) and then extracted with ethyl acetate (3 x 250 mL). The organic layers were combined, washed with brine (250 mL), and dried over anhydrous Na₂SO₄. The mixture was filtered and then concentrated under reduced pressure. The residue was purified by silica gel column chromatography (CH₂Cl₂/ EtOAc (4:1) which gave a racemic mixture of tert-butyl (1S,2R,3R,5R)-2-fluoro-3-((3-(methylthio)-1,2,4-triazin-6-yl)amino)-8-azabicyclo[3.2.1]octane-8-carboxylate, and tert-butyl (1R,2S,3S,5S)-2-fluoro-3-((3-(methylthio)-1,2,4-triazin-6-yl)amino)-8-azabicyclo[3.2.1]octane-8-carboxylate as a yellow solid (24g, 55.8%) [M+H]+=370.

### Step 2: Preparation of racemic mixture of tert-butyl (1S,2R,3R,5R)-2-fluoro-3-(methyl(3-(methylthio)-1,2,4-triazin-6-yl)amino)-8-azabicyclo[3.2.1]octane-8-carboxylate, tert-butyl (1R,2S,3S,5S)-2-fluoro-3-(methyl(3-(methylthio)-1,2,4-triazin-6-yl)amino)-8-azabicyclo[3.2.1]octane-8-carboxylate.

tert-butyl (1S,2R,3R,5R)-2-fluoro-3-((3-(methylthio)-1,2,4-triazin-6-yl)amino)-8-azabicyclo[3.2.1]octane-8-carboxylate, and tert-butyl (1R,2S,3S,5S)-2-fluoro-3-((3-(methylthio)-1,2,4-triazin-6-yl)amino)-8-azabicyclo[3.2.1]octane-8-carboxylate (21 g, 56.84 mmol) and dimethylformamide (420 mL) was added to a 1000mL 3-necked round-bottom flask under nitrogen. The resultant mixture was cooled to 0°C followed by portion wise addition of sodium hydride (4.54 g, 113.51 mmol, 60%) with the internal temperature maintained at 0°C under nitrogen. The cooling bath was removed, and the mixture was stirred for additional 0.5 h and allowed to come to room temperature under nitrogen. Methyl iodide (7.20 mL, 115.66 mmol) was added dropwise at room temperature with stirring. The resulting mixture was stirred for additional 1 h at room temperature. The reaction mixture was then cooled to 0°C and quenched with water (1 L). The resulting mixture was extracted with ethyl acetate (3 x 250 mL). The combined organic layers were washed with water (2x250 mL) and saturated brine (250 mL), and then dried over anhydrous Na₂SO₄. After filtration, the filtrate was concentrated under reduced pressure and purified by silica gel column chromatography (CH₂Cl₂/EtOAc (19:1)) which gave 17 g (78%) of tert-butyl (1S,2R,3R,5R)-2-fluoro-3-(methyl(3-(methylthio)-1,2,4-triazin-6-yl)amino)-8-azabicyclo[3.2.1]octane-8-carboxylate and tert-butyl (1R,2S,3S,5S)-2-fluoro-3-(methyl(3-(methylthio)-1,2,4-triazin-6-yl)amino)-8-azabicyclo[3.2.1]octane-8-carboxylate as a racemic yellow solid, [M+H]⁺=384.

### Step 3: Chiral Separation of tert-butyl (1S,2R,3R,5R)-2-fluoro-3-(methyl(3-(methylthio)-1,2,4-triazin-6-yl)amino)-8-azabicyclo[3.2.1]octane-8-carboxylate and tert-butyl (1R,2S,3S,5S)-2-fluoro-3-(methyl(3-(methylthio)-1,2,4-triazin-6-yl)amino)-8-azabicyclo[3.2.1]octane-8-carboxylate.

The racemic mixture of tert-butyl (1S,2R,3R,5R)-2-fluoro-3-(methyl(3-(methylthio)-1,2,4-triazin-6-yl)amino)-8-azabicyclo[3.2.1]octane-8-carboxylate and tert-butyl (1R,2S,3S,5S)-2-fluoro-3-(methyl(3-(methylthio)-1,2,4-triazin-6-yl)amino)-8-azabicyclo[3.2.1]octane-8-carboxylate (11.00 g, 28.684 mmol) was purified into individual isomers by Prep-SFC. The following conditions were used (Column: CHIRALPAK IG, 2*25cm,5um; Mobile Phase A: CO₂, Mobile Phase B: EtOH--HPLC; Flow rate: 40 mL/min; Gradient: 50% B; 250 nm; RT1: 3.99 min; RT2: 6.19 min; Injection volume: 4 ml; Number Of Runs: 80;) which gave Isomer 1 (5.0 g) as a yellow solid and Isomer 2 (5.2g) as a yellow solid.

### Step 4: Preparation of 4-bromo-3-methoxy-N-methylbenzohydrazide.

To a 500-mL 3-necked round-bottom flask that was purged and maintained with an inert atmosphere of nitrogen, was added 4-bromo-3-methoxybenzoic acid (15.00 g, 65 mmol), methyl hydrazine (11.96 g, 260 mmol), DMF (150 mL), EDCI (14.93 g, 78 mmol and HOBT (10.53 g, 78 mmol) at 0°C under nitrogen. The resulting solution was stirred for 4 h under nitrogen and allowed to come to room temperature. The reaction was then quenched with 300 mL of water. The mixture was extracted with 3x200 mL of ethyl acetate. The organic layers were combined and washed with 3 x500 ml of saturated brine. The mixture was then dried over anhydrous sodium sulfate and concentrated. The crude product was purified by Flash-Prep-HPLC (IntelFlash-1: Column: C18 Column; mobile phase A: Water (10MMOL/L NH₄HCO₃), Mobile Phase B: ACN; Flow rate: 80 mL/min; Gradient:20 B to 80 B in 25 min) which gave 12.0 g of 4-bromo-3-methoxy-N-methylbenzohydrazide as light yellow oil.

### Step 5: Preparation of 4-bromo-3-methoxy-N-methyl- N'-methylidenebenzohydrazide.

To a nitrogen purged 250 mL 3-necked round-bottom flask was added 4-bromo-3-methoxy-N-methylbenzohydrazide (12.00 g, 46 mmol), hydroxymethylidene (1.67 g, 0.055 mmol), and toluene (150 mL) under nitrogen. The resulting mixture was placed in a preheated oil bath at 120°C and stirred for 16 hours. The reaction mixture was removed from heat and cooled in a water/ice bath. The mixture was then concentrated to a residue and chromatographed (silica gel column: ethyl acetate/petroleum ether (1:3)) which gave 12 g (95.57%) of 4-bromo-3-methoxy-N-methyl- N'-methylidenebenzohydrazide as a white solid.

### Step 6: Preparation of 6-bromo-7-methoxy-2-methylphthalazin-1-one.

To a 500-mL 3-necked round-bottom flask purged with dry nitrogen was placed 4-bromo-3-methoxy-N-methyl-N (10 g, 36.89 mmol), quinone (5.98 g, 55.33 mmol), palladium acetate (0.83 g, 3.69 mmol), and acetic acid (200 mL). The resulting solution was placed in a preheated oil bath and stirred at 120°C for 12 hours under nitrogen. The reaction mixture was removed from heat and cooled with a water/ice bath. The resulting mixture was concentrated, and the pH of the residue was adjusted to 8 with NaHCO₃. The resulting mixture was extracted with 3x150 mL of ethyl acetate, the organic layers were combined and dried over anhydrous sodium sulfate. The mixture was filtered, concentrated and purified (silica gel: ethyl acetate/petroleum ether (1:3)) which gave 6 g (60.45%) of 6-bromo-7-methoxy-2-methylphthalazin-1-one as a light yellow solid.

### Step 7: Preparation of 7-methoxy-2-methyl-1-oxophthalazin-6-ylboronic acid.

To a 500-mL 3-necked round-bottom flask purged with dry nitrogen was placed 6-bromo-7-methoxy-2-methylphthalazin-1-one (6 g, 22 mmol), 4,4,4',4',5,5,5',5'-octamethyl-2,2'-bi(1,3,2-dioxaborolane) (8.5 g, 33 mmol), 1,1 -Bis(diphenylphosphino)ferrocene-palladium(II)dichloride dichloromethane complex (0.91 g, 1 mmol), potassium acetate (4.38 g, 44 mmol), and dioxane (120 mL). The resulting mixture was placed in a preheated oil bath and stirred at 100°C for 2 hours under nitrogen. The reaction mixture was removed from heat and cooled in a water/ice bath. The resulting mixture was concentrated and chromatographed on silica gel (1:2 ethyl acetate/petroleum ether). A solid residue was isolated and was recrystallized from ether-petroleum which gave after isolation 3.5 g of 7-methoxy-2-methyl-1-oxophthalazin-6-ylboronic acid as a white solid.

### Step 8: Preparation of tert-butyl (1S,2R,3R,5R)-2-fluoro-3-((3-(7-methoxy-2-methyl-1-oxo-1,2-dihydrophthalazin-6-yl)-1,2,4-triazin-6-yl)(methyl)amino)-8-azabicyclo[3.2.1]octane-8-carboxylate and tert-butyl (1R,2S,3S,5S)-2-fluoro-3-((3-(7-methoxy-2-methyl-1-oxo-1,2-dihydrophthalazin-6-yl)-1,2,4-triazin-6-yl)(methyl)amino)-8-azabicyclo[3.2.1]octane-8-carboxylate.

To a 40-mL sealed tube purged with nitrogen was placed Isomer 1 (150 mg, 0.391 mmol), 7-methoxy-2-methyl-1-oxophthalazin-6-yl boronic acid (183.1 mg, 0.782 mmol), tetrakis(triphenylphosphine)palladium(0) (22.6 mg, 0.02 mmol), copper(I)-3-methylsalicylate (168mg, 0.782 mmol), and tetrahydrofuran (3.00 mL). The resulting mixture was placed in a preheated oil bath and stirred at 70°C for 3 hours under nitrogen. The reaction mixture was removed from heat and cooled in a water/ice bath. The crude reaction was concentrated and purified directly by Flash-Prep-HPLC ((IntelFlash-1): Column: C18 Column; mobile phase A: Water (10MMOL/L NH₄HCO₃), Mobile Phase B: ACN; Flow rate: 80 mL/min; Gradient:20 B to 70 B in 25 min) which gave 100 mg of INT 8A as a yellow solid.

Following the procedure above, but starting with Isomer 2 (150 mg, 0.391 mmol) gave 100 mg (48.64%) of INT 8B as a yellow solid.

### Step 9: Preparation of 6-(6-(((1S,2S,3R,SR)-2-fluoro-8-azabicyclo[3.2.1]octan-3-yl)(methyl)amino)-1,2,4-triazin-3-yl)-7-hydroxy-2-methylphthalazin-1(2H)-one and 6-(6-(((1R,2R,3S,5S)-2-fluoro-8-azabicyclo[3.2.1]octan-3-yl)(methyl)amino)-1,2,4-triazin-3-yl)-7-hydroxy-2-methylphthalazin-1(2H)-one (Compound 2A & 2B).

To a 40-mL sealed tube purged with nitrogen was placed was placed INT 8A (120 mg, 0.228 mmol), and combined with dichloromethane (5 mL ) and stirred. Boron tribromide (3 mL) was added dropwise at 0°C and the resulting solution was stirred for 2 h at room temperature. The reaction was then quenched by the addition of 100 mL of saturated sodium bicarbonate. The resulting solution was extracted with 4x150 mL of dichloromethane/acetonitrile (10:1), dried over anhydrous sodium sulfate and concentrated. The crude product was purified by Flash-Prep-HPLC ((IntelFlash-1): Kinetex EVO C18 Column, 21.2*150,5um; Mobile Phase A: Water (10mmol/L NH₄HCO₃), Mobile Phase B: ACN; Flow rate: 25 mL/min; Gradient:5 B to 50 B in 30 min; 254/220 nm) which after isolation gave 46.9 mg of Compound 2A as a yellow solid, (ES, m/z): [M+H]+=412.2. 1H NMR (300 MHz, DMSO-d6) δ ppm 13.21 (s, 1H),8.90 (s, 1H), 8.86 (s, 1H), 8.47 (s, 1H), 7.65 (s, 1H), 4.97-4.80 (m, 1H), 4.76-4.63 (m, 1H), 3.71 (s, 3H), 3.64-3.56 (m, 2H), 3.18 (s, 3H), 2.33-2.25 (m, 1H), 1.81 - 1.60 (m, 5H).

Following the procedure above, but starting with INT 8B gave 21.1 mg (16.95%) of Compound 2B as a yellow solid (ES, m/z): [M+H]⁺=412.2. 1H NMR (300 MHz, DMSO-d6) δ ppm 13.23 (s, 1H),8.91 (s, 1H), 8.86 (s, 1H), 8.47 (s, 1H), 7.65 (s, 1H), 4.99-4.82 (m, 1H), 4.78-4.61 (m, 1H), 3.71 (s, 3H), 3.64-3.55 (m, 2H), 3.18 (s, 3H), 2.31-2.25 (m, 1H), 1.81 - 1.58 (m, 5H).

### Example 3: Preparation of 7-(6-(((1S,2S,3R,SR)-2-fluoro-8-azabicyclo[3.2.1]octan-3-yl)(methyl)amino)-1,2,4-triazin-3-yl)-6-hydroxy-3-methylquinazolin-4(3H)-one and 7-(6-(((1R,2R,3S,5S)-2-fluoro-8-azabicyclo[3.2.1]octan-3-yl)(methyl)amino)-1,2,4-triazin-3-yl)-6-hydroxy-3-methylquinazolin-4(3H)-one (Compound 3A & 3B).

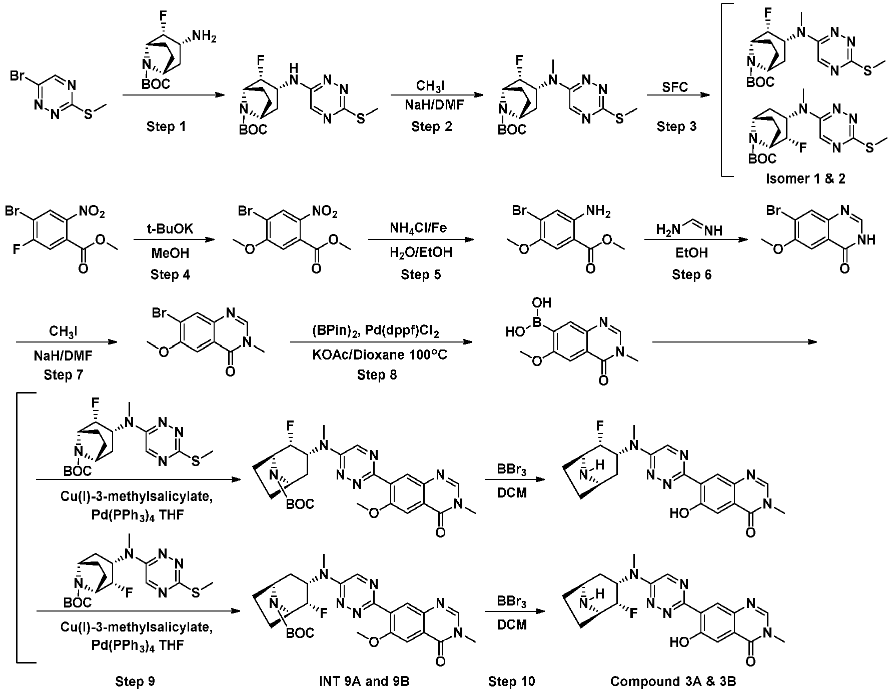

### Step 1: Preparation of racemic tert-butyl (1S,2R,3R,5R)-2-fluoro-3-((3-(methylthio)-1,2,4-triazin-6-yl)amino)-8-azabicyclo[3.2.1]octane-8-carboxylate, and tert-butyl (1R,2S,3S,5S)-2-fluoro-3-((3-(methylthio)-1,2,4-triazin-6-yl)amino)-8-azabicyclo[3.2.1]octane-8-carboxylate.

To a 1000 mL 3-necked round-bottom flask was added 6-bromo-3-(methylsulfanyl)-1,2,4-triazine (24.00 g, 116.5 mmol), a racemic mixture of tert-butyl (1S,2R,3R,5R)-3-amino-2-fluoro-8-azabicyclo[3.2.1]octane-8-carboxylate and tert-butyl (1R,2S,3S,5S)-3-amino-2-fluoro-8-azabicyclo[3.2.1]octane-8-carboxylate (43.0 g, 176 mmol), DIEA(60 mL, 344.5 mmol) and n-butanol (360 mL) under nitrogen. The resulting solution was placed in a preheated oil bath stirred at 120°C for 2 hours under nitrogen. The reaction mixture was removed from heat and cooled to room temperature and diluted with water (700 mL) and then extracted with ethyl acetate (3 x 250 mL). The organic layers were combined, washed with brine (250 mL), and dried over anhydrous Na₂SO₄. The mixture was filtered and then concentrated under reduced pressure. The residue was purified by silica gel column chromatography (CH₂Cl₂/ EtOAc (4:1) which gave a racemic mixture of tert-butyl (1S,2R,3R,5R)-2-fluoro-3-((3-(methylthio)-1,2,4-triazin-6-yl)amino)-8-azabicyclo[3.2.1]octane-8-carboxylate, and tert-butyl (1R,2S,3S,5S)-2-fluoro-3-((3-(methylthio)-1,2,4-triazin-6-yl)amino)-8-azabicyclo[3.2.1]octane-8-carboxylate as a yellow solid (24g, 55.8%) [M+H]+=370.

### Step 2: Preparation of racemic mixture of tert-butyl (1S,2R,3R,5R)-2-fluoro-3-(methyl(3-(methylthio)-1,2,4-triazin-6-yl)amino)-8-azabicyclo[3.2.1]octane-8-carboxylate, tert-butyl (1R,2S,3S,5S)-2-fluoro-3-(methyl(3-(methylthio)-1,2,4-triazin-6-yl)amino)-8-azabicyclo[3.2.1]octane-8-carboxylate.

To a 1000 mL 3-necked round-bottom flask was added tert-butyl (1S,2R,3R,5R)-2-fluoro-3-((3-(methylthio)-1,2,4-triazin-6-yl)amino)-8-azabicyclo[3.2.1]octane-8-carboxylate, and tert-butyl (1R,2S,3S,5S)-2-fluoro-3-((3-(methylthio)-1,2,4-triazin-6-yl)amino)-8-azabicyclo[3.2.1]octane-8-carboxylate (21 g, 56.84 mmol) and dimethylformamide (420 mL) under nitrogen. The resultant mixture was cooled to 0°C followed by portion wise addition of sodium hydride (4.54 g, 113.51 mmol, 60%) with the internal temperature maintained at 0°C under nitrogen. The cooling bath was removed, and the mixture was stirred for additional 0.5 h and allowed to come to room temperature under nitrogen. Methyl iodide (7.20 mL, 115.66 mmol) was added dropwise at room temperature with stirring. The resulting mixture was stirred for additional 1 h at room temperature. The reaction mixture was then cooled to 0°C and quenched with water (1 L). The resulting mixture was extracted with ethyl acetate (3 x 250 mL). The combined organic layers were washed with water (2x250 mL) and saturated brine (250 mL), and then dried over anhydrous Na₂SO₄. After filtration, the filtrate was concentrated under reduced pressure and purified by silica gel column chromatography (CH₂Cl₂/EtOAc (19:1)) which gave 17 g (78%) of tert-butyl (1S,2R,3R,5R)-2-fluoro-3-(methyl(3-(methylthio)-1,2,4-triazin-6-yl)amino)-8-azabicyclo[3.2.1]octane-8-carboxylate and tert-butyl (1R,2S,3S,5S)-2-fluoro-3-(methyl(3-(methylthio)-1,2,4-triazin-6-yl)amino)-8-azabicyclo[3.2.1]octane-8-carboxylate as a racemic yellow solid, [M+H]⁺=384.

### Step 3: Chiral Separation of tert-butyl (1S,2R,3R,5R)-2-fluoro-3-(methyl(3-(methylthio)-1,2,4-triazin-6-yl)amino)-8-azabicyclo[3.2.1]octane-8-carboxylate and tert-butyl (1R,2S,3S,5S)-2-fluoro-3-(methyl(3-(methylthio)-1,2,4-triazin-6-yl)amino)-8-azabicyclo[3.2.1]octane-8-carboxylate.

The racemic mixture of tert-butyl (1S,2R,3R,5R)-2-fluoro-3-(methyl(3-(methylthio)-1,2,4-triazin-6-yl)amino)-8-azabicyclo[3.2.1]octane-8-carboxylate and tert-butyl (1R,2S,3S,5S)-2-fluoro-3-(methyl(3-(methylthio)-1,2,4-triazin-6-yl)amino)-8-azabicyclo[3.2.1]octane-8-carboxylate (11.00 g, 28.684 mmol) was purified into individual isomers by Prep-SFC. The following conditions were used (Column: CHIRALPAK IG, 2*25cm,5um; Mobile Phase A: CO₂, Mobile Phase B: EtOH--HPLC; Flow rate: 40 mL/min; Gradient: 50% B; 250 nm; RT1: 3.99 min; RT2: 6.19 min; Injection volume: 4 ml; Number Of Runs: 80;) which gave isomer 1 (5.0 g) as a yellow solid and isomer 2 (5.2g) as a yellow solid.

### Step 4: Preparation of methyl 4-bromo-5-methoxy-2-nitrobenzoate.

To a 1000-mL 3-necked round-bottom flask, was added methyl 4-bromo-5-fluoro-2-nitrobenzoate (60.00 g, 215.8 mmol), methanol (600 mL), and potassium tert butoxide (29.06 g, 258.974 mmol). The mixture was stirred for 3 hours at room temperature. The mixture was then concentrated and washed with water (300 mL), extracted with dichloromethane (2x200 mL) of and the organic layers combined and then concentrated which gave after isolation 59 g (94.25%) of methyl 4-bromo-5-methoxy-2-nitrobenzoate as a solid.

### Step 5: Preparation of methyl 2-amino-4-bromo-5-methoxybenzoate.

To a 1000-mL 3-necked round-bottom flask purged and maintained with an inert atmosphere of nitrogen, was added methyl 4-bromo-5-methoxy-2-nitrobenzoate (59.00 g, 203.4 mmol), ethanol, water, acetic acid, and Zinc metal powder (26.61 g, 406.8 mmol). The resulting mixture was placed in a preheated oil bath and was stirred for 3 hours at 60°C then removed from heat, allowed to cool. The resulting solution was stirred for 3 hours at 60 degrees C. The mixture removed from heat, allowed to cool, and filtered. The resultant filtrate was concentrated and washed with saturated aqueous sodium bicarbonate (200 mL) and then extracted with dichloromethane (3x300 mL). The organic layers combined, dried over sodium sulfate, filtered and concentrated to a solid which gave after isolation 40 g (75.61%) of methyl 2-amino-4-bromo-5-methoxybenzoate as a solid that was used directly in the next reaction.

### Step 6: Preparation of 7-bromo-6-methoxy-3H-quinazolin-4-one.

To a 1000-mL 3-necked round-bottom flask purged and maintained with an inert atmosphere of nitrogen, was added methyl 2-amino-4-bromo-5-methoxybenzoate (34.00 g, 130.725 mmol), ethanol (340.00 mL), acetic acid; and formamidine (13.61 g, 130.728 mmol). The resulting mixture was placed in a preheated oil bath and was stirred for 3 hours at 80°C then removed from heat, allowed to cool. The reaction was then carefully quenched with saturated aqueous sodium bicarbonate (100 mL). The resulting mixture was concentrated and then extracted with dichloromethane (3x200 mL). The organic layers combined, dried over sodium sulfate, filtered and concentrated under reduced pressure which gave after isolation 32 g (95.97%) of 7-bromo-6-methoxy-3H-quinazolin-4-one as a solid that was used directly in the next reaction.

### Step 7: Preparation of 7-bromo-6-methoxy-3-methylquinazolin-4-one.

To a 250-mL 3-necked round-bottom flask purged and maintained with an inert atmosphere of nitrogen, was added dimethylformamide (50 mL), and sodium hydride (0.56 g, 23.523 mmol). The mixture was cooled to 0°C followed by the addition of 7-bromo-6-methoxy-3H-quinazolin-4-one (5 g, 19.602 mmol) at degrees C. The mixture was allowed to stir for 30 min followed by the addition of methyl iodide (2.78 g, 19.586 mmol). The cooling bath was removed and the mixture was stirred for an additional 2 hours at room temperature. The reaction was carefully quenched with ice/water (30 mL). The mixture was extracted with ethyl acetate (3x80 mL). The combined organic layers was washed with saturated brine (3x50 mL), dried over anhydrous sodium sulfate, filtered and concentrated which gave after isolation 4 g (75.83%) of 7-bromo-6-methoxy-3-methylquinazolin-4-one as a solid that was used directly in the next reaction.

### Step 8: Preparation of 6-methoxy-3-methyl-7-(4,4,5,5-tetramethyl-1,3,2-dioxaborolan-2-yl)quinazolin-4-one.

To a 40-mL vial purged and maintained with an inert atmosphere of nitrogen, was placed 7-bromo-6-methoxy-3-methylquinazolin-4-one (1.20 g, 4.459 mmol), 4,4,4',4',5,5,5',5'-octamethyl-2,2'-bi(1,3,2-dioxaborolane) (1.36 g, 5.351 mmol), 1,1 - Bis(diphenylphosphino)ferrocene-palladium(II)dichloride dichloromethane complex (0.16 g, 0.219 mmol), potassium acetate (0.88 g, 8.919 mmol), and dioxane (10 mL). The resulting mixture was placed in a preheated oil bath and stirred at 100°C for 12 hours. The reaction was removed from heat, allowed to cool, and concentrated under vacuum. The crude residue was purified by silica gel chromatography (ethyl acetate/petroleum ether (1:3)) which after isolation gave 400 mg (28.37%) of 6-methoxy-3-methyl-7-(4,4,5,5-tetramethyl-1,3,2-dioxaborolan-2-yl)quinazolin-4-one as a solid.

### Step 9: Preparation of tert-butyl (1S,2R,3R,5R)-2-fluoro-3-((3-(6-methoxy-3-methyl-4-oxo-3,4-dihydroquinazolin-7-yl)-1,2,4-triazin-6-yl)(methyl)amino)-8-azabicyclo[3.2.1]octane-8-carboxylate and tert-butyl (1R,2S,3S,5S)-2-fluoro-3-((3-(6-methoxy-3-methyl-4-oxo-3,4-dihydroquinazolin-7-yl)-1,2,4-triazin-6-yl)(methyl)amino)-8-azabicyclo[3.2.1]octane-8-carboxylate.

To an 8-mL vial purged and maintained with an inert atmosphere of nitrogen, was added Isomer 1 (120 mg, 0.313 mmol), copper(I)-3-methylsalicylate (167.95 mg, 0.782 mmol), 6-methoxy-3-methyl-7-(4,4,5,5-tetramethyl-1,3,2-dioxaborolan-2-yl)quinazolin-4-one (197.86 mg, 0.626 mmol), tetrakis(triphenylphosphine)palladium(0) (18.08 mg, 0.016 mmol), and tetrahydrofuran (1.20 mL). The resulting mixture was placed in a preheated oil bath and stirred at 60°C for 2 hours under nitrogen. The resulting mixture was concentrated and crude product was purified by Flash-Prep-HPLC ((IntelFlash-1): Column, C18 silica gel; mobile phase, ACN/5MMNH₄CO₃=0.2 increasing to ACN/5MMNH₄CO₃=1 within 15 min; Detector, 254 nm) which after isolation gave 120 mg (72.96%) of **INT 9A** as a solid.

Following the procedure above, but starting with Isomer 2 (120 mg, 0.313 mmol), which after isolation gave 86 mg (52.29%) of INT 9B as a yellow solid.

### Step 10: Preparation of 7-(6-(((1S,2S,3R,5R)-2-fluoro-8-azabicyclo[3.2.1]octan-3-yl)(methyl)amino)-1,2,4-triazin-3-yl)-6-hydroxy-3-methylquinazolin-4(3H)-one and 7-(6-(((1R,2R,3S,5S)-2-fluoro-8-azabicyclo[3.2.1]octan-3-yl)(methyl)amino)-1,2,4-triazin-3-yl)-6-hydroxy-3-methylquinazolin-4(3H)-one (Compound 3A & 3B).

To a 25-mL round-bottom flask purged and maintained with an inert atmosphere of nitrogen, was added **INT 9A** (110.00 mg, 0.209 mmol), dichloromethane (2.00 mL), and dropwise addition of boron tribromide in dichloromethane (2.00 mL). The resulting solution was stirred for 2 hours at room temperature. The reaction was then quenched by the addition of 2 mL of NaHCO₃.aq. The mixture was extracted with dichloromethane (3x10mL), the organic layers were combined, dried over sodium sulfate, filtered and concentrated. The crude product was purified by Flash-Prep-HPLC ((IntelFlash-1): Column, C18 silica gel; mobile phase, ACN/5MMNH₄CO₃=0.1 increasing to ACN/5MMNH₄CO₃=0.5 within 20 min; Detector, 254 nm) which after isolation gave 31.9 mg (37.05%) of **Compound 3A** as a solid (ES, m/z):[M+H]⁺=412. ¹H NMR (300 MHz, DMSO-d6) δ 12.67 (s, 1H), 8.87 (s, 1H), 8.44 (s, 1H), 8.24 (s, 1H), 7.57 (s, 1H), 4.91 (d, J = 30.9 Hz, 1H), 4.69 (d, J = 52.3 Hz, 1H), 3.54 (s, 2H), 3.50 (s, 3H), 3.17 (d, J = 1.7 Hz, 3H), 2.28 (t, J = 11.3 Hz, 1H), 1.85 - 1.51 (m, 6H). Following the procedure above, but starting with **INT 9B** gave 29.3 mg (43.52%) of **Compound 3B** as a yellow solid (ES, m/z):[M+H]⁺=412. ¹H NMR (300 MHz, DMSO-d6) δ 12.67 (s, 1H), 8.87 (s, 1H), 8.44 (s, 1H), 8.24 (s, 1H), 7.57 (s, 1H), 4.91 (d, J = 30.9 Hz, 1H), 4.69 (d, J = 52.3 Hz, 1H), 3.54 (s, 2H), 3.50 (s, 3H), 3.17 (d, J = 1.7 Hz, 3H), 2.28 (t, J = 11.3 Hz, 1H), 1.91 - 1.50 (m, 6H).

### Example 4: Preparation of 6-(6-(((1S,2S,3R,SR)-2-fluoro-8-azabicyclo[3.2.1]octan-3-yl)(methyl)amino)-1,2,4-triazin-3-yl)-7-hydroxy-2-methylisoquinolin-1(2H)-one and 6-(6-(((1R,2R,3S,5S)-2-fluoro-8-azabicyclo[3.2.1]octan-3-yl)(methyl)amino)-1,2,4-triazin-3-yl)-7-hydroxy-2-methylisoquinolin-1(2H)-one (Compound 4A & 4B).

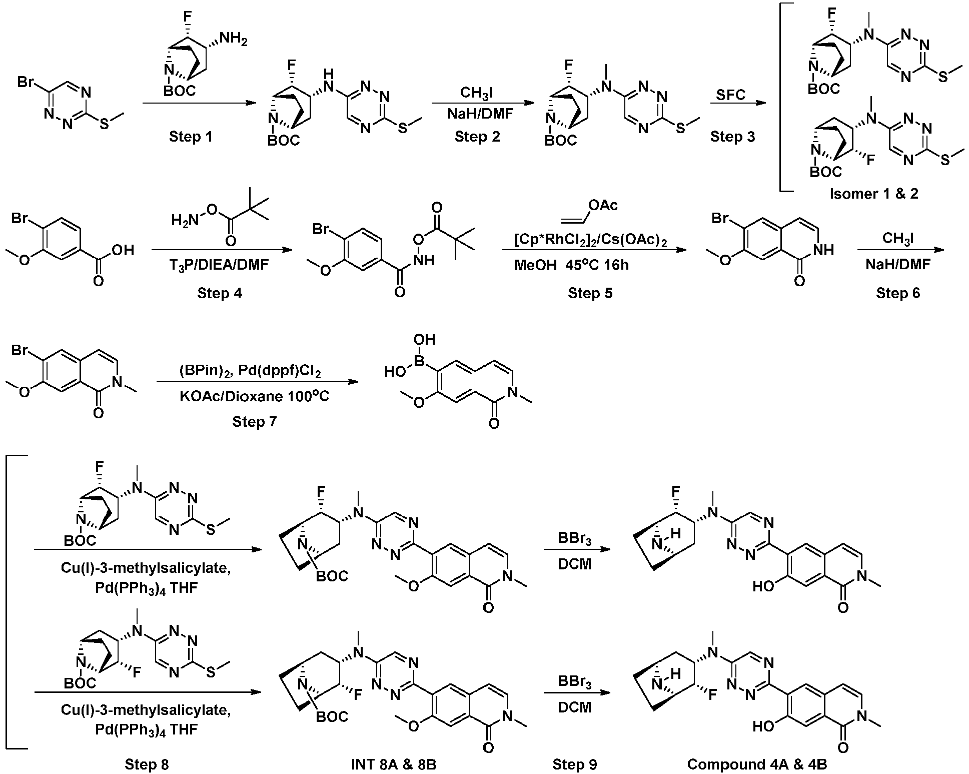

### Step 1: Preparation of racemic tert-butyl (1S,2R,3R,SR)-2-fluoro-3-((3-(methylthio)-1,2,4-triazin-6-yl)amino)-8-azabicyclo[3.2.1]octane-8-carboxylate, and tert-butyl (1R,2S,3S,5S)-2-fluoro-3-((3-(methylthio)-1,2,4-triazin-6-yl)amino)-8-azabicyclo[3.2.1]octane-8-carboxylate.

To a 1000 mL 3-necked round-bottom flask was added 6-bromo-3-(methylsulfanyl)-1,2,4-triazine (24.00 g, 116.5 mmol), a racemic mixture of tert-butyl (1S,2R,3R,5R)-3-amino-2-fluoro-8-azabicyclo[3.2.1]octane-8-carboxylate and tert-butyl (1R,2S,3S,5S)-3-amino-2-fluoro-8-azabicyclo[3.2.1]octane-8-carboxylate (43.0 g, 176 mmol), DIEA(60 mL, 344.5 mmol) and n-butanol (360 mL) under nitrogen. The resulting solution was placed in a preheated oil bath stirred at 120°C for 2 hours under nitrogen. The reaction mixture was removed from heat and cooled to room temperature and diluted with water (700 mL) and then extracted with ethyl acetate (3 x 250 mL). The organic layers were combined, washed with brine (250 mL), and dried over anhydrous Na₂SO₄. The mixture was filtered and then concentrated under reduced pressure. The residue was purified by silica gel column chromatography (CH₂Cl₂/ EtOAc (4:1) which gave a racemic mixture of tert-butyl (1S,2R,3R,5R)-2-fluoro-3-((3-(methylthio)-1,2,4-triazin-6-yl)amino)-8-azabicyclo[3.2.1]octane-8-carboxylate, and tert-butyl (1R,2S,3S,5S)-2-fluoro-3-((3-(methylthio)-1,2,4-triazin-6-yl)amino)-8-azabicyclo[3.2.1]octane-8-carboxylate as a yellow solid (24g, 55.8%) [M+H]+=370.

### Step 2: Preparation of racemic mixture of tert-butyl (1S,2R,3R,5R)-2-fluoro-3-(methyl(3-(methylthio)-1,2,4-triazin-6-yl)amino)-8-azabicyclo[3.2.1]octane-8-carboxylate, tert-butyl (1R,2S,3S,5S)-2-fluoro-3-(methyl(3-(methylthio)-1,2,4-triazin-6-yl)amino)-8-azabicyclo[3.2.1]octane-8-carboxylate.

To a 1000 mL 3-necked round-bottom flask was added tert-butyl (1S,2R,3R,5R)-2-fluoro-3-((3-(methylthio)-1,2,4-triazin-6-yl)amino)-8-azabicyclo[3.2.1]octane-8-carboxylate, and tert-butyl (1R,2S,3S,5S)-2-fluoro-3-((3-(methylthio)-1,2,4-triazin-6-yl)amino)-8-azabicyclo[3.2.1]octane-8-carboxylate (21 g, 56.84 mmol) and dimethylformamide (420 mL) under nitrogen. The resultant mixture was cooled to 0°C followed by portion wise addition of sodium hydride (4.54 g, 113.51 mmol, 60%) with the internal temperature maintained at 0°C under nitrogen. The cooling bath was removed, and the mixture was stirred for additional 0.5 h and allowed to come to room temperature under nitrogen. Methyl iodide (7.20 mL, 115.66 mmol) was added dropwise at room temperature with stirring. The resulting mixture was stirred for additional 1 h at room temperature. The reaction mixture was then cooled to 0°C and quenched with water (1 L). The resulting mixture was extracted with ethyl acetate (3 x 250 mL). The combined organic layers were washed with water (2x250 mL) and saturated brine (250 mL), and then dried over anhydrous Na₂SO₄. After filtration, the filtrate was concentrated under reduced pressure and purified by silica gel column chromatography (CH₂Cl₂/EtOAc (19:1)) which gave 17 g (78%) of tert-butyl (1S,2R,3R,5R)-2-fluoro-3-(methyl(3-(methylthio)-1,2,4-triazin-6-yl)amino)-8-azabicyclo[3.2.1]octane-8-carboxylate and tert-butyl (1R,2S,3S,5S)-2-fluoro-3-(methyl(3-(methylthio)-1,2,4-triazin-6-yl)amino)-8-azabicyclo[3.2.1]octane-8-carboxylate as a racemic yellow solid, [M+H]⁺=384.

### Step 3: Chiral Separation of tert-butyl (1S,2R,3R,5R)-2-fluoro-3-(methyl(3-(methylthio)-1,2,4-triazin-6-yl)amino)-8-azabicyclo[3.2.1]octane-8-carboxylate and tert-butyl (1R,2S,3S,5S)-2-fluoro-3-(methyl(3-(methylthio)-1,2,4-triazin-6-yl)amino)-8-azabicyclo[3.2.1]octane-8-carboxylate.

The racemic mixture of tert-butyl (1S,2R,3R,5R)-2-fluoro-3-(methyl(3-(methylthio)-1,2,4-triazin-6-yl)amino)-8-azabicyclo[3.2.1]octane-8-carboxylate and tert-butyl (1R,2S,3S,5S)-2-fluoro-3-(methyl(3-(methylthio)-1,2,4-triazin-6-yl)amino)-8-azabicyclo[3.2.1]octane-8-carboxylate (11.00 g, 28.684 mmol) was purified into individual isomers by Prep-SFC. The following conditions were used (Column: CHIRALPAK IG, 2*25cm,5um; Mobile Phase A: CO₂, Mobile Phase B: EtOH--HPLC; Flow rate: 40 mL/min; Gradient: 50% B; 250 nm; RT1: 3.99 min; RT2: 6.19 min; Injection volume: 4 ml; Number Of Runs: 80;) which gave isomer 1 (5.0 g) as a yellow solid and isomer 2 (5.2g) as a yellow solid.

### Step 4: Preparation of 4-bromo-3-methoxy-N-(pivaloyloxy)benzamide.

To a 500-mL 3-necked round-bottom flask purged and maintained with an inert atmosphere of nitrogen, was added 4-bromo-3-methoxybenzoic acid (20.00 g, 86.563 mmol), dimethylformamide (100.02 mL), diisopropylethylamine (89.50 g, 692.506 mmol), propanephosphonic acid anhydride (41.31 g, 129.8 mmol), and O-pivaloylhydroxylamine (15.21 g, 129.845 mmol). The resulting mixture was stirred for 2 hours at room temperature. The reaction was then quenched with water. The resulting mixture was extracted with ethyl acetate (5x300 mL). The organics were combined, dried over anhydrous Na₂SO₄, filtered, and concentrated to dryness under reduced pressure. The crude product was purified by preparative HPLC which gave after isolation in 9.7 g (33.94%) of 4-bromo-3-methoxy-N-(pivaloyloxy)benzamide as brown oil (ES, m/z): [M+H]+=330.1.

### Step 5: Preparation of 6-bromo-7-methoxy-2H-isoquinolin-1-one.

To a 250-mL 3-necked round-bottom flask purged and maintained with an inert atmosphere of nitrogen, was added 4-bromo-3-methoxy-N-(pivaloyloxy)benzamide (9.00 g, 27.258 mmol), methanol (100.00 mL), pentamethylcyclopentadienyl rhodium dichloride (0.17 g, 0.273 mmol), vinyl acetate (3.52 g, 40.887 mmol), and cesium acetate (1.57 g, 8.177 mmol). The resulting mixture was placed in a preheated oil bath at 45°C, stirred for 16 hours and then removed from heat, and allowed to cool. The resulting mixture was concentrated under vacuum which gave a solid. The crude product was purified by re-crystallization from MTBE. The solids were collected by filtration and dried under vacuum which gave 5.3 g (76.53%) of 6-bromo-7-methoxy-2H-isoquinolin-1-one as an off-white solid (ES, m/z): [M+H]+=254.0.

### Step 6: Preparation of 6-bromo-7-methoxy-2-methylisoquinolin-1-one.

To a 100-mL 3-necked round-bottom flask purged and maintained with an inert atmosphere of nitrogen, was added tetrahydrofuran (30.00 mL, 370.290 mmol), and 6-bromo-7-methoxy-2H-isoquinolin-1-one (2.60 g, 10.233 mmol). The mixture was cooled in an ice/water bath followed by the addition of sodium hydride (0.61 g, 25.419 mmol). Methyl iodide (2.18 g, 15.350 mmol) was then added and the resulting solution was stirred for an additional 1 hour at 0°C. The reaction was then quenched by the addition of 50 mL of water/ice. The resulting mixture was extracted with ethyl acetate (3x50 mL), the organics were combined, dried over anhydrous Na₂SO₄, filtered, and concentrated to dryness under reduced pressure. The residue was purified by silica gel chromatography (dichloromethane/petroleum ether) which gave after isolation 2.2 g (80.19%) of 6-bromo-7-methoxy-2-methylisoquinolin-1-one as a white solid.

### Step 7: Preparation of 7-methoxy-2-methyl-1-oxoisoquinolin- 6-ylboronic acid.

To a 100-mL 3-necked round-bottom flask purged and maintained with an inert atmosphere of nitrogen, was added bis(pinacolato)diboron (2.73 g, 10.742 mmol), 6-bromo-7-methoxy-2-methylisoquinolin-1-one (2.40 g, 8.952 mmol), 1,1 - Bis(diphenylphosphino)ferrocene-palladium(II)dichloride dichloromethane complex (0.33 g, 0.448 mmol, 0.05), dioxane (30 mL), and potassium acetate (1.76 g, 17.903 mmol). The resulting mixture was placed in a preheated oil bath and was stirred for 2 hours at 100°C then removed from heat, allowed to cool. The mixture was concentrated, purified by preparative-HPLC which gave after isolation 950 mg (45.54%) of 7-methoxy-2-methyl-1-oxoisoquinolin-6-ylboronic acid as a off-white solid.

### Step 8: Preparation of tert-butyl (1S,2R,3R,5R)-2-fluoro-3-((3-(7-methoxy-2-methyl-1-oxo-1,2-dihydroisoquinolin-6-yl)-1,2,4-triazin-6-yl)(methyl)amino)-8-azabicyclo[3.2.1]octane-8-carboxylate and tert-butyl (1R,2S,3S,5S)-2-fluoro-3-((3-(7-methoxy-2-methyl-1-oxo-1,2-dihydroisoquinolin-6-yl)-1,2,4-triazin-6-yl)(methyl)amino)-8-azabicyclo[3.2.1]octane-8-carboxylate.

To an 8-mL vial purged and maintained with an inert atmosphere of nitrogen, was added Isomer 1 (120 mg, 0.313 mmol), 7-methoxy-2-methyl-1-oxoisoquinolin-6-ylboronic acid (145.84 mg, 0.626 mmol), copper(I)-3-methylsalicylate (147.79 mg, 0.689 mmol), tetrakis(triphenylphosphine)palladium(0) (18.08 mg, 0.016 mmol), tetrahydrofuran (2 mL). The resulting mixture was placed in a preheated oil bath at 70°C and was stirred for 2 hours then removed from heat, allowed to cool. The mixture was purified by preparative HPLC which gave after isolation 111 mg (67.62%) **of INT 8A** as a yellow solid (ES, m/z): [M+H]+=525.3.

Following the procedure above, but starting with Isomer 2 (120 mg, 0.313 mmol), which after isolation gave 112 mg (68.23%) of **INT 8B** as a yellow solid.

### Step 9: Preparation of 6-(6-(((1S,2S,3R,SR)-2-fluoro-8-azabicyclo[3.2.1]octan-3-yl)(methyl)amino)-1,2,4-triazin-3-yl)-7-hydroxy-2-methylisoquinolin-1(2H)-one and 6-(6-(((1R,2R,3S,5S)-2-fluoro-8-azabicyclo[3.2.1]octan-3-yl)(methyl)amino)-1,2,4-triazin-3-yl)-7-hydroxy-2-methylisoquinolin-1(2H)-one.

To an 8-mL vial purged and maintained with an inert atmosphere of nitrogen was added **INT 8A** (111.00 mg, 0.212 mmol), dichloromethane (1 mL), and boron tribromide (1.00 mL, 10.578 mmol). The resulting solution was stirred for 1 hour at room temperature. The reaction was then quenched by the addition of water/ice. The pH of the solution was adjusted to 8 with saturated aqueous sodium bicarbonate. The resulting mixture was extracted with dichloromethane (3x30 ml). The organics were combined, dried over anhydrous Na₂SO₄, filtered and concentrated to dryness under reduced pressure to a residue. The crude product was purified by preparative-HPLC which after isolation gave 51.3 mg of **Compound 4A** (ES, m/z): [M+H]+=411.2. ¹H NMR (300 MHz, Chloroform-d) δ 12.34 (s, 1H), 8.49 (s, 1H), 8.40 (d, J = 0.9 Hz, 1H), 8.06 (s, 1H), 6.98 - 6.90 (m, 1H), 6.53 (d, J = 7.3 Hz, 1H), 5.44 - 5.18 (m, 1H), 4.74 (d, J = 51.9 Hz, 1H), 3.77 (s, 2H), 3.62 (d, J = 0.9 Hz, 3H), 3.27 - 3.15 (m, 3H), 2.36 - 2.21 (m, 1H), 2.15 - 1.97 (m, 2H), 1.92 - 1.81 (m, 2H).

Following the procedure above, but starting with **INT 8B** (112.00 mg, 0.213 mmol), gave 29.3 mg of **Compound 4B** as a yellow solid (ES, m/z):[M+H]⁺=411.2. ¹H NMR (300 MHz, Chloroform-d) δ 12.34 (s, 1H), 8.50 (s, 1H), 8.40 (s, 1H), 8.06 (s, 1H), 6.94 (d, J = 7.3 Hz, 1H), 6.53 (d, J = 7.4 Hz, 1H), 5.32 (d, J = 37.0 Hz, 1H), 4.74 (d, J = 51.6 Hz, 1H), 3.77 (s, 2H), 3.62 (s, 3H), 3.22 (d, J = 1.6 Hz, 3H), 2.36 - 2.21 (m, 1H), 2.06 (d, J = 6.8 Hz, 2H), 1.91 - 1.80 (m, 2H).

### Example 5: Preparation of 7-(6-(((1S,2S,3R,5R)-2-fluoro-8-azabicyclo[3.2.1]octan-3-yl)(methyl)amino)-1,2,4-triazin-3-yl)isoquinolin-6-ol and 7-(6-(((1R,2R,3S,5S)-2-fluoro-8-azabicyclo[3.2.1]octan-3-yl)(methyl)amino)-1,2,4-triazin-3-yl)isoquinolin-6-ol (Compound 5A and 5B).

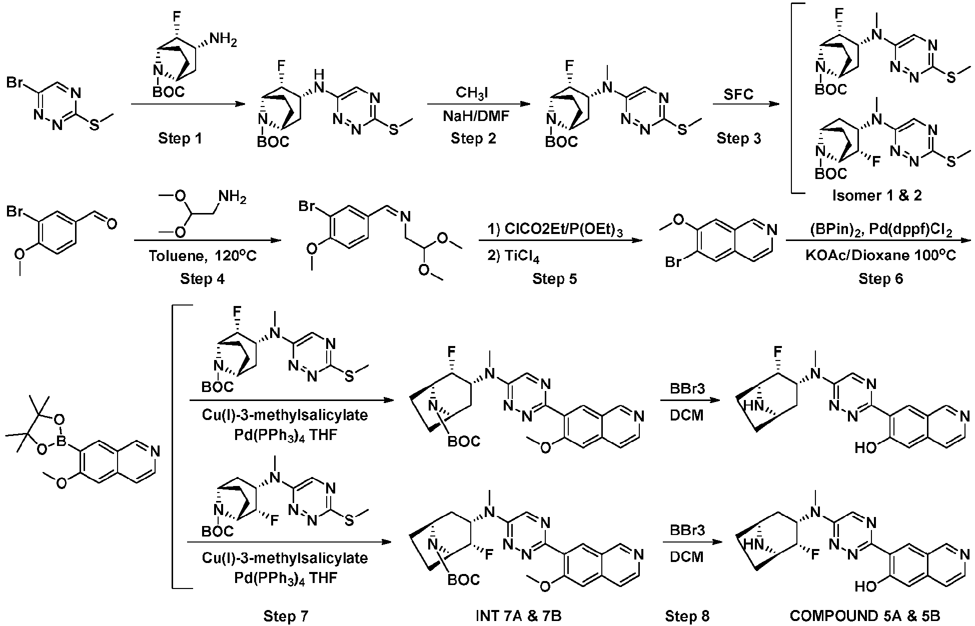

### Step 1: Preparation of racemic tert-butyl (1S,2R,3R,SR)-2-fluoro-3-((3-(methylthio)-1,2,4-triazin-6-yl)amino)-8-azabicyclo[3.2.1]octane-8-carboxylate, and tert-butyl (1R,2S,3S,5S)-2-fluoro-3-((3-(methylthio)-1,2,4-triazin-6-yl)amino)-8-azabicyclo[3.2.1]octane-8-carboxylate.

To a 1000 mL 3-necked round-bottom flask was added 6-bromo-3-(methylsulfanyl)-1,2,4-triazine (24.00 g, 116.5 mmol), a racemic mixture of tert-butyl (1S,2R,3R,5R)-3-amino-2-fluoro-8-azabicyclo[3.2.1]octane-8-carboxylate and tert-butyl (1R,2S,3S,5S)-3-amino-2-fluoro-8-azabicyclo[3.2.1]octane-8-carboxylate (43.0 g, 176 mmol), DIEA(60 mL, 344.5 mmol) and n-butanol (360 mL) under nitrogen. The resulting solution was placed in a preheated oil bath stirred at 120°C for 2 hours under nitrogen. The reaction mixture was removed from heat and cooled to room temperature and diluted with water (700 mL) and then extracted with ethyl acetate (3 x 250 mL). The organic layers were combined, washed with brine (250 mL), and dried over anhydrous Na₂SO₄. The mixture was filtered and then concentrated under reduced pressure. The residue was purified by silica gel column chromatography (CH₂Cl₂/ EtOAc (4:1) which gave a racemic mixture of tert-butyl (1S,2R,3R,5R)-2-fluoro-3-((3-(methylthio)-1,2,4-triazin-6-yl)amino)-8-azabicyclo[3.2.1]octane-8-carboxylate, and tert-butyl (1R,2S,3S,5S)-2-fluoro-3-((3-(methylthio)-1,2,4-triazin-6-yl)amino)-8-azabicyclo[3.2.1]octane-8-carboxylate as a yellow solid (24g, 55.8%) [M+H]+=370.

### Step 2: Preparation of racemic mixture of tert-butyl (1S,2R,3R,5R)-2-fluoro-3-(methyl(3-(methylthio)-1,2,4-triazin-6-yl)amino)-8-azabicyclo[3.2.1]octane-8-carboxylate, tert-butyl (1R,2S,3S,5S)-2-fluoro-3-(methyl(3-(methylthio)-1,2,4-triazin-6-yl)amino)-8-azabicyclo[3.2.1]octane-8-carboxylate.

To a 1000 mL 3-necked round-bottom flask was added tert-butyl (1S,2R,3R,5R)-2-fluoro-3-((3-(methylthio)-1,2,4-triazin-6-yl)amino)-8-azabicyclo[3.2.1]octane-8-carboxylate, and tert-butyl (1R,2S,3S,5S)-2-fluoro-3-((3-(methylthio)-1,2,4-triazin-6-yl)amino)-8-azabicyclo[3.2.1]octane-8-carboxylate (21 g, 56.84 mmol) and dimethylformamide (420 mL) under nitrogen. The resultant mixture was cooled to 0°C followed by portion wise addition of sodium hydride (4.54 g, 113.51 mmol, 60%) with the internal temperature maintained at 0°C under nitrogen. The cooling bath was removed, and the mixture was stirred for additional 0.5 h and allowed to come to room temperature under nitrogen. Methyl iodide (7.20 mL, 115.66 mmol) was added dropwise at room temperature with stirring. The resulting mixture was stirred for additional 1 h at room temperature. The reaction mixture was then cooled to 0°C and quenched with water (1 L). The resulting mixture was extracted with ethyl acetate (3 x 250 mL). The combined organic layers were washed with water (2x250 mL) and saturated brine (250 mL), and then dried over anhydrous Na₂SO₄. After filtration, the filtrate was concentrated under reduced pressure and purified by silica gel column chromatography (CH₂Cl₂/EtOAc (19:1)) which gave 17 g (78%) of tert-butyl (1S,2R,3R,5R)-2-fluoro-3-(methyl(3-(methylthio)-1,2,4-triazin-6-yl)amino)-8-azabicyclo[3.2.1]octane-8-carboxylate and tert-butyl (1R,2S,3S,5S)-2-fluoro-3-(methyl(3-(methylthio)-1,2,4-triazin-6-yl)amino)-8-azabicyclo[3.2.1]octane-8-carboxylate as a racemic yellow solid, [M+H]⁺=384.

### Step 3: Chiral Separation of tert-butyl (1S,2R,3R,5R)-2-fluoro-3-(methyl(3-(methylthio)-1,2,4-triazin-6-yl)amino)-8-azabicyclo[3.2.1]octane-8-carboxylate and tert-butyl (1R,2S,3S,5S)-2-fluoro-3-(methyl(3-(methylthio)-1,2,4-triazin-6-yl)amino)-8-azabicyclo[3.2.1]octane-8-carboxylate.

The racemic mixture of tert-butyl (1S,2R,3R,5R)-2-fluoro-3-(methyl(3-(methylthio)-1,2,4-triazin-6-yl)amino)-8-azabicyclo[3.2.1]octane-8-carboxylate and tert-butyl (1R,2S,3S,5S)-2-fluoro-3-(methyl(3-(methylthio)-1,2,4-triazin-6-yl)amino)-8-azabicyclo[3.2.1]octane-8-carboxylate (11.00 g, 28.684 mmol) was purified into individual isomers by Prep-SFC. The following conditions were used (Column: CHIRALPAK IG, 2*25cm,5um; Mobile Phase A: CO₂, Mobile Phase B: EtOH--HPLC; Flow rate: 40 mL/min; Gradient: 50% B; 250 nm; RT1: 3.99 min; RT2: 6.19 min; Injection volume: 4 ml; Number Of Runs: 80;) which gave isomer 1 (5.0 g) as a yellow solid and isomer 2 (5.2g) as a yellow solid.

### Step 4: Preparation of 1-(3-bromo-4-methoxyphenyl)-N-(2,2-dimethoxyethyl)methanimine.

To a 500 mL 3-necked round-bottom flask was added 3-bromo-4-methoxybenzaldehyde (25 g, 116.000 mmol), toluene (250 mL) and 2,2-dimethoxyethan-1-amine (19 mL, 174.1 mmol, 1.50). The reaction flask was fitted with a Dean-Stark trap and the mixture was placed in a preheated oil bath at 120°C and was stirred for 6 hours, removed from heat, and allowed to cool. The resulting mixture was then concentrated under vacuum to a constant weight which gave after isolation 37g of 1-(3-bromo-4-methoxyphenyl)-N-(2,2-dimethoxyethyl)methanimine that was used directly in the next step m/z: 302(M+H⁺).

### Step 5: Preparation of 7-bromo-6-methoxyisoquinoline.

To a 1000mL 1-necked round-bottom flask was added 1-(3-bromo-4-methoxyphenyl)-N-(2,2-dimethoxyethyl)methanimine (37 g, 122.45 mmol), tetrahydrofuran (370 mL). The mixture was then cooled to 0°C and isopropyl chloroformate (15.01 g, 122.45 mmol) was added dropwise. After stirring for 5 min, triethyl phosphite (24.42 g, 146.970 mmol) was added and mixture was stirred for 20 min, then the cooling bath was removed. The resulting mixture was then stirred for 18 hours at room temperature. The solvent was then removed, and the remaining residue was azeotroped with 100ml of toluene. To the resultant mixture, titanium tetrachloride (94.16 g) and chloroform (375 mL) were added and the mixture was heated to reflux for 48 hr. The mixture was removed from heat and then poured on ice/water. The pH was then adjusted to 9 with ammonium hydroxide. The reaction mixture was extracted with ethyl acetate (4X200mL). The organics were combined and concentrated to dryness under reduced pressure which was purified by silica gel column(ethyl acetate: petroleum ether 3:7) which gave after isolation 6.1 g of 7-bromo-6-methoxyisoquinoline as a solid. m/z: 238(M+H⁺).

### Step 6: Preparation of 6-methoxy-7-(4,4,5,5-tetramethyl-1,3,2-dioxaborolan-2-yl)isoquinoline.

To a 40 mL vial purged and maintained with an inert atmosphere of nitrogen was added 7-bromo-6-methoxyisoquinoline (1.50 g, 6.3 mmol), bis(pinacolato)diboron (1.92 g, 7.561 mmol), 1,1 -Bis(diphenylphosphino)ferrocene-palladium(II)dichloride dichloromethane complex (0.51 g, 0.625 mmol), potassium acetate (1.24 g, 12.635 mmol) and dioxane (30 mL, 354.123 mmol). The resulting mixture was placed in a preheated oil bath and was stirred for 4 hours at 100°C then removed from heat, allowed to cool. The resulting mixture was diluted with ethyl acetate (200 mL), filtered through a celite pad and concentrated under reduced pressure. The residue was purified by silica gel column chromatography (CH₂Cl₂/MeOH 9:1) which gave a reddish brown solid which was further purified by Preparative-HPLC (C18; mobile phase: NH₄HCO₃ (aq) / MeCN; Gradient: 10% B to 50% B in 30 min; Detector: 254 nm) which gave after isolation 1.4 g of 6-methoxy-7-(4,4,5,5-tetramethyl- 1,3,2-dioxaborolan-2-yl)isoquinoline as a solid m/z: 286(M+H⁺).

### Step 7: Preparation of tert-butyl (1S,2R,3R,5R)-2-fluoro-3-((3-(6-methoxyisoquinolin-7-yl)-1,2,4-triazin-6-yl)(methyl)amino)-8-azabicyclo[3.2.1]octane-8-carboxylate and tert-butyl (1R,2S,3S,5S)-2-fluoro-3-((3-(6-methoxyisoquinolin-7-yl)-1,2,4-triazin-6-yl)(methyl)amino)-8-azabicyclo[3.2.1]octane-8-carboxylate.

To a 40 mL vial purged and maintained with an inert atmosphere of nitrogen was added Isomer 1 (300 mg, 0.782 mmol), 6-methoxy-7-(4,4,5,5-tetramethyl-1,3,2-dioxaborolan-2-yl)isoquinoline (670 mg, 2.35 mmol), tetrakis(triphenylphosphine) palladium(0) (135 mg, 0.117 mmol, 0.15), copper(I)-3-methylsalicylate (504 mg, 2.348 mmol) and tetrahydrofuran (7.50 mL). The resulting mixture was placed in a preheated oil bath at 70°C and was stirred for 12 hours then removed from heat, allowed to cool. The resulting mixture was diluted with CH₂Cl₂ (100mL). The resulting mixture was washed with 3x25 mL of ammonium hydroxide (10%) and 50 mL of brine. The combined organic layers were dried over anhydrous Na₂SO₄, filtered, and concentrated under reduced pressure. The residue was purified by silica gel column chromatography (ethyl acetate: 100%) which gave after isolation, 278 mg of **INT 7A** as a light yellow solid. m/z:495(M+H⁺).

Following the procedure above, but starting with Isomer 2 (350 mg, 0.913 mmol) which after isolation gave 350 mg of **INT 7B** as a yellow solid, m/z:495(M+H⁺).

### Step 8: Preparation of 7-(6-(((1S,2S,3R,SR)-2-fluoro-8-azabicyclo[3.2.1]octan-3-yl)(methyl)amino)-1,2,4-triazin-3-yl)isoquinolin-6-ol and 7-(6-(((1R,2R,3S,5S)-2-fluoro-8-azabicyclo[3.2.1]octan-3-yl)(methyl)amino)-1,2,4-triazin-3-yl)isoquinolin-6-ol (Compound 5A and 5B).

To a 100-mL round-bottom flask, was placed **INT 7A** (150 mg, 0.303 mmol), dichloromethane (5 mL, 78.65 mmol), and boron tribromide (759.82 mg, 3.033 mmol). The resulting solution was stirred for 4 hours at room temperature followed by quenching with aqueous sodium bicarbonate to a final pH of 9. The mixture was extracted with dichloromethane (3x50 mL), the organic layers were combined, dried over anhydrous sodium sulfate, filtered, and concentrated under vacuum. The crude product was purified by Flash-Prep-HPLC, (IntelFlash-1): Column: C18; mobile phase: NH₄HCO₃ (aq) / MeCN; Gradient: 10% B to 50% B in 30 min; Detector254 nm, which gave after isolation 30 mg of **Compound 5A** as a yellow solid (ES, m/z): [M+1]⁺ = 381.2. ¹H NMR (300 MHz, DMSO-d6) δ ppm 12.96 (s, 1H), 9.31 (d, J = 1.1 Hz, 1H), 8.99 (s, 1H), 8.87 (s, 1H), 8.39 (d, J = 5.8 Hz, 1H), 7.66 (d, J = 5.9 Hz, 1H), 7.38 (s, 1H), 4.94 (d, J = 24.8 Hz, 1H), 4.70 (d, J = 52.0 Hz, 1H), 3.55 (s, 2H), 3.18 (d, J = 1.7 Hz, 3H), 2.35 - 2.19 (m, 1H), 1.87 - 1.52 (m, 5H).

Following the procedure above, but starting with **INT 7B** (150 mg, 0.303 mmol), gave 25 mg of **Compound 5B** as a yellow solid (ES, m/z): [M+1]⁺ = 381.2. ¹H NMR (300 MHz, DMSO-d6) δ ppm 12.96 (s, 1H), 9.31 (d, J = 1.1 Hz, 1H), 8.99 (s, 1H), 8.87 (s, 1H), 8.39 (d, J = 5.8 Hz, 1H), 7.66 (d, J = 5.9 Hz, 1H), 7.38 (s, 1H), 4.94 (d, J = 24.8 Hz, 1H), 4.70 (d, J = 52.0 Hz, 1H), 3.55 (s, 2H), 3.18 (d, J = 1.7 Hz, 3H), 2.35 - 2.19 (m, 1H), 1.87 - 1.52 (m, 5H).

### Example 6: Preparation of 7-(6-(((1S,2S,3R,SR)-2-fluoro-8-azabicyclo[3.2.1]octan-3-yl)(methyl)amino)-1,2,4-triazin-3-yl)-6-hydroxy-2-methyl-4H-chromen-4-one and 7-(6-(((1R,2R,3S,5S)-2-fluoro-8-azabicyclo[3.2.1]octan-3-yl)(methyl)amino)-1,2,4-triazin-3-yl)-6-hydroxy-2-methyl-4H-chromen-4-one (Compound 6A and 6B).

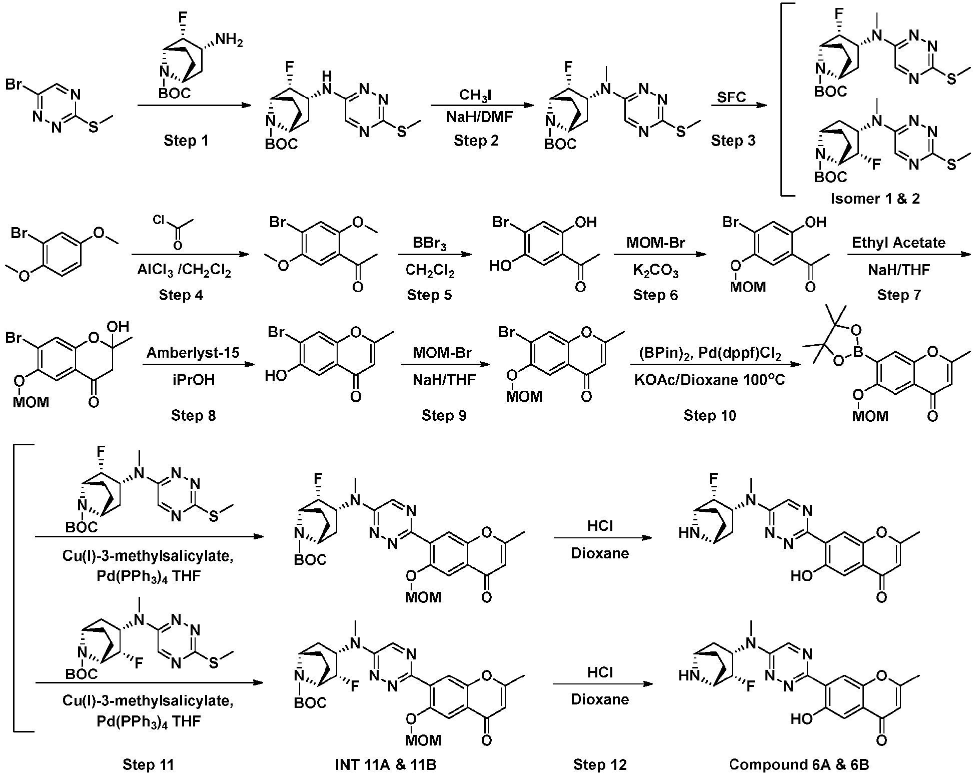

### Step 1: Preparation of racemic tert-butyl (1S,2R,3R,SR)-2-fluoro-3-((3-(methylthio)-1,2,4-triazin-6-yl)amino)-8-azabicyclo[3.2.1]octane-8-carboxylate, and tert-butyl (1R,2S,3S,5S)-2-fluoro-3-((3-(methylthio)-1,2,4-triazin-6-yl)amino)-8-azabicyclo[3.2.1]octane-8-carboxylate.

To a 1000 mL 3-necked round-bottom flask was added 6-bromo-3-(methylsulfanyl)-1,2,4-triazine (24.00 g, 116.5 mmol), a racemic mixture of tert-butyl (1S,2R,3R,5R)-3-amino-2-fluoro-8-azabicyclo[3.2.1]octane-8-carboxylate and tert-butyl (1R,2S,3S,5S)-3-amino-2-fluoro-8-azabicyclo[3.2.1]octane-8-carboxylate (43.0 g, 176 mmol), DIEA(60 mL, 344.5 mmol) and n-butanol (360 mL) under nitrogen. The resulting solution was placed in a preheated oil bath stirred at 120°C for 2 hours under nitrogen. The reaction mixture was removed from heat and cooled to room temperature and diluted with water (700 mL) and then extracted with ethyl acetate (3 x 250 mL). The organic layers were combined, washed with brine (250 mL), and dried over anhydrous Na₂SO₄. The mixture was filtered and then concentrated under reduced pressure. The residue was purified by silica gel column chromatography (CH₂Cl₂/ EtOAc (4:1) which gave a racemic mixture of tert-butyl (1S,2R,3R,5R)-2-fluoro-3-((3-(methylthio)-1,2,4-triazin-6-yl)amino)-8-azabicyclo[3.2.1]octane-8-carboxylate, and tert-butyl (1R,2S,3S,5S)-2-fluoro-3-((3-(methylthio)-1,2,4-triazin-6-yl)amino)-8-azabicyclo[3.2.1]octane-8-carboxylate as a yellow solid (24g, 55.8%) [M+H]+=370.

### Step 2: Preparation of racemic mixture of tert-butyl (1S,2R,3R,5R)-2-fluoro-3-(methyl(3-(methylthio)-1,2,4-triazin-6-yl)amino)-8-azabicyclo[3.2.1]octane-8-carboxylate, tert-butyl (1R,2S,3S,5S)-2-fluoro-3-(methyl(3-(methylthio)-1,2,4-triazin-6-yl)amino)-8-azabicyclo[3.2.1]octane-8-carboxylate.

To a 1000 mL 3-necked round-bottom flask was added tert-butyl (1S,2R,3R,5R)-2-fluoro-3-((3-(methylthio)-1,2,4-triazin-6-yl)amino)-8-azabicyclo[3.2.1]octane-8-carboxylate, and tert-butyl (1R,2S,3S,5S)-2-fluoro-3-((3-(methylthio)-1,2,4-triazin-6-yl)amino)-8-azabicyclo[3.2.1]octane-8-carboxylate (21 g, 56.84 mmol) and dimethylformamide (420 mL) under nitrogen. The resultant mixture was cooled to 0°C followed by portion wise addition of sodium hydride (4.54 g, 113.51 mmol, 60%) with the internal temperature maintained at 0°C under nitrogen. The cooling bath was removed, and the mixture was stirred for additional 0.5 h and allowed to come to room temperature under nitrogen. Methyl iodide (7.20 mL, 115.66 mmol) was added dropwise at room temperature with stirring. The resulting mixture was stirred for additional 1 h at room temperature. The reaction mixture was then cooled to 0°C and quenched with water (1 L). The resulting mixture was extracted with ethyl acetate (3 x 250 mL). The combined organic layers were washed with water (2x250 mL) and saturated brine (250 mL), and then dried over anhydrous Na₂SO₄. After filtration, the filtrate was concentrated under reduced pressure and purified by silica gel column chromatography (CH₂Cl₂/EtOAc (19:1)) which gave 17 g (78%) of tert-butyl (1S,2R,3R,5R)-2-fluoro-3-(methyl(3-(methylthio)-1,2,4-triazin-6-yl)amino)-8-azabicyclo[3.2.1]octane-8-carboxylate and tert-butyl (1R,2S,3S,5S)-2-fluoro-3-(methyl(3-(methylthio)-1,2,4-triazin-6-yl)amino)-8-azabicyclo[3.2.1]octane-8-carboxylate as a racemic yellow solid, [M+H]⁺=384.

### Step 3: Chiral Separation of tert-butyl (1S,2R,3R,5R)-2-fluoro-3-(methyl(3-(methylthio)-1,2,4-triazin-6-yl)amino)-8-azabicyclo[3.2.1]octane-8-carboxylate and tert-butyl (1R,2S,3S,5S)-2-fluoro-3-(methyl(3-(methylthio)-1,2,4-triazin-6-yl)amino)-8-azabicyclo[3.2.1]octane-8-carboxylate.

The racemic mixture of tert-butyl (1S,2R,3R,5R)-2-fluoro-3-(methyl(3-(methylthio)-1,2,4-triazin-6-yl)amino)-8-azabicyclo[3.2.1]octane-8-carboxylate and tert-butyl (1R,2S,3S,5S)-2-fluoro-3-(methyl(3-(methylthio)-1,2,4-triazin-6-yl)amino)-8-azabicyclo[3.2.1]octane-8-carboxylate (11.00 g, 28.684 mmol) was purified into individual isomers by Prep-SFC. The following conditions were used (Column: CHIRALPAK IG, 2*25cm,5um; Mobile Phase A: CO₂, Mobile Phase B: EtOH--HPLC; Flow rate: 40 mL/min; Gradient: 50% B; 250 nm; RT1: 3.99 min; RT2: 6.19 min; Injection volume: 4 ml; Number Of Runs: 80;) which gave isomer 1 (5.0 g) as a yellow solid and isomer 2 (5.2g) as a yellow solid.

### Step 4: Preparation of 1-(4-bromo-2,5-dimethoxyphenyl)ethan-1-one.

To a 1000 mL 3-necked round-bottom flask purged and maintained with an inert atmosphere of nitrogen was added 2-bromo-1,4-dimethoxybenzene (60 g, 276.419 mmol), dichloromethane (500 mL, 7865.023 mmol), and anhydrous aluminum chloride (55.29 g, 414.628 mmol). The reaction mixture was cooled to 0°C followed by the dropwise addition of acetyl chloride (29.59 mL, 414.637 mmol) over a period of 10 minutes. The cooling bath was then removed, and the resulting solution was stirred for 2 hours at room temperature. The reaction mixture was then carefully quenched by the slow addition of 200 mL of water/ice. The resulting mixture was extracted with dichloromethane (3x300 mL), dried over anhydrous sodium sulfate, filtered and concentrated under vacuum which gave after isolation 45 g of crude 1-(4-bromo-2,5-dimethoxyphenyl)ethan-1-one as a yellow solid (m/z:259 (M+H⁺)) that was used directly in the next step.

### Step 5: Preparation of 1-(4-bromo-2,5-dihydroxyphenyl)ethan-1-one.

To a 1000 mL 3-necked round-bottom flask purged and maintained with an inert atmosphere of nitrogen, was placed 1-(4-bromo-2,5-dimethoxyphenyl)ethanone (45 g, 173.679 mmol), and dichloromethane (300 mL) which was then cooled to 0°C followed by the addition of boron tribromide (1M in dichloromethane, 694 mL, 694 mmol) dropwise with stirring while maintain the internal temperature at 0°C. The cooling bath was then removed and the resulting solution was stirred overnight at room temperature. The mixture was then cooled to 0°C and then carefully quenched by the slow addition of 200 mL of water/ice. The resulting mixture was extracted with dichloromethane (3x300 mL) dried over anhydrous sodium sulfate, filtered and concentrated under vacuum which gave 16 g of 1-(4-bromo-2,5-dihydroxyphenyl)ethan-1-one as a yellow solid (m/z:231(M+H⁺)) that was used directly in the next step.

### Step 6: Preparation of 1-(4-bromo-2-hydroxy-5-(methoxymethoxy)phenyl)ethan-1-one.

To a 500-mL 3-necked round-bottom flask purged and maintained with an inert atmosphere of nitrogen, was added 1-(4-bromo-2,5-dihydroxyphenyl)ethan-1-one (16 g, 69.251 mmol), potassium carbonate (19.14 g, 138.489 mmol), and acetone (150 mL). The mixture was then cooled to 0°C followed by the dropwise addition of bromo(methoxy)methane (8.65 g, 69.251 mmol). The cooling bath was removed, and the resulting mixture was stirred for 2 hours at room temperature. The mixture was filtered and concentrated under vacuum which after isolation gave 15 g of 1-(4-bromo-2-hydroxy-5-(methoxymethoxy)phenyl)ethan-1-one as a yellow solid, m/z:275(M+H⁺) that was used directly in the next step.

### Step 7: Preparation of 7-bromo-2-hydroxy-6-(methoxymethoxy)-2-methylchroman-4-one.

To a 500-mL 3-necked round-bottom flask purged and maintained with an inert atmosphere of nitrogen, was added 1-(4-bromo-2-hydroxy-5-(methoxymethoxy)phenyl)ethan-1-one (15 g, 54.526 mmol), tetrahydrofuran (200 mL, 2468.598 mmol), and sodium hydride (5.23 g, 218.1 mmol, 4.00). The mixture was cooled to 0°C followed by the dropwise addition of dry ethyl acetate (9.61 g, 109.074 mmol). The cooling bath was removed, and the mixture was stirred for 2 hours at room temperature. The mixture was then carefully quenched with 50 mL of water/ice. The mixture was then extracted with ethyl acetate (2x200 mL), the organics were combined, dried over anhydrous sodium sulfate, filtered and concentrated under vacuum which gave 12 g of 7-bromo-2-hydroxy-6-(methoxymethoxy)-2-methylchroman-4-one as a yellow solid, m/z:317(M+H+), that was used directly in the next step.

### Step 8: Preparation of 7-bromo-6-hydroxy-2-methyl-4H-chromen-4-one.

To a 500-mL 3-necked round-bottom flask purged and maintained with an inert atmosphere of nitrogen, was added 7-bromo-2-hydroxy-6-(methoxymethoxy)-2-methylchroman-4-one (12 g), propan-2-ol (100 mL). The resulting mixture was placed in a preheated oil bath and stirred at 65°C followed by the dropwise addition of Amberlyst-15 (15 g). The resulting mixture was stirred for an additional 2 hours at 65°C then removed from heat and allowed to cool. The mixture was filtered and resulting filtrate was concentrated under vacuum to dryness which gave after isolation 9 g of 7-bromo-6-hydroxy-2-methyl-4H-chromen-4-one as a yellow solid, m/z: 255(M+H⁺), that was used directly in the next step.

### Step 9: Preparation of 7-bromo-6-(methoxymethoxy)-2-methyl-4H-chromen-4-one.

To a 500-mL 3-necked round-bottom flask purged and maintained with an inert atmosphere of nitrogen, was added 7-bromo-6-hydroxy-2-methyl-4H-chromen-4-one (9.00 g, 35.285 mmol), tetrahydrofuran (100 mL). The mixture was cooled to 0°C followed by the portion-wise addition of sodium hydride (1.69 g, 70.423 mmol) at 0°C. To this mixture, methoxymethyl bromide (8.82 g, 70.57 mmol) was added dropwise after which the cooling bath was removed, and the resulting mixture was stirred for 2 hours at room temperature. The reaction was then quenched by the addition of 50 mL of water/ice. The resulting mixture was extracted with ethyl acetate (3x200 mL), dried over anhydrous sodium sulfate, filtered and concentrated under vacuum which gave 7 g of 7-bromo-6-(methoxymethoxy)-2-methyl-4H-chromen-4-one as a yellow solid, m/z: 299(M+H⁺), that was used directly in the next step.

### Step 10: Preparation of 6-(methoxymethoxy)-2-methyl-7-(4,4,5,5-tetramethyl-1,3,2-dioxaborolan-2-yl)-4H-chromen-4-one.

To a 250-mL 3-necked round-bottom flask purged and maintained with an inert atmosphere of nitrogen, was added 7-bromo-6-(methoxymethoxy)-2-methyl-4H-chromen-4-one (5.00 g, 16.716 mmol), bis(pinacolato)diboron (8.49 g, 33.433 mmol), 1,1 - Bis(diphenylphosphino)ferrocene-palladium(II)dichloride dichloromethane complex (1.22 g, 1.667 mmol), Dioxane (100 mL) and potassium acetate (3.28 g, 33.421 mmol). The resulting mixture was placed in a preheated oil bath and was stirred for 2 hours at 100°C then removed from heat, allowed to cool. The resulting solution was diluted with ethyl acetate (500 mL) and washed water (3 x50). The organic layer was dried over anhydrous sodium sulfate, filtered and then concentrated to dryness under vacuum. The residue was chromatographed on silica gel [(ethyl acetate/hexane: 1:1), Flash-prep-HPLC (IntelFlash-1): Colum: C18; mobile phase: ACN(50%), H₂O ; Detector: 254nm] which gave after isolation 2 g of 6-(methoxymethoxy)-2-methyl-7-(4,4,5,5-tetramethyl-1,3,2-dioxaborolan-2-yl)-4H-chromen-4-one as a yellow solid. m/z: 265(M+H⁺).

### Step 11: Preparation of tert-butyl (1S,2R,3R,5R)-2-fluoro-3-((3-(6-methoxy-2-methyl-4-oxo-4H-chromen-7-yl)-1,2,4-triazin-6-yl)(methyl)amino)-8-azabicyclo[3.2.1]octane-8-carboxylate and tert-butyl (1R,2S,3S,5S)-2-fluoro-3-((3-(6-methoxy-2-methyl-4-oxo-4H-chromen-7-yl)-1,2,4-triazin-6-yl)(methyl)amino)-8-azabicyclo[3.2.1]octane-8-carboxylate.

To a 40-mL round-bottom flask purged and maintained with an inert atmosphere of nitrogen, was placed Isomer 1 (150 mg, 0.391 mmol), copper(I)-3-methylsalicylate (250.74 mg, 1.173 mmol), 6-(methoxymethoxy)-2-methyl-7-(4,4,5,5-tetramethyl-1,3,2-dioxaborolan-2-yl)-4H-chromen-4-one (309.83 mg, 1.173 mmol), tetrakis(triphenylphosphine)palladium(0) (45.20 mg, 0.039 mmol) and tetrahydrofuran (10 mL). The resulting mixture was placed in a preheated oil bath at 70°C and was stirred for 2 hours then removed from heat, allowed to cool. The reaction mixture was diluted with ethyl acetate (100 mL), washed with water (3 x30). The mixture was dried over anhydrous sodium sulfate, filtered, concentrated under vacuum, and purified by preparative HPLC which gave after isolation gave 60 mg **INT 11A** as a yellow solid, m/z: 556(M+H⁺).

Following the procedure above, but starting with Isomer 2 (150 mg, 0.391 mmol) which after isolation gave 61 mg of **INT 11B** as a yellow solid, m/z:556(M+H+).

### Step 12: Preparation of 7-(6-(((1S,2S,3R,5R)-2-fluoro-8-azabicyclo[3.2.1]octan-3-yl)(methyl)amino)-1,2,4-triazin-3-yl)-6-hydroxy-2-methyl-4H-chromen-4-one and 7-(6-(((1R,2R,3S,5S)-2-fluoro-8-azabicyclo[3.2.1]octan-3-yl)(methyl)amino)-1,2,4-triazin-3-yl)-6-hydroxy-2-methyl-4H-chromen-4-one (Compound 6A and 6B).

To a 100-mL 3-necked round-bottom flask, purged and maintained with dry nitrogen was added **INT 11A** (100 mg, 0.180 mmol), dichloromethane (5 mL), and HCl (4M in 1,4-dioxane, 5 mL). The mixture was stirred for 2 hours at room temperature. The pH of the mixture was then adjusted to 9 with aqueous sodium bicarbonate (1 M). The resulting mixture was extracted with dichloromethane (3x30 mL), dried over anhydrous sodium sulfate, filtered, and concentrated under vacuum. The crude product was purified by Flash-Prep-HPLC (IntelFlash-1): Column: Column C18; mobile phase: NH₄HCO₃ (aq) / MeCN; Gradient: 10% B to 50% B in 30 min; Detector: 254 nm which gave after isolation 10.6 mg of **Compound 6A** as a yellow solid (ES, m/z): [M+1]⁺ = 412.2 ¹H NMR (300 MHz, DMSO-d6) δ ppm 12.46 (s, 1H), 8.86 (s, 1H), 8.28 (s, 1H), 7.43 (s, 1H), 6.23 (s, 1H), 5.03 - 4.80 (m, 1H), 4.68 (d, J = 52.1 Hz, 1H), 3.54 (s, 2H), 3.17 (d, J = 1.7 Hz, 3H), 2.41 (d, J = 0.7 Hz, 3H), 2.28 (d, J = 25.2 Hz, 1H), 1.87 - 1.50 (m, 5H).

Following the procedure above, but starting with **INT 11B** (100 mg, 0.180 mmol) gave 29.3 mg of **Compound 6B** as a yellow solid (ES, m/z):[M+H]⁺=412.2. ¹H NMR (300 MHz, DMSO-d6) δ ppm 12.46 (s, 1H), 8.86 (s, 1H), 8.28 (s, 1H), 7.43 (s, 1H), 6.23 (s, 1H), 5.03 -4.80 (m, 1H), 4.68 (d, J = 52.1 Hz, 1H), 3.54 (s, 2H), 3.17 (d, J = 1.7 Hz, 3H), 2.41 (d, J = 0.7 Hz, 3H), 2.28 (d, J = 25.2 Hz, 1H), 1.87 - 1.50 (m, 5H).

### Example 7. Preparation of 4-Fluoro-6-(6-{[(2R,3S,5S)-2-fluoro-8-azabicyclo[3.2.1]octan-3-yl](methyl)amino}-1,2,4-triazin-3-yl)-7-hydroxy-2-methylisoquinolin-1-one (Compound 66).

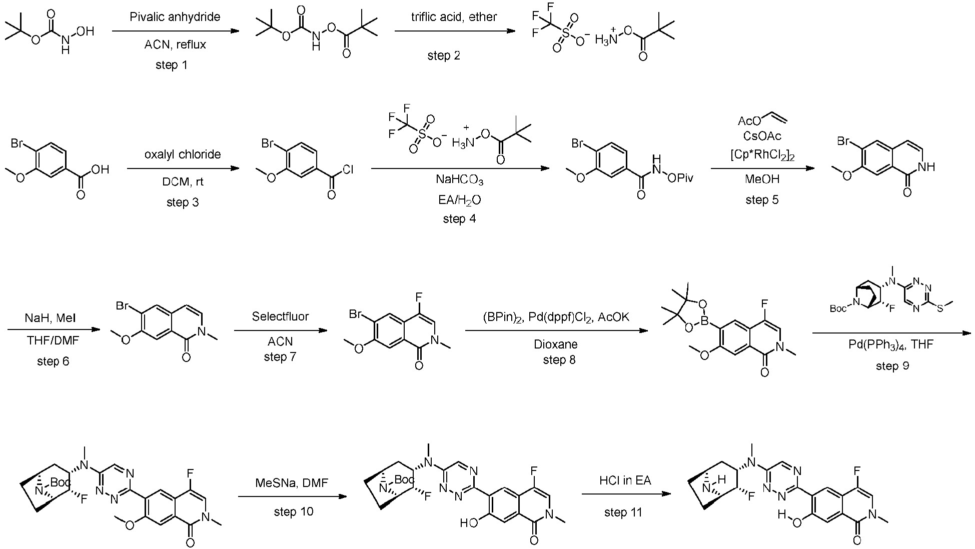

### Step 1: Preparation of (tert-butoxycarbonyl)amino 2,2-dimethylpropanoate.

Into a 10-L 4-necked round-bottom flask were added tert-butyl N-hydroxycarbamate (250.00 g, 1877.6 mmol) and ACN (5 L). Then pivalic anhydride (384.68 g, 2065.4 mmol) was added dropwise and the resulting mixture heated to reflux overnight. The reaction was allowed to cool to ambient temperature, then concentrated under vacuum. The residue was partitioned between EtOAc (7 L) and saturated aqueous sodium bicarbonate solution (5 L). The organic layers were separated and washed with saturated aqueous sodium bicarbonate solution (3 x 2 L), dried over sodium sulfate, filtered and concentrated under reduced pressure to afford 350 g (85.8%) (tert-butoxycarbonyl)amino 2,2-dimethylpropanoate as an off-white solid.

### Step 2: Preparation of [(2,2-dimethylpropanoyl)oxy]azanium triflate.

In a 10 L 4-necked round-bottom flask, to (tert-butoxycarbonyl)amino 2,2-dimethylpropanoate (350.00 g, 1610.9 mmol) in diethyl ether (7.0L) at 0°C was added 2,2,2-trifluoroethanesulfonic acid (241.75 g, 1610.9 mmol) (vigorous evolution of gas noted). The reaction mixture was stirred at 0°C for 5 min and then allowed to warm to rt. After 1 h hexane (3 L) was added and the mixture stirred for 10 min. The resulting solid was filtered, washed with hexanes (3 x 1 L) and dried in a vacuum oven to afford 300 g (69.69%) [(2,2-dimethylpropanoyl)oxy] azanium triflate as an off-white solid.

### Step 3: preparation of 4-bromo-3-methoxybenzoyl chloride.

In a 5 L 4-necked round-bottom flask, to a stirred suspension of 4-bromo-3-methoxybenzoic acid (300 g, 1298.4 mmol) in DCM (3 L) and DMF (30.15 mL, 389.5 mmol) was added oxalyl chloride (197.77 g, 1558.1 mmol) dropwise at ambient temperature under nitrogen atmosphere. The resulting solution was stirred for 3h at RT. The resulting mixture was concentrated under reduced pressure. This resulted in 4-bromo-3-methoxybenzoyl chloride (380 g, crude) as an off-white solid, which was used directly in next step. [M+H]⁺=248.9.

### Step 4: Preparation of bromo-3-methoxyphenyl)formamido 2,2-dimethylpropanoate.

In a 3 L 4-necked round-bottom flask, to [(2,2-dimethylpropanoyl)-oxy] azanium triflate (152.00 g, 568.8 mmol) in EA (500 mL) was added a solution of sodium bicarbonate (88.00 g, 1047.5 mmol) in water (500 mL) dropwise at 0°C. After stirring for 30 min, a solution of 4-bromo-3-methoxybenzoyl chloride (120.00 g, 481 mmol) in EA (1.20 L) was added dropwise at 0°C. The resulting mixture was then stirred for 2h at RT. The organic phase was separated, and the aqueous phase extracted with ethyl acetate (2 x 500 mL). The combined organic layers were washed with saturated aqueous sodium bicarbonate solution (2 x 500 mL), dried over anhydrous sodium sulfate and concentrated. The residue was purified by silica gel column chromatography with PE/EA (5/1) as the eluent to afford 126 g (79.6%) (4-bromo-3-methoxyphenyl)formamido 2,2-dimethylpropanoate as a white solid. [M+H]⁺=331.02.

### Step 5: Preparation of 6-bromo-7-methoxy-2H-isoquinolin-1-one.

Into a 3 L 4-necked round-bottom flask were added (4-bromo-3-methoxyphenyl)formamido 2,2-dimethylpropanoate (250.00 g, 757.2 mmol), vinyl acetate (99.73 g, 1158.5 mmol), cesium acetate (45.05 g, 234.7 mmol), bis[(pentamethylcyclopentadienyl)dichloro-rhodium] (4.68 g, 7.6mmol) and MeOH (2.5 L). The resulting mixture was stirred for 16 h at 40°C under nitrogen atmosphere. The mixture was allowed to cool down to RT. The precipitated solids were collected by filtration and washed with cold diethyl ether (3x1000 mL). This resulted in 6-bromo-7-methoxy-2H-isoquinolin-1-one (120 g, 62.38%) as an off-white solid. [M+H]⁺=254.97.

### Step 6: Preparation of 6-bromo-7-methoxy-2-methylisoquinolin-1-one.

In a 1-L 3-necked round-bottom flask, to a stirred suspension of 6-bromo-7-methoxy-2H-isoquinolin-1-one (120 g, 472.3 mmol) in THF (1.2 L) and DMF (0.5 L) was added sodium hydride as a 60% suspension in mineral oil (28.71 g, 717.9 mmol) in portions at 0°C under nitrogen atmosphere The resulting solution was stirred at 0°C for 30 min. Methyl iodide (81.76 g, 524.2 mmol) was added dropwise at 0°C under nitrogen atmosphere. The resulting solution was warmed to RT. After 1h, the reaction mixture was poured into cold water (2L) and stirred for 15 min. The precipitated solids were collected by filtration and washed with cold water (3x1 L). The residue was purified by silica gel column chromatography eluted with PE / EA (3:1) to afford 6-bromo-7-methoxy-2-methylisoquinolin-1-one (102 g,85.55%) as an off-white solid. [M+H]⁺=268.

### Step 7: Preparation of 6-bromo-4-fluoro-7-methoxy-2-methylisoquinolin-1-one.

In a 3-necked round-bottom flask, to 6-bromo-7-methoxy-2-methylisoquinolin-1-one (102 g, 380.4 mmol) in acetonitrile (2 L) was added selectfluor (134.77 g, 380.4 mmol) at RT. The resulting mixture was stirred for 1 h at 80°C under nitrogen atmosphere. The mixture was allowed to cool down to RT. The reaction was quenched
with water (1000 ml). The resulting mixture was extracted with dichloromethane (3 x 1000 mL). The combined organic layers were washed with brine (1x1000 mL), dried over anhydrous sodium sulfate and filtrated. The filtrate was concentrated under reduced pressure to a residue which was purified by silica gel column chromatography eluting with PE / EA (1:01) to afford 6-bromo-4-fluoro-7-methoxy-2-methylisoquinolin-1-one (30 g, 27.56%) as a white solid. [M+H]⁺=285.9.

### Step 8: Preparation of 4-fluoro-7-methoxy-2-methyl-6-(4,4,5,5-tetramethyl-1,3,2-dioxaborolan-2-yl)isoquinolin-1-one.

A mixture of 6-bromo-4-fluoro-7-methoxy-2-methylisoquinolin-1-one (30 g, 104.9 mmol), 4,4,5,5-tetramethyl-2-(tetramethyl-1,3,2-dioxaborolan-2-yl)-1,3,2-dioxaborolane (53.26 g, 209.7 mmol), Pd(dppf)Cl₂ (3.84 g, 5.243 mmol), and potassium acetate (15.44 g, 157.3 mmol) in dioxane (300 mL) was stirred at 100°C for 2 h. After cooing to RT, the resulting mixture was concentrated under reduced pressure. The residue was purified by silica gel column chromatography, eluted with dichloromethane / methanol (20:01) to afford 4-fluoro-7-methoxy-2-methyl-6-(4,4,5,5-tetramethyl-1,3,2-dioxaborolan-2-yl)isoquinolin-1-one (19 g, 54.39%) as a white solid. [M+H]⁺=334.2.

### Step 9: Preparation of tert-butyl (2S,3S,5S)-2-fluoro-3-{[3-(4-fluoro-7-methoxy-2-methyl-1-oxoisoquinolin-6-yl)-1,2,4-triazin-6-yl](methyl)amino}-8-azabicyclo[3.2.1]octane-8-carboxylate.

Into a 40 mL 3-necked round-bottom flask purged and maintained with an inert atmosphere of nitrogen, was placed tert-butyl(1R,2S,3S,5S)-2-fluoro-3-[methyl[3-(methylsulfanyl)-1,2,4-triazin-6-yl]amino]-8-azabicyclo[3.2.1]octane-8-carboxylate (1.00 g, 2.6 mmol), 4-fluoro-7-methoxy-2-methyl-6-(4,4,5,5-tetramethyl-1,3,2-dioxaborolan-2-yl)isoquinolin-1-one (1.74 g, 5.2 mmol), copper(I)-3-methylsalicylate (1.40 g, 6.5 mmol), Pd(PPh₃)₄ (0.30 g, 0.3 mmol) and THF (10.00 mL). The resulting solution was stirred for 2 h at 70°C. The reaction was quenched by addition of 30 mL 10% wt ammonia-water and the solids were filtered. The resulting solution was extracted with 3x50 mL of dichloromethane. The combined organic phases were washed with 50 ml of brine, dried over anhydrous sodium sulfate, and concentrated under vacuum. The residue was purified by reverse flash chromatography with the following conditions: column, C18 silica gel; mobile phase, ACN in water (0.5% NH₄HCO₃), 25% to 85% gradient in 30 min; detector, UV 254 nm. This resulted in tert-butyl (285,3S,5S)-2-fluoro-3-{[3-(4-fluoro-7-methoxy-2-methyl-1-oxoisoquinolin-6-yl)-1,2,4-triazin-6-yl](methyl)amino}-8-azabicyclo[3.2.1]octane-8-carboxylate (800 mg, 56.54%) as a light yellow solid.

This procedure was repeated 13 times in parallel to afford tert-butyl (2S,3S,5S)-2-fluoro-3-{[3-(4-fluoro-7-methoxy-2-methyl-1-oxoisoquinolin-6-yl)-1,2,4-triazin-6-yl](methyl)amino}-8-azabicyclo[3.2.1]octane-8-carboxylate (12 g) as a yellow solid. The combined tert-butyl (2S,3S,5S)-2-fluoro-3-{[3-(4-fluoro-7-methoxy-2-methyl-1-oxoisoquinolin-6-yl)-1,2,4-triazin-6-yl](methyl)amino}-8-azabicyclo[3.2.1]octane-8-carboxylate (12 ) in THF (240 mL) was mixed with sulfhydryl functionalized Silica PSB-20 (1.2 g) at room temperature. The resulting mixture was stirred for 0.5 h at 50°C under nitrogen atmosphere, cooled downed to RT. Filtration, wash with THF (3 x 100 mL) and concentration gave the product. This procedure was repeated 8 times and the filtrate was concentrated under reduced pressure to afford tert-butyl (2S,3S,5S)-2-fluoro-3-{[3-(4-fluoro-7-methoxy-2-methyl-1-oxoisoquinolin-6-yl)-1,2,4-triazin-6-yl](methyl)amino}-8-azabicyclo[3.2.1]octane-8-carboxylate (12 g) as a yellow solid. [M+H]⁺=543.1.

### Step 10: Preparation of tert-butyl (2S,3S,5S)-2-fluoro-3-{[3-(4-fluoro-7-hydroxy-2-methyl-1-oxoisoquinolin-6-yl)-1,2,4-triazin-6-yl](methyl)amino}-8-azabicyclo[3.2.1]octane-8-carboxylate.

In a 3-necked round-bottom flask, to tert-butyl (2S,3S,5S)-2-fluoro-3-{[3-(4-fluoro-7-methoxy-2-methyl-1-oxoisoquinolin-6-yl)-1,2,4-triazin-6-yl](methyl)amino}-8-azabicyclo[3.2.1]octane-8-carboxylate (12 g, 22.1 mmol) in DMF (120 mL) was added sodium methanethiolate (7.75 g, 110.6 mmol). The resulting mixture was stirred for 2 h at 100°C under nitrogen atmosphere. The reaction was monitored by LCMS. The mixture was allowed to cool down to room temperature and quenched with water (100 ml). The aqueous layer was extracted with ethyl acetate (3 x 200 mL). The resulting mixture was concentrated under reduced pressure. The residue was purified by reverse flash chromatography with the following conditions: column, C18 silica gel; mobile phase, CH3CN in water, 25% to 85% gradient in 30 min; detector, UV 254 nm. This resulted in tert-butyl (2S,3S,5S)-2-fluoro-3-{[3-(4-fluoro-7-hydroxy-2-methyl-1-oxoisoquinolin-6-yl)-1,2,4-triazin-6-yl](methyl)amino}-8-azabicyclo[3.2.1]octane-8- carboxylate (8 g, 68.44%) as a light yellow solid. [M+H]⁺=529.4.

### Step 11: Preparation of 4-fluoro-6-(6-{[(2R,3S,5S)-2-fluoro-8-azabicyclo[3.2.1]octan-3-yl](methyl)amino}-1,2,4-triazin-3-yl)-7-hydroxy-2-methylisoquinolin-1-one (Compound 66).

To a stirred solution of tert-butyl (25,3S,5S)-2-fluoro-3-{[3-(4-fluoro-7-hydroxy-2-methyl-1-oxoisoquinolin-6-yl)-1,2,4-triazin-6-yl](methyl)amino}-8-azabicyclo[3.2.1]octane-8-carboxylate (8 g) in ethyl acetate (100 mL) was added saturated HCl in ethyl acetate (80 mL) dropwise at room temperature under nitrogen atmosphere. The resulting mixture was stirred for 1.5 h at room temperature under nitrogen atmosphere. The resulting mixture was concentrated under reduced pressure. The mixture was basified to pH 8~9 with saturated aqueous sodium bicarbonate solution. The aqueous layer was extracted with ethyl acetate (3 x 300 mL) and concentrated under reduced pressure. This resulted in 4-fluoro-6-(6-{[(2R,3S,5S)-2-fluoro-8-azabicyclo[3.2.1]octan-3-yl](methyl)amino}-1,2,4-triazin-3-yl)-7-hydroxy-2-methylisoquinolin-1-one (Compound 66) (5.3 g,80.18%) as a light yellow solid. LC[M+H]+=429.2. ¹H NMR (300 MHz, Chloroform-d) δ 12.57 (s, 1H), 8.68 (s, 1H), 8.40 (s, 1H), 8.03 (d, J = 2.2 Hz, 1H), 6.90 (d, J = 6.1 Hz, 1H), 5.29 (dddd, J = 34.3, 12.8, 5.8, 2.8 Hz, 1H), 4.74 (dt, J = 51.8, 3.4 Hz, 1H), 3.77 (s, 2H), 3.58 (s, 3H), 3.22 (d, J = 1.7 Hz, 3H), 2.29 (td, J = 12.7, 3.2 Hz, 1H), 2.12 - 1.99 (m, 2H), 1.92 - 1.79 (m, 2H), 1.77 - 1.67 (m, 1H).

### Example 8. Preparation of 2-Ethyl-4-fluoro-6-(6-{[(2R,3S,5S)-2-fluoro-8-azabicyclo[3.2.1]octan-3-yl](methyl)amino}-1,2,4-triazin-3-yl)-7-hydroxyisoquinolin-1-one (Compound 163).

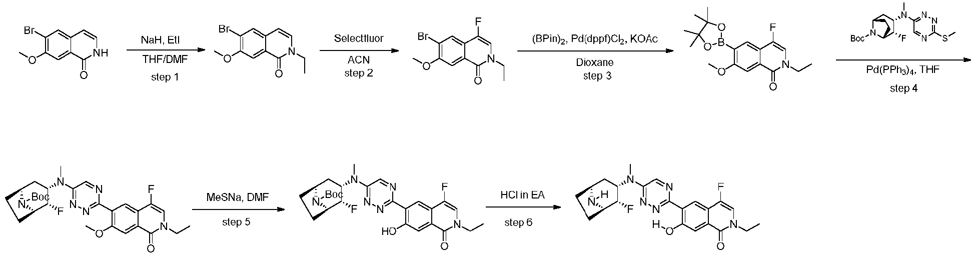

### Step 1: Preparation of 6-bromo-7-methoxy-2-ethylisoquinolin-1-one.

To the stirred suspension of 6-bromo-7-methoxy-2H-isoquinolin-1-one (245.00 g, 964.6 mmol) in THF (2.5 L) and DMF ( 1 L) in a 1 L 3-necked round-bottom flask was added sodium hydride (60% in mineral oil) (57.87 g, 1446.9 mmol) in portions at 0°C under nitrogen. After 30 min at 0°C , ethyl iodide (180.57 g, 1157.5 mmol) was added dropwise at 0°C. The resulting solution was warmed to ambient temperature and stirred for 1 h. The reaction mixture was poured into cold water (2L) and stirred for 15 min. The precipitated solids were collected by filtration and washed with cold water (3x1 L). The residue was purified by silica gel column chromatography, eluting with PE / EA (3:01) to afford 6-bromo-7-methoxy-2-ethylisoquinolin-1-one (230 g, 84.56%) as an off-white solid.

### Step 2: Preparation of 6-bromo-2-ethyl-4-fluoro-7-methoxyisoquinolin-1-one.

To 6-bromo-2-ethyl-7-methoxyisoquinolin-1-one (230 g, 815.2 mmol) in acetonitrile (4560 ml) was added selectfluor (288.80 g, 815.2 mmol) at room temperature. The resulting mixture was stirred for 1 h at 80°C under nitrogen. After cooling down to RT the reaction was quenched with water (800 ml).The resulting mixture was extracted with dichloromethane (3 x 1000 mL). The combined organic layers were washed with brine (1x1000 mL), dried over anhydrous sodium sulfate. Filtration and concentration gave a residue, which was purified by silica gel column chromatography eluting with PE / EA (1:01) to afford 6-bromo-2-ethyl-4-fluoro-7-methoxyisoquinolin-1-one (120 g, crude). The crude product was purified by reverse flash chromatography with the following conditions: column, C18 silica gel; mobile phase, ACN in water (0.1% NH4HCO3), 25% to 85% gradient in 30 min; detector, UV 254 nm to afford 6-bromo-2-ethyl-4-fluoro-7-methoxyisoquinolin-1-one (75 g, 30.65%) as a white solid. [M+H]+=300.

### Step 3: Preparation of afford 2-ethyl-4-fluoro-7-methoxy-6-(4,4,5,5-tetramethyl-1,3,2-dioxaborolan-2-yl)isoquinolin-1-one.

A mixture of 6-bromo-2-ethyl-4-fluoro-7-methoxyisoquinolin-1-one (75 g, 249.9 mmol), 4,4,5,5-tetramethyl-2-(tetramethyl-1,3,2-dioxaborolan-2-yl)-1,3,2-dioxaborolane (126.92 g, 499.8 mmol), Pd(dppf)Cl₂ (9.14 g, 12.5 mmol), potassium acetate (36.79 g, 374.8 mmol) in dioxane (750 mL) was stirred at 100°C for 2 h. After cooling, the resulting mixture was concentrated under reduced pressure. The residue was purified by silica gel column chromatography eluting with dichloromethane / methanol (20 : 1) to afford 2-ethyl-4-fluoro-7-methoxy-6-(4,4,5,5-tetramethyl-1,3,2-dioxaborolan-2-yl)isoquinolin-1-one (65 g, 74.92%) as a yellow solid. [M+H]+=348.

### Step 4: Preparation of tert-butyl (2S,3S,5S)-3-{[3-(2-ethyl-4-fluoro-7-methoxy-1-oxoisoquinolin-6-yl)-1,2,4-triazin-6-yl](methyl)amino}-2-fluoro-8-azabicyclo[3.2.1]octane-8-carboxylate.

A mixture of tert-butyl(1R,2S,3S,5S)-2-fluoro-3-[methyl[3-(methylsulfanyl)-1,2,4-triazin-6-yl]amino]-8-azabicyclo[3.2.1]octane-8-carboxylate (1.00 g, 2.6 mmol), 2-ethyl-4-fluoro-7-methoxy-6-(4,4,5,5-tetramethyl-1,3,2-dioxaborolan-2-yl)isoquinolin-1-one (1.81 g, 5.2 mmol), copper(I)-3-methylsalicylate (1.40 g, 6.5 mmol), Pd(PPh3)4 (0.30 g, 0.26 mmol) in THF (10 mL) was stirred for 2 h at 70°C under N. The reaction was quenched by the addition of 30 mL 10% ammonia / water and the solids were filtered out. The resulting solution was extracted with 3 x 50 mL of dichloromethane. The combined organic phases were washed with 50 ml of brine, dried over anhydrous sodium sulfate, and concentrated under vacuum. The residue was purified by reverse flash chromatography with the following conditions: column, C18 silica gel; mobile phase, ACN in water (0.5% NH4HCO3), 25% to 85% gradient in 30 min; detector, UV 254 nm. This resulted in tert-butyl (2S,3S,5S)-3-{[3-(2-ethyl-4-fluoro-7-methoxy-1-oxoisoquinolin-6-yl)-1,2,4-triazin-6-yl](methyl)amino}-2-fluoro-8-azabicyclo[3.2.1]octane-8-carboxylate 0.80 g (55.12%) as a light yellow solid.

This procedure was repeated 20 times in parallel to afford tert-butyl (2S,3S,5S)-3-f [3-(2-ethyl-4-fluoro-7-methoxy-1-oxoisoquinolin-6-yl)-1,2,4-triazin-6-yl](methyl)amino}-2-fluoro-8-azabicyclo[3.2.1]octane-8-carboxylate (22 g) as a yellow solid. The combined tert-butyl (2S,3S,5S)-3-{[3-(2-ethyl-4-fluoro-7-methoxy-1-oxoisoquinolin-6-yl)-1,2,4-triazin-6-yl](methyl)amino}-2-fluoro-8-azabicyclo[3.2.1]octane-8-carboxylate (22 g, 39.55 mmol) in THF (240 mL) was mixed with sulfhydryl functionalised silica PSB-20 (2.2 g) at room temperature. The resulting mixture was stirred for 0.5 h at 50°C under N, cooled to RT and filtered, the filter cake was washed with THF (3x10 mL). This procedure was repeated 8 times and the filtrate was concentrated under reduced pressure to afford tert-butyl (2S,3S,5S)-3-{[3-(2-ethyl-4-fluoro-7-methoxy-1-oxoisoquinolin-6-yl)-1,2,4-triazin-6-yl](methyl)amino}-2-fluoro-8-azabicyclo[3.2.1]octane-8-carboxylate (21 g) as a yellow solid. [M+H]⁺=556.26.

### Step 5: Preparation of tert-butyl (2S,3S,5S)-3-{[3-(2-ethyl-4-fluoro-7-hydroxy-1-oxoisoquinolin-6-yl)-1,2,4-triazin-6-yl](methyl)amino}-2-fluoro-8-azabicyclo[3.2.1]octane-8-carboxylate.

To tert-butyl (2S,3S,5S)-3-{[3-(2-ethyl-4-fluoro-7-methoxy-1-oxoisoquinolin-6-yl)-1,2,4-triazin-6-yl](methyl)amino}-2-fluoro-8-azabicyclo[3.2.1]octane-8-carboxylate (20 g, 35.931 mmol) in DMF (182 mL) was added sodium methanethiolate (12.59 g, 179.7 mmol). The resulting mixture was stirred for 2 h at 100°C. The reaction was monitored by LCMS. The mixture was allowed to cool down to room temperature and quenched with water (200ml). The aqueous layer was extracted with ethyl acetate (3 x 200 mL). Evaporation gave the residue which was purified by reverse flash chromatography with the following conditions: column, C18 silica gel; mobile phase, CH3CN in water, 25% to 85% gradient in 30 min; detector, UV 254 nm. This resulted in tert-butyl (2S,3S,5S)-3-{[3-(2-ethyl-4-fluoro-7-hydroxy-1-oxoisoquinolin-6-yl)-1,2,4-triazin-6-yl](methyl)amino}-2-fluoro-8-azabicyclo[3.2.1]octane-8-carboxylate (15 g, 76.94%) as a light yellow solid. [M+H]+=542.2.

### Step 6: Preparation of 2-ethyl-4-fluoro-6-(6-{[(2R,3S,5S)-2-fluoro-8-azabicyclo[3.2.1]octan-3-yl](methyl)amino}-1,2,4-triazin-3-yl)-7-hydroxyisoquinolin-1-one (Compound 163).

To a stirred solution of tert-butyl (2S,3S,5S)-3-{[3-(2-ethyl-4-fluoro-7-hydroxy-1-oxoisoquinolin-6-yl)-1,2,4-triazin-6-yl](methyl)amino}-2-fluoro-8-azabicyclo[3.2.1]octane-8-carboxylate (15 g) in ethyl acetate (150 mL) was added HCl(gas) in EA (150 mL) dropwise at room temperature under N. The resulting mixture was stirred for 1.5 h at RT under. The resulting mixture was concentrated under reduced pressure. The mixture was basified to pH 8~9 with saturated aqueous sodium bicarbonate solution. The aqueous layer was extracted with ethyl acetate (3 x 300 mL) and concentrated under reduced pressure. This resulted in 2-ethyl-4-fluoro-6-(6-{[(2R,3S,5S)-2-fluoro-8-azabicyclo[3.2.1]octan-3-yl](methyl)amino}-1,2,4-triazin-3-yl)-7-hydroxyisoquinolin-1-one (Compound 171) (11.6 g) as a light yellow solid. M+H]+=443.25. ¹H- NMR (300 MHz, Chloroform-d) δ 12.56 (s, 1H), 8.71 (s, 1H), 8.42 (s, 1H), 8.06 (d, J = 2.2 Hz, 1H), 6.91 (d, J = 6.2 Hz, 1H), 5.31 (dddd, J = 34.3, 12.8, 5.8, 2.8 Hz, 1H), 4.86 - 4.61 (m, 1H), 4.03 (q, J = 7.2 Hz, 2H), 3.78 (s, 2H), 3.22 (d, J = 1.6 Hz, 3H), 2.30 (td, J = 12.7, 3.2 Hz, 1H), 2.06 (s, 3H), 1.93 - 1.79 (m, 2H), 1.71 (dd, J = 12.2, 5.3 Hz, 1H), 1.40 (t, J = 7.2 Hz, 3H).

### Example 9. SMN Quantitative Splicing Assay.

Spinal muscular atrophy (SMA) patient fibroblasts (GM03813, Coriell) were plated in 96-well plates at 50,000 cells/well. Immediately after plating, cells were dosed with compounds for 24 h at concentrations ranging from 2.5µM to 0.6nM (0.1% DMSO). Treated cells were lysed and cDNA synthesized using the Fast Advanced Cells-to-Ct kit (Thermofisher A35378) according to the manufacturer's instructions. 2 µL of each cDNA were used in qPCR reactions. The qPCR reactions were prepared in 384-well plates in 10 µL volume, using TaqMan^{™} Fast Advanced Master Mix (ThermoFisher; 4444965) with primers and probes shown in Table 4 below. Reactions were run in a Quant Studio 6 qPCR instrument with default settings.

**Table 4**

| Target | Primer 1 | Probe | Primer 2 |
|---|---|---|---|
| SMN FL-FAM | (SEQ ID NO: 1) | (SEQ ID NO: 2) | (SEQ ID NO: 3) |
| | 5'GCTCACATTCCT TAAATTAAGGAGA AA3' | 5'CTGGCATAGAGCAGCA CTAAATGACACCAC3' | 5'TCCAGATCTGTC TGATCGTTTCTT3' |
| SMN Δ7-FAM | (SEQ ID NO: 4) | (SEQ ID NO: 5) | (SEQ ID NO: 6) |
| | 5’TGGCTATCATAC TGGCTATTATATG GAA3’ | 5'CTGGCATAGAGCAGCA CTAAATGACACCAC3' | 5'TCCAGATCTGTC TGATCGTTTCTT3' |
| TBP-YAK (endogeno us control) | (SEQ ID NO: 7) | (SEQ ID NO: 8) | (SEQ ID NO: 9) |
| | 5’TCGGAGAGTTCT GGGATT 3’ | 5'CCGCAGCTGCAAAATA TTGTATCCACA3' | 5'AAGTGCAATGGT CTTTAGGT3' |

### Example 10. SMN Protein Assay.

Spinal muscular atrophy (SMA) patient fibroblasts were plated in 96-well plates at 7,000 cells/well. Compounds were added in concentrations ranging from 0.6nM to 2.5 µM, incubated for 48 hours and the cells were lysed with 100 µL of lysis buffer. 20 µL of lysate was used for SMN protein measurement by Mesoscale Discovery (MSD) assay developed by PharmOptima (Michigan). A standard curve prepared with SMN protein ranging from 1µg/ml to 19.5pg/ml was used in each MSD plate to calculate the absolute SMN protein amount in each sample.

Results for both the SMN Quantitative Splicing Assay and Protein Assay are shown in Table 5 below.

**Table 5 - * EC₅₀/IC₅₀ range (nM): 0.01 ≤ A ≤ 15; 16 ≤ B ≤ 50; 51 ≤ C ≤ 100; 101 ≤ D ≤ 500; 501 ≤ E ≤ 10,000; N/T - not tested**

| (reference compounds are denoted with a dagger symbol (†)). | | |
|---|---|---|
| **Compound** | **SMN2 Protein EC50 (nM)** | **SMN2 Splicing D7 IC50 (nM)** |
| 1A | B | B |
| 1B | A | B |
| 2A | A | A |
| 2B | A | A |
| 3A | B | B |
| 3B | A | A |
| 4A | B | B |
| 4B | A | A |
| 5A | D | D |
| 5B | A | B |
| 6A | A | B |
| 6B | A | A |
| 7A | A | A |
| 7B | A | B |
| 9 | A | N/T |
| 17 | B | B |
| 18 | A | A |
| 19 | D | D |
| 20 | B | B |
| 21 (†) | B | D |
| 22 (†) | A | B |
| 23 | E | D |
| 24 | B | B |
| 25 | E | D |
| 26 | B | C |
| 27 | A | A |
| 28 | A | A |
| 29 | E | E |
| 30 | D | D |
| 31 | C | C |
| 32 | A | A |
| 33 | B | D |
| 34 | A | A |
| 35 | A | A |
| 36 | B | B |
| 37 | A | A |
| 38 | A | A |
| 39 (†) | C | D |
| 40 (†) | A | B |
| 43 (†) | B | C |
| 44 (†) | A | A |
| 45 | B | B |
| 46 | A | A |
| 47 (†) | B | C |
| 48 (†) | A | A |
| 51 | C | C |
| 52 | A | B |
| 53 | D | C |
| 54 | D | B |
| 55 | B | B |
| 56 | B | A |
| 57 | B | D |
| 58 | B | B |
| 59 | B | B |
| 60 | A | B |
| 61 | A | C |
| 62 | A | A |
| 63 | A | B |
| 64 | A | A |
| 65 | A | B |
| 66 | A | A |
| 67 | C | D |
| 68 | E | D |
| 69 | A | A |
| 72 | C | C |
| 75 | B | D |
| 76 | A | B |
| 77 | B | B |
| 78 | A | A |
| 79 | B | D |
| 80 | A | A |
| 81 | B | C |
| 82 | A | A |
| 83 | B | D |
| 84 | A | A |
| 85 | B | A |
| 86 | C | C |
| 87 | A | A |
| 88 | C | B |
| 89 | C | D |
| 90 | B | B |
| 93 | B | B |
| 96 | A | A |
| 99 | D | D |
| 100 | A | B |
| 101 | A | A |
| 102 | A | A |
| 105 | B | D |
| 106 | A | B |
| 109 | E | E |
| 110 | D | C |
| 111 | C | D |
| 112 | B | B |
| 115 | B | D |
| 116 | B | C |
| 117 | B | C |
| 118 | A | A |
| 121 | A | A |
| 122 | E | C |
| 123 | A | A |
| 124 | B | B |
| 125 | B | A |
| 126 | C | D |
| 127 | A | A |
| 128 | A | B |
| 131 | A | A |
| 132 | C | B |
| 135 | A | A |
| 136 | A | A |
| 137 | A | A |
| 138 | A | B |
| 139 | A | B |
| 140 | B | D |
| 141 | D | D |
| 142 | A | B |
| 143 | C | D |
| 144 | A | A |
| 147 | A | A |
| 148 | A | A |
| 149 | C | A |
| 150 | A | A |
| 151 | A | A |
| 152 | A | A |
| 155 | A | A |
| 156 | A | B |
| 157 | B | B |
| 158 | A | A |
| 159 | A | B |
| 160 | D | D |
| 161 | A | C |
| 162 | A | C |
| 163 | A | A |
| 164 | B | C |
| 165 | A | A |
| 166 | A | B |
| 167 | A | A |
| 168 | A | C |
| 169 | A | B |
| 170 | B | C |
| 171 | A | A |
| 172 | A | A |
| 173 | B | B |
| 174 | A | A |
| 175 | A | A |
| 176 | A | B |
| 177 | A | A |
| 178 | A | A |
| 181 | A | A |
| 182 | A | A |
| 183 | A | A |
| 184 | A | A |
| 185 | A | A |
| 186 | A | B |
| 187 | A | A |
| 188 | A | A |
| 191 | A | A |
| 192 | A | A |
| 193 | A | A |
| 194 | A | A |
| 195 | A | A |
| 196 | C | B |
| 199 | A | A |
| 200 | B | B |
| 201 | A | A |
| 202 | A | C |
| 204 | A | A |
| 205 | A | A |
| 206 | A | A |
| 207 | A | B |
| 208 | A | A |
| 209 | D | C |
| 210 | A | A |
| 211 | E | D |
| 212 | E | C |
| 213 | A | B |
| 214 | E | E |
| 215 | B | B |
| 218 | N/T | C |
| 219 | N/T | B |
| 221 | N/T | E |
| 222 | N/T | D |
| 223 | A | A |

## Claims

1. A compound of Formula (1): 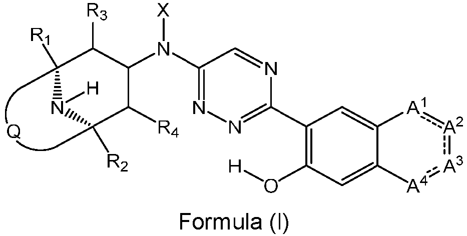
or a pharmaceutically acceptable salt thereof;
wherein:
Q is C₂-C₄ alkylene optionally substituted with 1, 2, 3, or 4 substituents each independently selected from fluorine, OH, CH₃, and OCH₃; or
Q is -CH₂OCH₂-, -OCH₂CH₂O-, -OCH₂CH₂OCH₂CH₂O-, or -OCH₂CH₂OCH₂CH₂OCH₂CH₂O-;
X is hydrogen or CH₃; or
X is unsubstituted C₃-C₆cycloalkyl; or
X is cyclopropyl, cyclobutyl, or cyclopentyl, each substituted with 1, 2, or 3 substituents each independently selected from fluorine, OH, CH₃, and OCH₃;
each R₁ and R₂ is independently hydrogen, halogen, or CH₃;
each R₃ and R₄ is independently hydrogen or halogen;
each A¹, A², and A⁴ is independently N, -NR^{Y1}-, -O-, -S-, or CR^{A1};
A³ is -NR^{Y1}-, -O-, -S-, or CR^{A1};
each 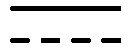 is independently a single or double bond;
each R^{A1} is independently hydrogen, halogen, =O, or C₁-C₆ alkyl, wherein C₁-C₆ alkyl is optionally substituted with one or more halogen; and
each R^{Y1} is independently hydrogen or C₁-C₆ alkyl, wherein C₁-C₆ alkyl is optionally substituted with one or more halogen.

2. The compound of claim 1, or a pharmaceutically acceptable salt thereof, wherein:
the compound has the structure of Formula (laa*):

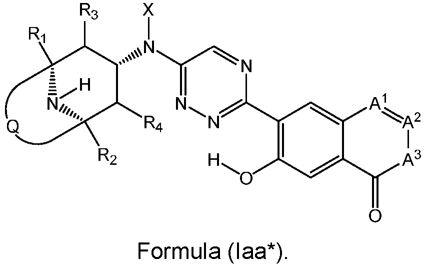

3. The compound of claim 1, or a pharmaceutically acceptable salt thereof, wherein:
A⁴ is CH, C(CH₃), N, O, or C(=O).

4. The compound of any one of claims 1-3, or a pharmaceutically acceptable salt thereof, wherein:
A¹ is CH, CF, C(CH₃), N, O, or C(=O).

5. The compound of any one of claims 1-4, or a pharmaceutically acceptable salt thereof, wherein:
A² is CH, C(CH₃), or N.

6. The compound of any one of claims 1-5, or a pharmaceutically acceptable salt thereof, wherein:
A³ is CH, C(CH₃), NH, or N(CH₃).

7. The compound of any one of claims 1 and 3-6, or a pharmaceutically acceptable salt thereof, wherein:
one of A¹, A², _{A}³, and A⁴ is C(=O).

8. The compound of any one of claims 1-7, or a pharmaceutically acceptable salt thereof, wherein:
at least one of R₃ and R₄ is fluorine.

9. The compound of any one of claims 1-8, or a pharmaceutically acceptable salt thereof, wherein:
R₃ is fluorine and R₄ is hydrogen; or
R₃ is hydrogen and R₄ is fluorine.

10. The compound of any one of claims 1-9, or a pharmaceutically acceptable salt thereof, wherein:
R₁ is hydrogen or CH₃; and
R₂ is hydrogen or CH₃.

11. The compound of any one of claims 1-10, or a pharmaceutically acceptable salt thereof, wherein:
Q is unsubstituted C₂-C₄ alkylene.

12. The compound of any one of claims 1-7, or a pharmaceutically acceptable salt thereof, wherein: 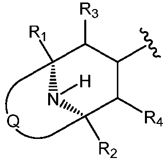 is selected from the group consisting of:

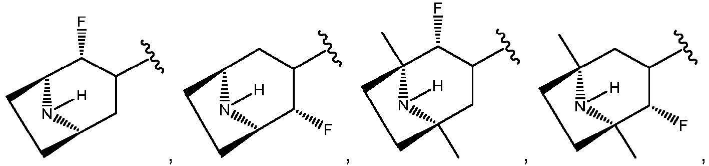

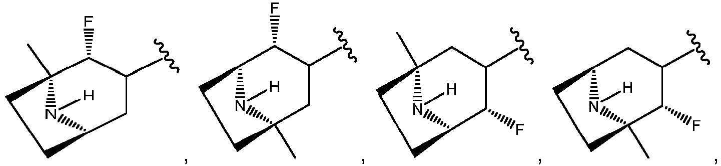

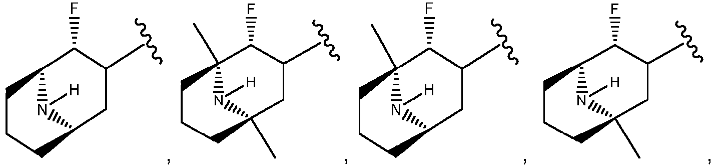

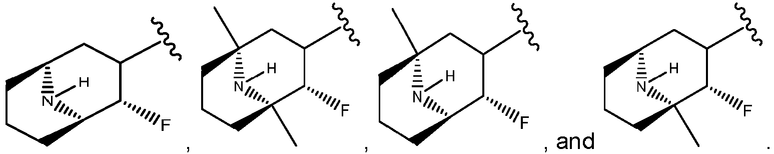

13. The compound of any one of claims 1-12, or a pharmaceutically acceptable salt thereof, wherein:
X is -CH₃.

14. The compound of claim 1, or a pharmaceutically acceptable salt thereof, wherein:
the compound is selected from:

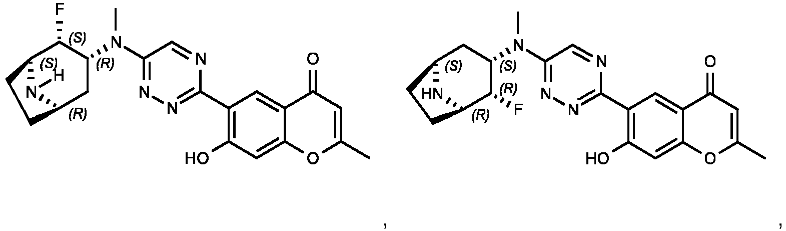

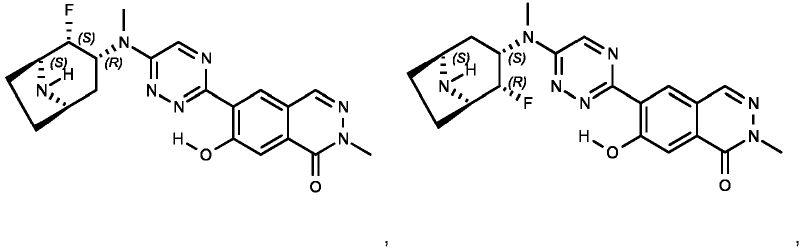

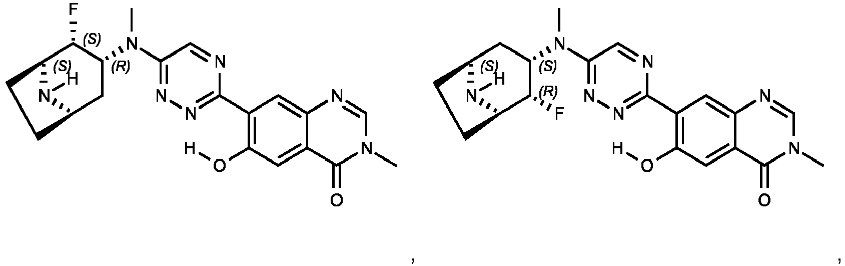

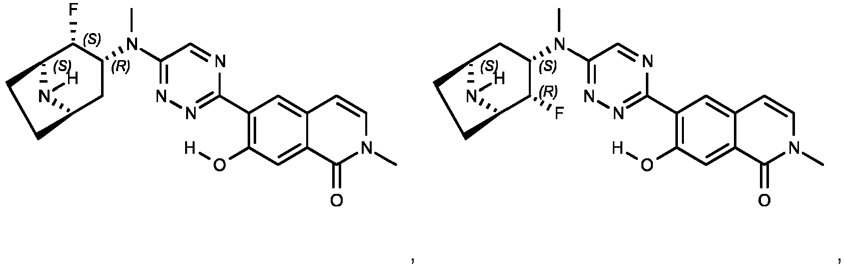

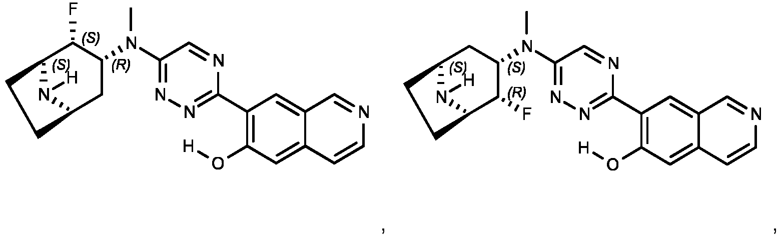

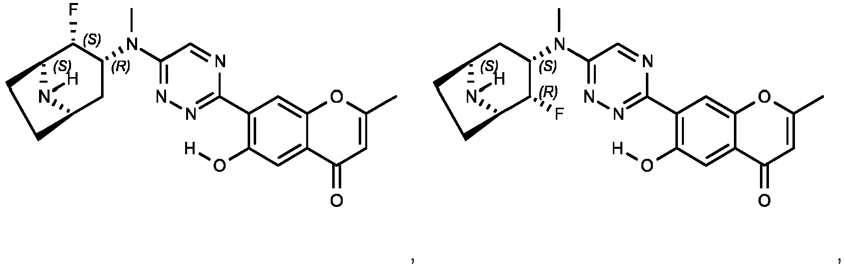

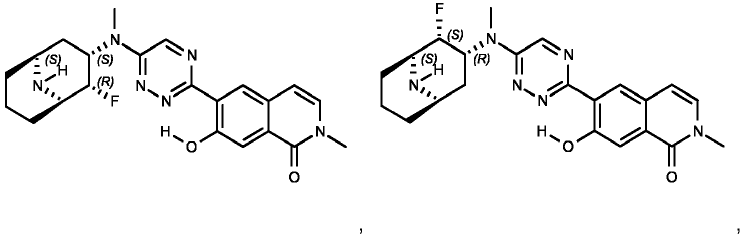

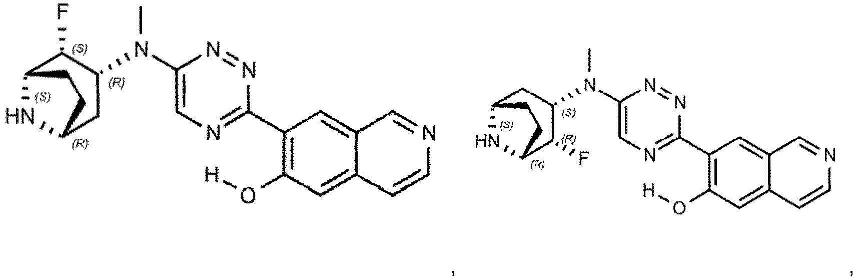

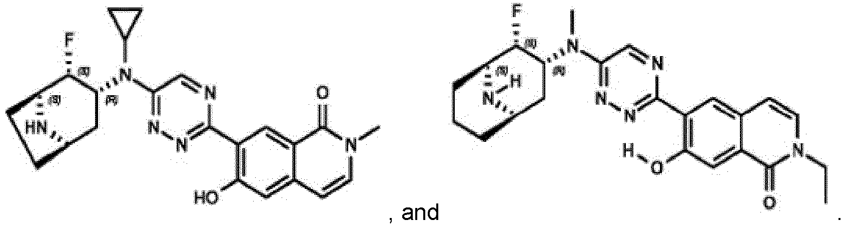

15. A pharmaceutical composition comprising a compound of any one of claims 1-14, or a pharmaceutically acceptable salt thereof, and a pharmaceutically acceptable excipient or carrier.

16. A compound of any one of claims 1-14, or a pharmaceutically acceptable salt thereof, or a pharmaceutical composition of claim 15,
for use in a method of treating a disease or condition,
wherein the method comprises administering the compound, salt, or pharmaceutical composition to a subject in need thereof.

## Patentansprüche

1. Verbindung der Formel (I): 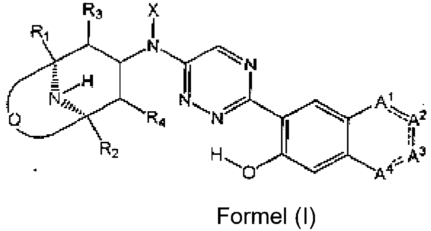
oder ein pharmazeutisch annehmbares Salz davon;
worin:
Q C₂₋₄-Alkylen ist, das gegebenenfalls mit 1, 2, 3 oder 4 Substituenten substituiert ist, die jeweils unabhängig aus Fluor, OH, CH₃ und OCH₃ ausgewählt sind oder
Q -CH₂OCH₂-, -OCH₂CH₂O-, -OCH₂CH₂OCH₂CH₂O- oder -OCH₂CH₂OCH₂CH₂O-CH₂CH₂O- ist;
X Wasserstoff oder CH₃ ist oder
X unsubstituiertes C₃₋₆-Cycloalkyl ist oder
X Cyclopropyl, Cyclobutyl oder Cyclopentyl ist, die jeweils mit 1, 2 oder 3 Substituenten substituiert sind, die jeweils unabhängig aus Fluor, OH, CH₃ und OCH₃ ausgewählt sind;
R₁ und R₂ jeweils unabhängig voneinander Wasserstoff, Halogen oder CH₃ sind;
R₃ und R₄ jeweils unabhängig voneinander Wasserstoff oder Halogen sind;
A¹, A² und A⁴ jeweils unabhäbngig voneinander N, -NR^{Y1}-, -O-, -S- oder CR^{A1} sind;
A³ -NR^{Y1}-, -O-, -S- oder CR^{A1} ist; 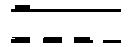
jeweils unabhängig eine Einfachbindung oder eine Doppelbindung ist;
R^{A1} jeweils unabhängig Wasserstoff, Halogen, =O oder C₁₋₆-Alkyl ist, worin das C₁₋₆-Alkyl gegebenenfalls mit einem oder mehreren Halogenatomen substituiert ist; und
R^{Y1} jeweils unabhängig Wasserstoff oder C₁₋₆-Alkyl ist, worin das C₁₋₆-Alkyl gegebenenfalls mit einem oder mehreren Halogenatomen substituiert ist.

2. Verbindung nach Anspruch 1 oder ein pharmazeutisch annehmbares Salz davon, worin:
die Verbindung eine Struktur der Formel (Iaa*) aufweist:

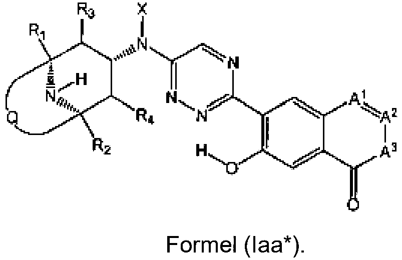

3. Verbindung nach Anspruch 1 oder ein pharmazeutisch annehmbares Salz davon, worin:
A⁴ CH, C(CH₃), N, O oder C(=O) ist.

4. Verbindung nach einem der Ansprüche 1 bis 3 oder ein pharmazeutisch annehmbares Salz davon, worin:
A¹ CH, CF, C(CH₃), N, O oder C(=O) ist.

5. Verbindung nach einem der Ansprüche 1 bis 4 oder ein pharmazeutisch annehmbares Salz davon, worin:
A² CH, C(CH₃) oder N ist.

6. Verbindung nach einem der Ansprüche 1 bis 5 oder ein pharmazeutisch annehmbares Salz davon, worin:
A³ CH, C(CH₃), NH der N(CH₃) ist.

7. Verbindung nach einem der Ansprüche 1 und 3 bis 6 oder ein pharmazeutisch annehmbares Salz davon, worin:
einer von A¹, A², A³ und A⁴ C(=O) ist.

8. Verbindung nach einem der Ansprüche 1 bis 7 oder ein pharmazeutisch annehmbares Salz davon, worin:
zumindest einer von R₃ und R₄ Fluor ist.

9. Verbindung nach einem der Ansprüche 1 bis 8 oder ein pharmazeutisch annehmbares Salz davon, worin:
R₃ Fluor ist und R₄ Wasserstoff ist; oder
R₃ Wasserstoff ist und R₄ Fluor ist.

10. Verbindung nach einem der Ansprüche 1 bis 9 oder ein pharmazeutisch annehmbares Salz davon, worin:
R₁ Wasserstoff oder CH₃ ist; und
R₂ Wasserstoff oder CH₃ ist.

11. Verbindung nach einem der Ansprüche 1 bis 10 oder ein pharmazeutisch annehmbares Salz davon, worin:
Q unsubstituiertes C₂₋₄-Alkylen ist.

12. Verbindung nach einem der Ansprüche 1 bis 7 oder ein pharmazeutisch annehmbares Salz davon, worin: 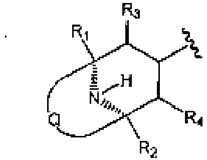 aus der aus Folgendem bestehenden Gruppe ausgewählt ist:

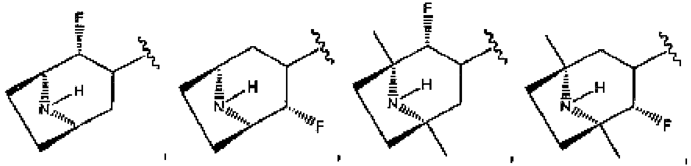

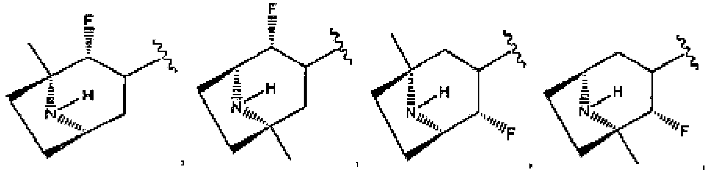

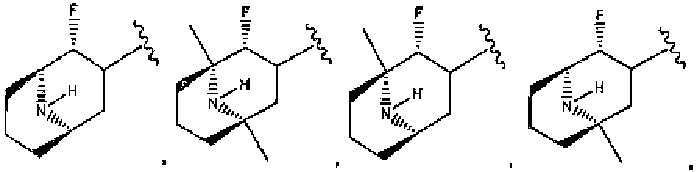

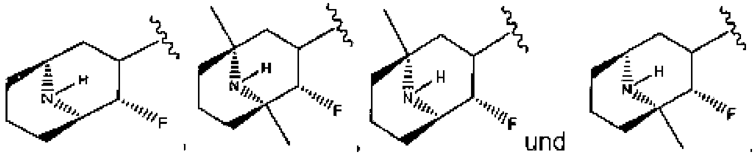

13. Verbindung nach einem der Ansprüche 1 bis 12 oder ein pharmazeutisch annehmbares Salz davon, worin:
X -CH₃ ist.

14. Verbindung nach Anspruch 1 oder ein pharmazeutisch annehmbares Salz davon, worin:
die Verbindung aus den folgenden ausgewählt ist:

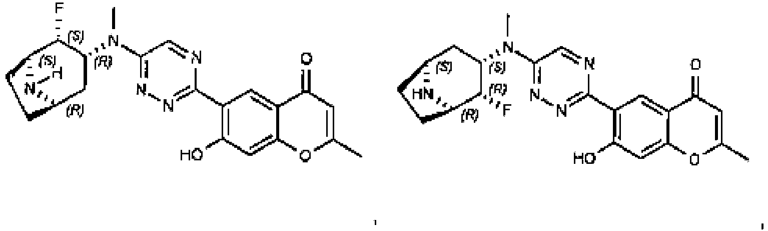

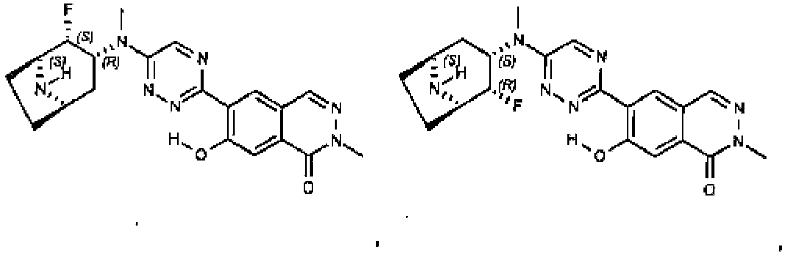

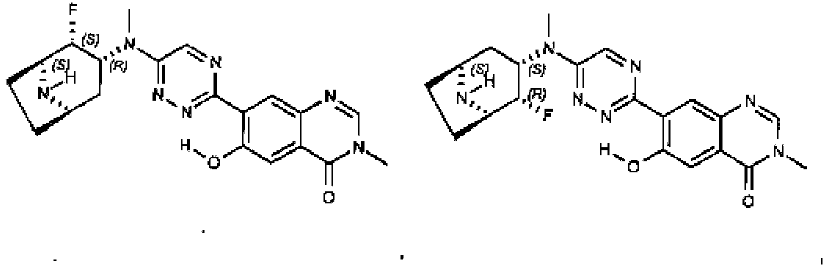

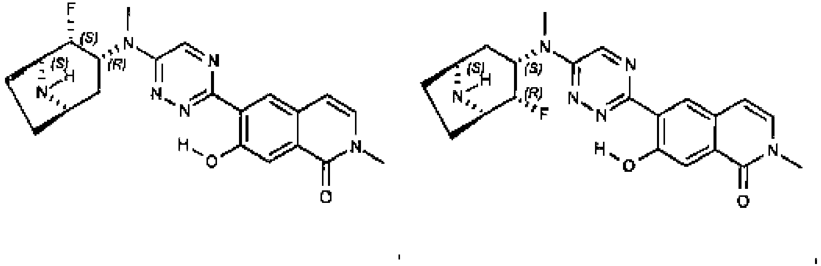

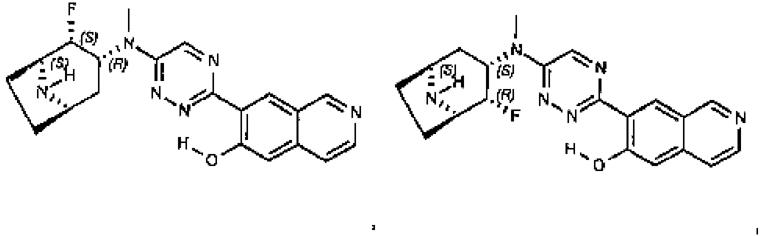

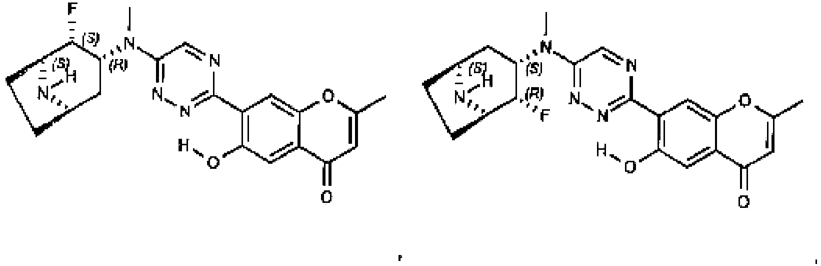

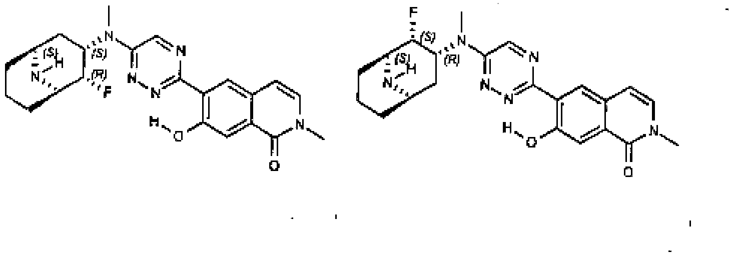

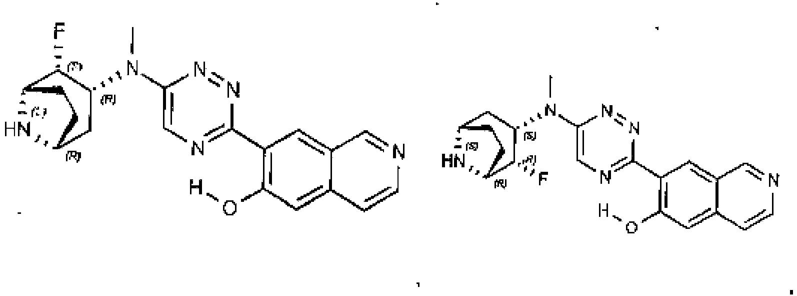

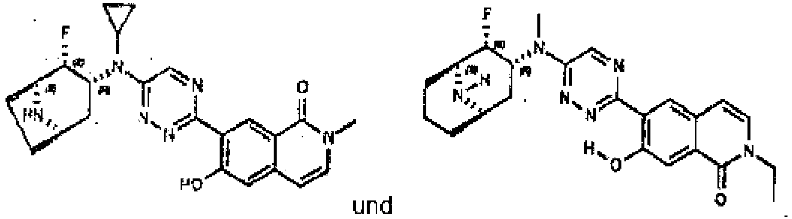

15. Pharmazeutische Zusammensetzung, die eine Verbindung nach einem der Ansprüche 1 bis 14 oder ein pharmazeutisch annehmbares Salz davon und einen pharmazeutisch annehmbaren Hilfsstoff oder Träger umfasst.

16. Verbindung nach einem der Ansprüche 1 bis 14 oder ein pharmazeutisch annehmbares Salz davon oder pharmazeutische Zusammensetzung nach Anspruch 15,
zur Verwendung in einem Verfahren zur Behandlung einer Erkrankung oder eines Leidens,
wobei das Verfahren das Verabreichen der Verbindung, des Salzes oder der pharmazeutischen Zusammensetzung an ein bedürftiges Individuum umfasst.

## Revendications

1. Composé de formule (I) : 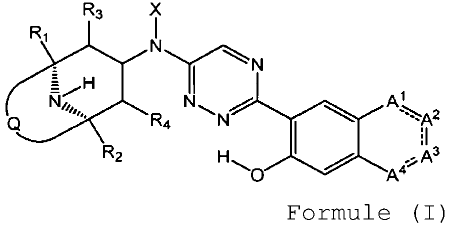
ou un sel pharmaceutiquement acceptable de celui-ci ;
dans lequel :
Q est un alkylène en C₂-C₄ facultativement substitué par 1, 2, 3 ou 4 substituants choisis chacun indépendamment parmi le fluor, OH, CH₃ et OCH₃ ; ou
Q est -CH₂OCH₂-, -OCH₂CH₂O-, -OCH₂CH₂OCH₂CH₂O-, ou - OCH₂CH₂OCH₂CH₂OCH₂CH2O- ;
X est de l'hydrogène ou CH₃ ; ou
X est un cycloalkyle en C₃-C₆ non substitué ; ou
X est un cyclopropyle, cyclobutyle ou cyclopentyle, chacun substitué par 1, 2 ou 3 substituants choisis chacun indépendamment parmi le fluor, OH, CH₃ et OCH₃ ;
chaque R₁ et R₂ est indépendamment de l'hydrogène, un halogène ou CH₃ ;
chaque R₃ et R₄ est indépendamment de l'hydrogène ou un halogène ;
chaque A¹, A² et A⁴ est indépendamment N, -NR^{Y1}-, -O-, -S- ou CR^{A1} ;
A³ est -NR^{Y1}-, -O-, -S-, ou CR^{A1} ;
chacun 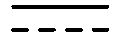 est indépendamment une liaison simple ou double ;
chaque R^{A1} est indépendamment de l'hydrogène, un halogène, =0 ou un alkyle en C₁-C₆, dans lequel un alkyle en C₁-C₆ est facultativement substitué par un ou plusieurs halogènes ; et
chaque R^{Y1} est indépendamment de l'hydrogène ou un alkyle en C₁-C₆, dans lequel un alkyle en C₁-C₆ est facultativement substitué par un ou plusieurs halogènes.

2. Composé selon la revendication 1, ou un sel pharmaceutiquement acceptable de celui-ci, dans lequel :
le composé présente la structure de formule (Iaa*) :

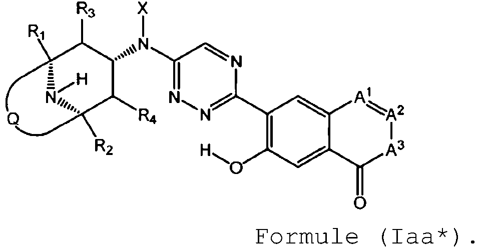

3. Composé selon la revendication 1, ou un sel pharmaceutiquement acceptable de celui-ci, dans lequel :
A⁴ est CH, C(CH₃), N, O ou C(=O).

4. Composé selon l'une quelconque des revendications 1 à 3, ou un sel pharmaceutiquement acceptable de celui-ci, dans lequel :
A¹ est CH, CF, C(CH₃), N, O ou C(=O).

5. Composé selon l'une quelconque des revendications 1 à 4, ou un sel pharmaceutiquement acceptable de celui-ci, dans lequel :
A² est CH, C(CH₃) ou N.

6. Composé selon l'une quelconque des revendications 1 à 5, ou un sel pharmaceutiquement acceptable de celui-ci, dans lequel :
A³ est CH, C(CH₃), NH ou N(CH₃).

7. Composé selon l'une quelconque des revendications 1 et 3 à 6, ou un sel pharmaceutiquement acceptable de celui-ci, dans lequel :
l'un de A¹, A², A³ et A⁴ est C(=O).

8. Composé selon l'une quelconque des revendications 1 à 7, ou un sel pharmaceutiquement acceptable de celui-ci, dans lequel :
au moins un de R₃ et R₄ est du fluor.

9. Composé selon l'une quelconque des revendications 1 à 8, ou un sel pharmaceutiquement acceptable de celui-ci, dans lequel :
R₃ est du fluor et R₄ est de hydrogène ; ou
R₃ est de l'hydrogène et R₄ est du fluor.

10. Composé selon l'une quelconque des revendications 1 à 9, ou un sel pharmaceutiquement acceptable de celui-ci, dans lequel :
R₁ est de l'hydrogène ou CH₃ ; et
R₂ est de l'hydrogène ou CH₃.

11. Composé selon l'une quelconque des revendications 1 à 10, ou un sel pharmaceutiquement acceptable de celui-ci, dans lequel :
Q est un alkylène en C₂-C₄ non substitué.

12. Composé selon l'une quelconque des revendications 1 à 7, ou un sel pharmaceutiquement acceptable de celui-ci, dans lequel : 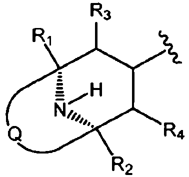 est choisi dans le groupe comprenant :

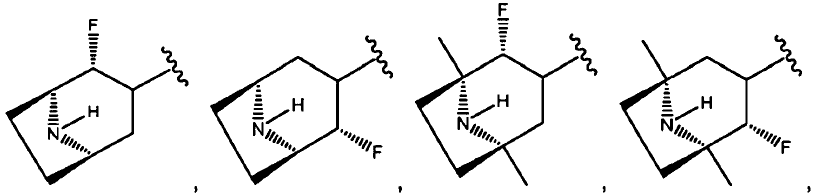

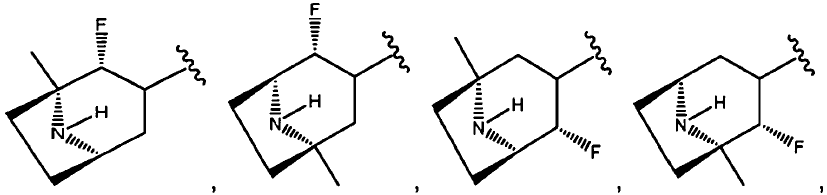

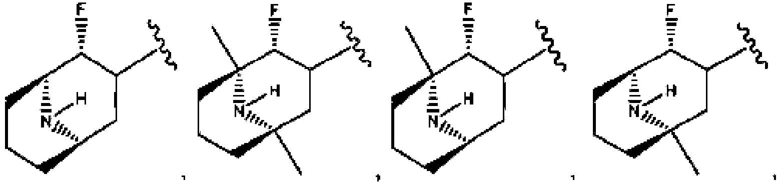

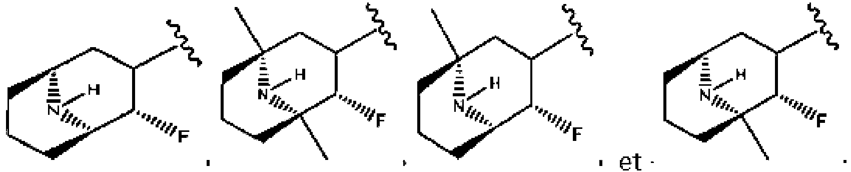

13. Composé selon l'une quelconque des revendications 1 à 12, ou un sel pharmaceutiquement acceptable de celui-ci, dans lequel :
X est -CH₃.

14. Composé selon la revendication 1, ou un sel pharmaceutiquement acceptable de celui-ci, dans lequel :
le composé est choisi parmi :

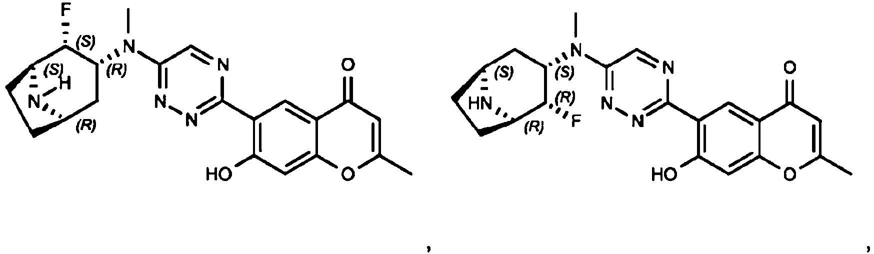

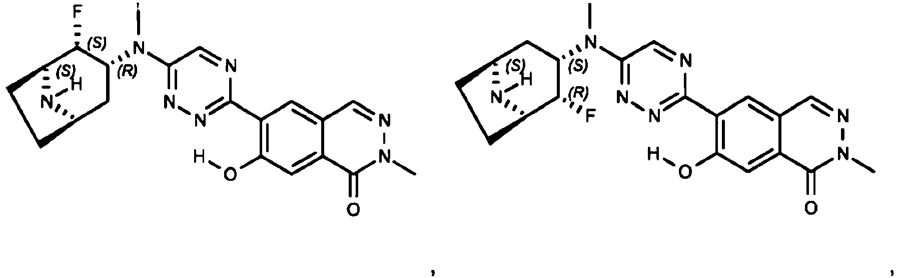

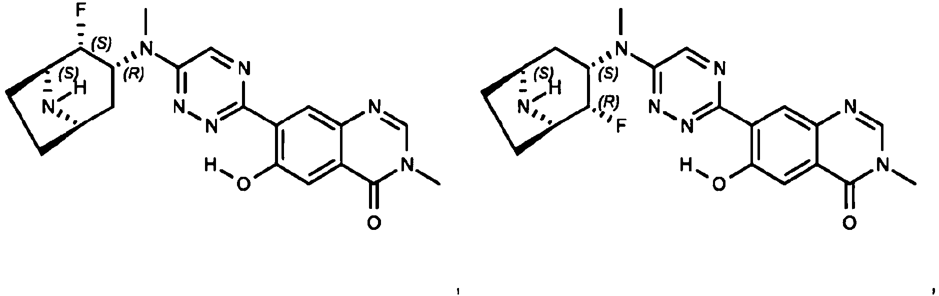

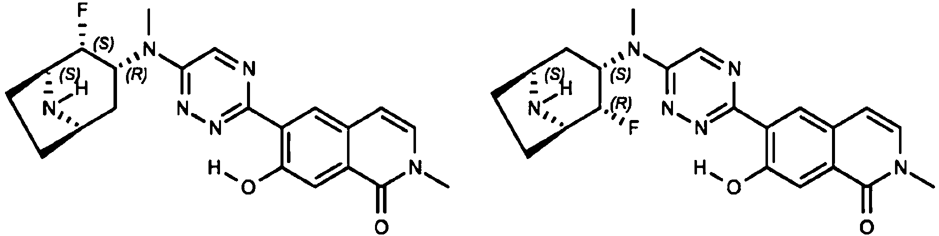

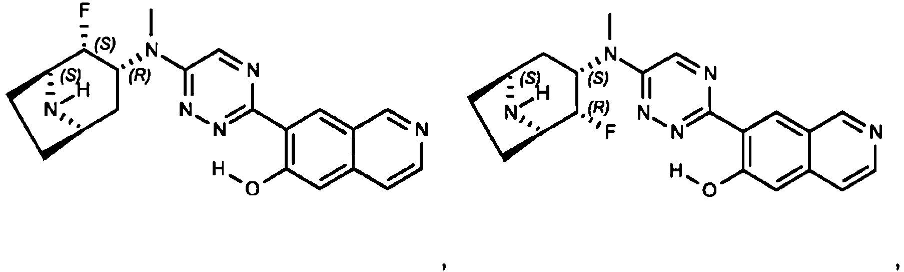

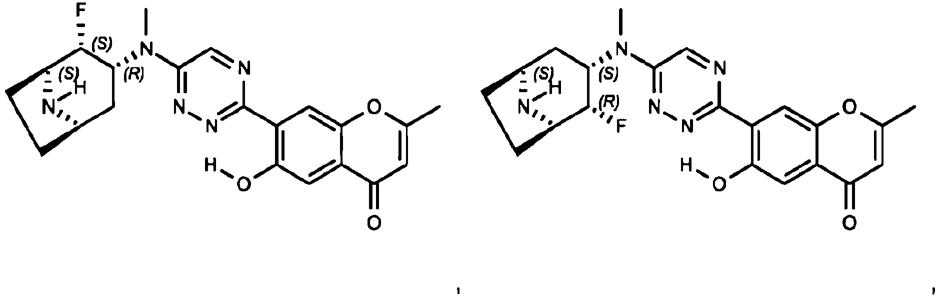

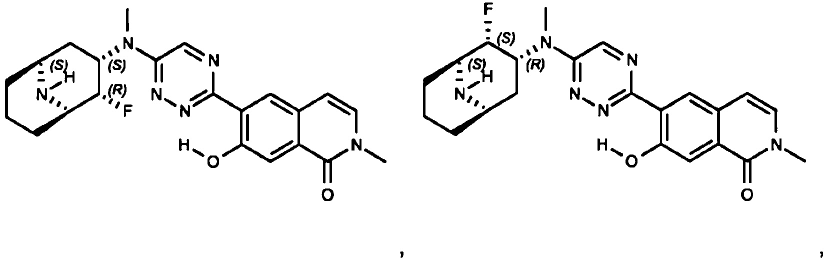

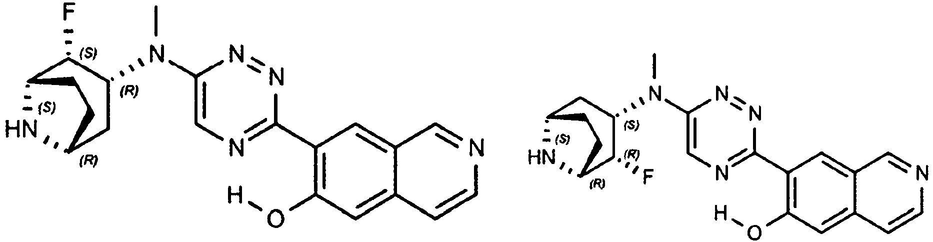

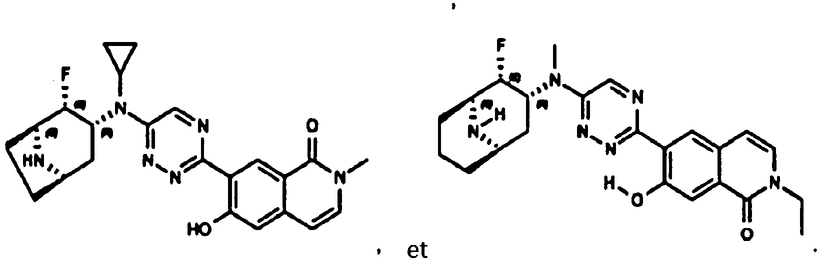

15. Composition pharmaceutique comprenant le composé selon l'une quelconque des revendications 1 à 14, ou un sel pharmaceutiquement acceptable de celui-ci, et un excipient pharmaceutiquement acceptable.

16. Composé selon l'une quelconque des revendications 1 à 14, ou un sel pharmaceutiquement acceptable de celui-ci, ou une composition pharmaceutique selon la revendication 15,
à utiliser dans un procédé de traitement d'une maladie ou d'un état,
dans lequel le procédé comprend l'administration du composé, du sel ou de la composition pharmaceutique à un sujet qui en a besoin.
